# EUROPEAN PATENT APPLICATION

(11) **EP 2 407 541 A1**
(43) Date of publication of application: **18.01.2012**
(21) Application number: 11184699.4
(22) Date of filing: 07.12.2007
(51) Int. Cl.: C12N 15/10, C12P 7/06

(54) **Global transcription machinery engineering**

(30) Priority: 07.12.2006 US 873419 P
(62) Divisional of application: 07874166.7
(71) Applicant: Massachusetts Institute of Technology, Cambridge MA 02142 (US); Whitehead Institute for Biomedical Research, Cambridge, MA 02142 (US)
(72) Inventor: Alper, Hal S., Cambridge, MA 02138 (US); Stephanopoulos, Gregory, Winchester, MA 01890 (US); Fink, Gerald, Chestnut Hill, MA 01267 (US)
(74) Representative: White, Nina Louise

(57) **Abstract**

The application relates to global transcription machinery engineering to produce altered cells having improved phenotypes.

SEQUENCE LISTING

<110>  MASSACHUSETTS INSTITUTE OF TECHNOLOGY
       WHITEHEAD INSTITUTE FOR BIOMEDICAL RESEARCH
       ALPER, Hal S.
       STEPHANOPOULOS, Gregory
       FINK, Gerald

<120>  GLOBAL TRANSCRIPTION MACHINERY ENGINEERING

<130>  M0656.70138WO00

<150>  US 60/873,419
<151>  2006-12-07

<160>  56

<170>  PatentIn version 3.4

<210>  1
<211>  44
<212>  DNA
<213>  Artificial

<220>
<223>  Primer CM_sense_AhdI

<400>  1
gttgcctgac tccccgtcgc caggcgttta agggcaccaa taac                        44


<210>  2
<211>  32
<212>  DNA
<213>  Artificial

<220>
<223>  Primer CM_anti_AatII

<400>  2
cagaagccac tggagcacct caaaactgca gt                                     32


<210>  3
<211>  34
<212>  DNA
<213>  Artificial

<220>
<223>  Primer pSC_sense_AflIII

<400>  3
cccacatgtc ctagacctag ctgcaggtcg agga                                   34


<210>  4
<211>  53
<212>  DNA
<213>  Artificial

<220>
<223>  Primer pSC_anti_NotI

<400>  4
aaggaaaaaa gcggccgcac gggtaagcct gttgatgata ccgctgcctt act          53


<210>  5
<211>  41
<212>  DNA
<213>  Artificial

<220>
<223>  Primer rpoD_sense_SacI

<400>  5
aacctaggag ctctgattta acggcttaag tgccgaagag c                       41


<210>  6
<211>  37
<212>  DNA
<213>  Artificial

<220>
<223>  Primer rpoD_anti_HindIII

<400>  6
tggaagcttt aacgcctgat ccggcctacc gattaat                            37


<210>  7
<211>  23
<212>  DNA
<213>  Artificial

<220>
<223>  Sequencing primer S1

<400>  7
ccatatgcgg tgtgaaatac cgc                                           23


<210>  8
<211>  24
<212>  DNA
<213>  Artificial

<220>
<223>  Sequencing primer S2

<400>  8
cacagctgaa acttcttgtc accc                                          24


<210>  9
<211>  22
<212>  DNA
<213>  Artificial

<220>
<223>  Sequencing primer S3

<400>  9
ttgttgaccc gaacgcagaa ga                                                22


<210>  10
<211>  20
<212>  DNA
<213>  Artificial

<220>
<223>  Sequencing primer S4

<400>  10
agaaaccggc ctgaccatcg                                                   20


<210>  11
<211>  24
<212>  DNA
<213>  Artificial

<220>
<223>  Sequencing primer A1

<400>  11
gcttcgatct gacggatacg ttcg                                              24


<210>  12
<211>  24
<212>  DNA
<213>  Artificial

<220>
<223>  Sequencing primer A2

<400>  12
caggttgcgt aggtggagaa cttg                                              24


<210>  13
<211>  22
<212>  DNA
<213>  Artificial

<220>
<223>  Sequencing primer A3

<400>  13
gtgactgcga cctttcgctt tg                                                22


<210>  14
<211>  20
<212>  DNA
<213>  Artificial

<220>
<223>  Sequencing primer A4

<400>  14
catcagatca tcggcatccg                                                    20


<210>  15
<211>  19
<212>  DNA
<213>  Artificial

<220>
<223>  Sequencing primer A5

<400>  15
gcttcggcag catcttcgt                                                     19


<210>  16
<211>  21
<212>  DNA
<213>  Artificial

<220>
<223>  Sequencing primer A6

<400>  16
cggaagcgat cacctatctg c                                                  21


<210>  17
<211>  613
<212>  PRT
<213>  Escherichia coli

<400>  17

Met Glu Gln Asn Pro Gln Ser Gln Leu Lys Leu Leu Val Thr Arg Gly
1               5                   10                  15


Lys Glu Gln Gly Tyr Leu Thr Tyr Ala Glu Val Asn Asp His Leu Pro
            20                  25                  30


Glu Asp Ile Val Asp Ser Asp Gln Ile Glu Asp Ile Ile Gln Met Ile
        35                  40                  45


Asn Asp Met Gly Ile Gln Val Met Glu Glu Ala Pro Asp Ala Asp Asp
    50                  55                  60


Leu Met Leu Ala Glu Asn Thr Ala Asp Glu Asp Ala Ala Glu Ala Ala
65                  70                  75                  80


Ala Gln Val Leu Ser Ser Val Glu Ser Glu Ile Gly Arg Thr Thr Asp
                85                  90                  95

Pro Val Arg Met Tyr Met Arg Glu Met Gly Thr Val Glu Leu Leu Thr
            100                 105                 110

Arg Glu Gly Glu Ile Asp Ile Ala Lys Arg Ile Glu Asp Gly Ile Asn
        115                 120                 125

Gln Val Gln Cys Ser Val Ala Glu Tyr Pro Glu Ala Ile Thr Tyr Leu
    130                 135                 140

Leu Glu Gln Tyr Asp Arg Val Glu Ala Glu Glu Ala Arg Leu Ser Asp
145                 150                 155                 160

Leu Ile Thr Gly Phe Val Asp Pro Asn Ala Glu Glu Asp Leu Ala Pro
                165                 170                 175

Thr Ala Thr His Val Gly Ser Glu Leu Ser Gln Glu Asp Leu Asp Asp
            180                 185                 190

Asp Glu Asp Glu Asp Glu Glu Asp Gly Asp Asp Asp Ser Ala Asp Asp
        195                 200                 205

Asp Asn Ser Ile Asp Pro Glu Leu Ala Arg Glu Lys Phe Ala Glu Leu
    210                 215                 220

Arg Ala Gln Tyr Val Val Thr Arg Asp Thr Ile Lys Ala Lys Gly Arg
225                 230                 235                 240

Ser His Ala Thr Ala Gln Glu Glu Ile Leu Lys Leu Ser Glu Val Phe
                245                 250                 255

Lys Gln Phe Arg Leu Val Pro Lys Gln Phe Asp Tyr Leu Val Asn Ser
            260                 265                 270

Met Arg Val Met Met Asp Arg Val Arg Thr Gln Glu Arg Leu Ile Met
        275                 280                 285

Lys Leu Cys Val Glu Gln Cys Lys Met Pro Lys Lys Asn Phe Ile Thr
    290                 295                 300

Leu Phe Thr Gly Asn Glu Thr Ser Asp Thr Trp Phe Asn Ala Ala Ile
305                 310                 315                 320

Ala Met Asn Lys Pro Trp Ser Glu Lys Leu His Asp Val Ser Glu Glu
                325                 330                 335

Val His Arg Ala Leu Gln Lys Leu Gln Gln Ile Glu Glu Glu Thr Gly
            340                 345                 350

Leu Thr Ile Glu Gln Val Lys Asp Ile Asn Arg Arg Met Ser Ile Gly
        355                 360                 365

Glu Ala Lys Ala Arg Arg Ala Lys Lys Glu Met Val Glu Ala Asn Leu
    370                 375                 380

Arg Leu Val Ile Ser Ile Ala Lys Lys Tyr Thr Asn Arg Gly Leu Gln
385                 390                 395                 400

Phe Leu Asp Leu Ile Gln Glu Gly Asn Ile Gly Leu Met Lys Ala Val
                405                 410                 415

Asp Lys Phe Glu Tyr Arg Arg Gly Tyr Lys Phe Ser Thr Tyr Ala Thr
            420                 425                 430

Trp Trp Ile Arg Gln Ala Ile Thr Arg Ser Ile Ala Asp Gln Ala Arg
        435                 440                 445

Thr Ile Arg Ile Pro Val His Met Ile Glu Thr Ile Asn Lys Leu Asn
    450                 455                 460

Arg Ile Ser Arg Gln Met Leu Gln Glu Met Gly Arg Glu Pro Thr Pro
465                 470                 475                 480

Glu Glu Leu Ala Glu Arg Met Leu Met Pro Glu Asp Lys Ile Arg Lys
                485                 490                 495

Val Leu Lys Ile Ala Lys Glu Pro Ile Ser Met Glu Thr Pro Ile Gly
            500                 505                 510

Asp Asp Glu Asp Ser His Leu Gly Asp Phe Ile Glu Asp Thr Thr Leu
        515                 520                 525

Glu Leu Pro Leu Asp Ser Ala Thr Thr Glu Ser Leu Arg Ala Ala Thr
    530                 535                 540

His Asp Val Leu Ala Gly Leu Thr Ala Arg Glu Ala Lys Val Leu Arg
545                 550                 555                 560

Met Arg Phe Gly Ile Asp Met Asn Thr Asp Tyr Thr Leu Glu Glu Val
                565                 570                 575

Gly Lys Gln Phe Asp Val Thr Arg Glu Arg Ile Arg Gln Ile Glu Ala
            580                 585                 590

Lys Ala Leu Arg Lys Leu Arg His Pro Ser Arg Ser Glu Val Leu Arg
        595                 600                 605

Ser Phe Leu Asp Asp
    610

<210>  18
<211>  612
<212>  PRT
<213>  Escherichia coli

<400>  18

Met Glu Gln Asn Pro Gln Ser Gln Leu Lys Leu Leu Val Thr Arg Gly
1               5                   10                  15

Lys Glu Gln Gly Tyr Leu Thr Tyr Ala Glu Val Asn Asp Leu Leu Pro
            20                  25                  30

Glu Asp Ile Val Asp Asp Gln Ile Glu Asp Ile Ile Gln Met Ile Asn
        35                  40                  45

Asp Met Gly Ile Gln Val Met Glu Glu Ala Ser Asp Ala Asp Asp Leu
    50                  55                  60

Met Leu Ala Glu Asn Thr Ala Asp Glu Asp Ala Ala Glu Ala Ala Ala
65                  70                  75                  80

Gln Val Leu Ser Ser Val Glu Ser Glu Ile Gly Arg Thr Ile Asp Pro
                85                  90                  95

Val Arg Met Tyr Met Arg Glu Met Gly Thr Val Glu Leu Leu Thr Arg
            100                 105                 110

Glu Gly Glu Ile Asp Ile Ala Lys Arg Ile Glu Asp Gly Ile Asn Gln
        115                 120                 125

Val Gln Cys Ser Val Ala Glu Tyr Pro Glu Ala Ile Thr Tyr Leu Leu
    130                 135                 140

Glu Gln Tyr Asp Arg Val Glu Ala Glu Glu Ala Arg Leu Ser Asp Leu
145                 150                 155                 160

Ile Thr Gly Phe Val Asp Pro Asn Ala Glu Glu Asp Leu Ala Pro Thr
                165                 170                 175

Ala Thr His Val Gly Ser Glu Leu Ser Gln Glu Asp Leu Asp Asp Asp
            180                 185                 190

Glu Asp Glu Asp Glu Glu Asp Gly Asp Asp Gly Ser Ala Asp Asp Asp
        195                 200                 205

Asn Ser Ile Asp Pro Glu Leu Ala Arg Glu Lys Leu Ala Glu Leu Arg
    210                 215                 220

Ala Gln Tyr Val Val Thr Arg Asp Thr Ile Lys Ala Lys Gly Arg Ser
225                 230                 235                 240

His Ala Thr Ala Gln Glu Glu Phe Leu Lys Leu Ser Glu Val Phe Lys
                245                 250                 255

Gln Phe Arg Leu Val Pro Lys Gln Phe Asp Tyr Leu Val Asn Ser Met
            260                 265                 270

Arg Val Met Met Asp Arg Val Arg Thr Gln Glu Arg Leu Val Met Lys
        275                 280                 285

Leu Cys Val Glu Gln Cys Lys Met Pro Lys Lys Asn Phe Ile Thr Leu
    290                 295                 300

Phe Thr Gly Asn Glu Thr Ser Asp Thr Trp Phe Asn Ala Ala Ile Ala
305                 310                 315                 320

Met Asn Lys Pro Trp Ser Glu Lys Leu His Asp Val Ser Glu Glu Val
                325                 330                 335

His Arg Ala Leu Gln Lys Leu Gln Gln Ile Glu Glu Glu Thr Gly Leu
            340                 345                 350

Thr Ile Glu Gln Val Lys Asp Ile Asn Arg Arg Met Ser Ile Gly Glu
        355                 360                 365

Ala Lys Ala Arg Arg Ala Lys Lys Glu Met Val Glu Ala Asn Leu Arg
    370                 375                 380

Leu Val Ile Ser Ile Ala Lys Lys Tyr Thr Asn Arg Gly Leu Gln Phe
385                 390                 395                 400

Leu Asp Leu Ile Gln Glu Gly Asn Ile Gly Leu Met Lys Ala Val Asp
                405                 410                 415

Lys Phe Glu Tyr Arg Arg Gly Tyr Lys Phe Ser Thr Tyr Ala Thr Trp
            420                 425                 430

Trp Ile Arg Gln Ala Ile Thr Arg Ser Ile Ala Asp Gln Ala Arg Thr
        435                 440                 445

Ile Arg Ile Pro Val His Met Ile Glu Thr Ile Asn Lys Leu Asn Arg
    450                 455                 460

Ile Ser Arg Gln Met Leu Gln Glu Met Gly Arg Glu Pro Thr Pro Glu
465                 470                 475                 480

Glu Leu Ala Glu Arg Met Leu Met Pro Glu Asp Lys Ile Arg Lys Val
                485                 490                 495

Leu Lys Ile Ala Lys Glu Pro Ile Ser Met Glu Thr Pro Val Gly Asp
            500                 505                 510

Asp Glu Asp Ser His Leu Gly Asp Phe Ile Glu Asp Thr Thr Leu Glu
        515                 520                 525

Leu Pro Leu Asp Ser Ala Thr Thr Glu Ser Leu Arg Ala Ala Thr His
    530                 535                 540

Asp Val Leu Ala Gly Leu Thr Ala Arg Glu Ala Lys Val Leu Arg Met
545                 550                 555                 560

Arg Phe Gly Ile Asp Met Asn Thr Asp Tyr Thr Leu Glu Glu Val Gly
                565                 570                 575

Lys Gln Phe Asp Val Thr Arg Glu Arg Ile Arg Gln Ile Glu Ala Lys
            580                 585                 590

Ala Leu Arg Lys Leu Arg His Pro Ser Cys Ser Glu Val Leu Arg Ser
        595                 600                 605

Phe Leu Asp Asp
    610

<210>  19
<211>  107
<212>  PRT

<213>  Escherichia coli

<400>  19

Met Glu Thr Pro Ile Gly Asp Asp Glu Asp Ser His Leu Gly Asp Phe
1               5                   10                  15

Ile Glu Asp Thr Thr Leu Glu Leu Pro Leu Asp Ser Ala Thr Thr Glu
            20                  25                  30

Ser Leu Arg Ala Ala Thr His Asp Val Leu Ala Gly Leu Thr Ala Arg
        35                  40                  45

Glu Ala Lys Val Leu Arg Met Arg Phe Gly Ile Asp Met Asn Thr Asp
    50                  55                  60

Tyr Thr Leu Glu Glu Val Gly Lys Gln Phe Asp Val Thr Arg Glu Arg
65                  70                  75                  80

Ile Arg Gln Ile Glu Ala Lys Ala Leu Arg Lys Leu Arg His Pro Ser
                85                  90                  95

Cys Ser Glu Val Leu Arg Ser Phe Leu Asp Asp
            100                 105

<210>  20
<211>  107
<212>  PRT
<213>  Escherichia coli

<400>  20

Met Glu Thr Pro Ile Gly Asp Asp Glu Asp Ser Leu Leu Gly Asp Phe
1               5                   10                  15

Ile Glu Asp Thr Thr Leu Glu Leu Pro Leu Asp Ser Ala Thr Thr Val
            20                  25                  30

Ser Leu Arg Val Ala Thr His Asp Val Leu Ala Gly Leu Thr Ala Arg
        35                  40                  45

Val Ala Lys Val Leu Arg Met Arg Phe Gly Ile Asn Met Asn Thr Asp
    50                  55                  60

Tyr Thr Leu Glu Glu Val Gly Lys Gln Phe Asp Val Thr Arg Glu Arg
65                  70                  75                  80

Ile Arg Gln Ile Glu Ala Lys Ala Leu Arg Lys Leu Arg His Pro Ser

85                  90                  95

Arg Ser Gly Val Gln Arg Ser Phe Leu Asp Asp
            100                 105

<210>  21
<211>  107
<212>  PRT
<213>  Escherichia coli

<400>  21

Met Glu Thr Pro Ile Gly Asp Asp Glu Asp Ser His Leu Gly Asp Tyr
1               5                   10                  15

Ile Glu Asp Thr Thr Leu Glu Leu Pro Leu Asp Ser Ala Thr Thr Glu
            20                  25                  30

Ser Leu Arg Ala Ala Thr His Asp Val Leu Ala Gly Met Thr Ala Arg
        35                  40                  45

Glu Ala Lys Val Leu Arg Met Arg Phe Gly Ile Asp Val Asn Thr Asp
    50                  55                  60

Tyr Thr Leu Glu Glu Val Gly Lys Gln Phe Asp Val Thr Arg Glu Arg
65                  70                  75                  80

Ile Ser Gln Ile Glu Ala Lys Ala Leu Arg Lys Leu Arg His Pro Ser
                85                  90                  95

Arg Ser Glu Val Leu Arg Ser Phe Leu Asp Asp
            100                 105

<210>  22
<211>  612
<212>  PRT
<213>  Escherichia coli

<400>  22

Met Glu Gln Asn Pro Gln Ser Gln Leu Lys Leu Leu Val Thr Arg Gly
1               5                   10                  15

Lys Glu Gly Tyr Leu Thr Tyr Ala Glu Val Asn Asp His Leu Pro Glu
            20                  25                  30

Asp Ile Val Asp Ser Asp Gln Ile Glu Asp Ile Ile Gln Met Ile Asn
        35                  40                  45

Asp Met Gly Ile Gln Val Met Glu Glu Ala Pro Asp Ala Asp Asp Leu
    50                  55                  60

Met Leu Ala Glu Asn Thr Ala Asp Glu Asp Ala Ala Glu Ala Ala Ala
65                  70                  75                  80

Gln Val Leu Ser Ser Val Glu Ser Gly Val Gly Arg Thr Thr Asp Pro
                85                  90                  95

Val Arg Met Tyr Met Arg Glu Met Gly Thr Val Glu Leu Leu Thr Arg
            100                 105                 110

Glu Gly Glu Ile Asp Ile Ala Lys Arg Ile Glu Asp Gly Asn Asn Gln
        115                 120                 125

Val Gln Cys Ser Val Ala Glu Tyr Pro Glu Ala Ile Thr Tyr Leu Leu
    130                 135                 140

Glu Gln Tyr Asp Arg Ala Glu Ala Glu Glu Ala Arg Leu Ser Asp Met
145                 150                 155                 160

Ile Thr Gly Phe Val Asp Pro Asn Ala Glu Glu Asp Leu Ala Pro Thr
                165                 170                 175

Ala Thr His Val Gly Ser Glu Leu Ser Gln Glu Asp Leu Asp Asp Asp
            180                 185                 190

Glu Asp Glu Asp Glu Val Asp Gly Asp Asp Asp Ser Ala Asp Asp Asp
        195                 200                 205

Asn Ser Ile Asp Pro Glu Leu Ala Arg Glu Lys Phe Ala Glu Leu Arg
    210                 215                 220

Ala Gln Tyr Val Val Thr Arg Asp Thr Ile Lys Ala Lys Gly Arg Ser
225                 230                 235                 240

His Ala Thr Ala Gln Glu Glu Ile Leu Lys Leu Ser Glu Val Phe Lys
                245                 250                 255

Gln Phe Arg Leu Val Pro Lys Gln Phe Asp Tyr Leu Val Asn Ser Met
            260                 265                 270

Arg Val Met Met Asp His Val Arg Thr Gln Glu Arg Leu Ile Met Lys
        275                 280                 285

Leu Cys Val Glu Gln Cys Lys Met Pro Lys Lys Asn Phe Ile Thr Leu
    290                 295                 300

Phe Thr Gly Asn Glu Thr Ser Asp Thr Trp Phe Asn Ala Ala Ile Ala
305                 310                 315                 320

Met Asn Arg Pro Trp Ser Glu Lys Leu His Asp Val Ser Glu Glu Val
                325                 330                 335

His Arg Ala Leu Gln Lys Leu Gln Gln Ile Glu Glu Glu Thr Gly Leu
            340                 345                 350

Thr Ile Glu Gln Val Lys Asp Ile Asn Arg Arg Met Ser Ile Gly Glu
        355                 360                 365

Ala Lys Ala Arg Arg Ala Lys Lys Glu Met Val Glu Ala Asn Leu Arg
    370                 375                 380

Leu Val Ile Ser Ile Ala Lys Lys Tyr Thr Asn Arg Gly Leu Gln Phe
385                 390                 395                 400

Leu Asp Leu Ile Gln Glu Gly Asn Ile Gly Leu Met Lys Ala Val Asp
                405                 410                 415

Lys Phe Glu Tyr Arg Arg Gly Tyr Lys Phe Ser Thr Tyr Ala Thr Trp
            420                 425                 430

Trp Ile Arg Gln Ala Ile Thr Arg Ser Ile Ala Asp Gln Ala Arg Thr
        435                 440                 445

Ile Arg Ile Pro Val His Met Ile Glu Thr Ile Asn Lys Leu Asn Arg
    450                 455                 460

Ile Ser Arg Gln Met Leu Gln Glu Met Gly Arg Glu Pro Thr Pro Glu
465                 470                 475                 480

Glu Leu Ala Glu Arg Met Leu Met Pro Glu Asp Lys Ile Arg Lys Val
                485                 490                 495

Leu Lys Ile Ala Lys Glu Pro Ile Ser Met Glu Thr Pro Ile Gly Asp
            500                 505                 510

Asp Glu Asp Ser His Leu Gly Asp Phe Ile Glu Asp Thr Thr Leu Glu
        515                 520                 525

Leu Pro Leu Asp Ser Ala Thr Thr Glu Ser Leu Arg Ala Ala Thr His
    530                 535                 540

Asp Val Leu Ala Gly Leu Thr Ala Arg Glu Ala Lys Val Leu Arg Met
545                 550                 555                 560

Arg Phe Gly Ile Asp Met Asn Thr Asp Tyr Thr Leu Glu Glu Val Gly
                565                 570                 575

Lys Gln Phe Asp Val Thr Arg Glu Arg Ile Arg Gln Ile Glu Ala Lys
            580                 585                 590

Ala Leu Arg Lys Leu Arg His Pro Ser Arg Ser Glu Val Leu Arg Ser
        595                 600                 605

Phe Leu Asp Asp
    610

<210>  23
<211>  613
<212>  PRT
<213>  Escherichia coli

<400>  23

Met Asp Gln Asn Pro Gln Ser Gln Leu Lys Leu Leu Val Thr Arg Gly
1               5                   10                  15

Lys Glu Gln Gly Tyr Leu Thr Tyr Ala Glu Val Asn Asp His Leu Pro
            20                  25                  30

Glu Asp Ile Val Asp Ser Asp Gln Ile Glu Asp Ile Ile Gln Met Ile
        35                  40                  45

Asn Asp Met Gly Ile Gln Val Met Glu Glu Ala Pro Asp Ala Asp Asp
    50                  55                  60

Leu Met Leu Ala Glu Asn Thr Ala Asp Glu Asp Ala Ala Glu Ala Val
65                  70                  75                  80

Ala Gln Val Leu Ser Ser Val Glu Ser Glu Ile Gly Arg Thr Thr Asp
                85                  90                  95

Pro Val Arg Met Tyr Met Arg Glu Met Gly Thr Val Glu Leu Leu Thr
            100                 105                 110

Arg Glu Gly Glu Ile Asp Ile Ala Lys Arg Ile Glu Asp Gly Ile Asn
        115                 120                 125

Gln Val Gln Cys Ser Val Ala Glu Tyr Pro Glu Ala Ile Thr Tyr Leu
    130                 135                 140

Leu Glu Gln Tyr Asp Arg Val Glu Ala Glu Glu Ala Arg Leu Ser Asp
145                 150                 155                 160

Leu Ile Thr Gly Phe Val Asp Pro Asn Ala Glu Glu Asp Leu Ala Pro
                165                 170                 175

Thr Ala Thr His Val Gly Ser Glu Pro Ser Gln Glu Asp Leu Asp Asp
            180                 185                 190

Asp Glu Asp Glu Asp Glu Glu Asp Gly Asp Asp Asp Ser Ala Asp Asp
        195                 200                 205

Asp Asn Ser Ile Asp Pro Glu Leu Ala Arg Glu Lys Phe Ala Glu Leu
    210                 215                 220

Arg Ala Gln Tyr Val Val Thr Arg Asp Thr Ile Lys Ala Lys Gly Arg
225                 230                 235                 240

Ser His Ala Thr Ala Gln Glu Glu Ile Leu Lys Leu Ser Glu Val Phe
                245                 250                 255

Lys Gln Phe Arg Leu Val Pro Lys Gln Phe Asp Tyr Leu Val Asn Ser
            260                 265                 270

Met Arg Val Met Met Asp Arg Val Arg Thr Gln Glu Arg Leu Ile Met
        275                 280                 285

Lys Leu Cys Val Glu Gln Cys Lys Met Pro Lys Lys Asn Phe Ile Thr
    290                 295                 300

Leu Phe Thr Gly Asn Glu Thr Ser Asp Thr Trp Phe Asn Ala Ala Ile
305                 310                 315                 320

Ala Met Asn Lys Leu Trp Ser Glu Lys Leu His Asp Val Ser Glu Glu
                325                 330                 335

Val His Arg Ala Leu Gln Lys Leu Arg Gln Ile Glu Glu Glu Thr Gly
            340                 345                 350

Leu Thr Ile Glu Gln Val Lys Asp Ile Asn Arg Arg Met Ser Ile Gly
        355                 360                 365

Glu Ala Lys Ala Arg Arg Ala Lys Lys Glu Met Val Glu Ala Asn Leu
    370                 375                 380

Arg Leu Val Ile Ser Ile Ala Lys Lys Tyr Thr Asn Arg Gly Leu Gln
385                 390                 395                 400

Phe Leu Asp Leu Ile Gln Glu Gly Asn Ile Gly Leu Met Lys Ala Val
                405                 410                 415

Asp Lys Phe Glu Tyr Arg Arg Gly Tyr Lys Phe Ser Thr Tyr Ala Thr
            420                 425                 430

Trp Trp Ile Arg Gln Ala Ile Thr Arg Phe Ile Ala Asp Gln Ala Arg
        435                 440                 445

Thr Ile Arg Ile Pro Val His Met Ile Glu Thr Ile Asn Lys Leu Asn
    450                 455                 460

Arg Ile Ser Arg Gln Met Leu Gln Glu Met Gly Arg Glu Pro Thr Pro
465                 470                 475                 480

Glu Glu Leu Ala Glu Arg Met Leu Met Pro Glu Asp Lys Ile Arg Lys
                485                 490                 495

Val Leu Lys Ile Ala Lys Glu Pro Ile Ser Met Glu Thr Pro Ile Gly
            500                 505                 510

Asp Asp Glu Asp Ser His Leu Gly Asp Phe Ile Glu Asp Thr Thr Leu
        515                 520                 525

Glu Leu Pro Leu Asp Ser Ala Thr Thr Glu Ser Leu Arg Ala Ala Thr
    530                 535                 540

His Asp Val Leu Ala Gly Leu Thr Ala Arg Glu Ala Lys Val Leu Arg
545                 550                 555                 560

Met Arg Phe Gly Ile Asp Val Asn Thr Asp Tyr Thr Leu Glu Glu Val
                565                 570                 575

Ser Lys Gln Phe Asn Val Thr Arg Glu Arg Ile Arg Gln Ile Glu Ala
            580                 585                 590

Lys Ala Leu Arg Lys Leu Arg His Pro Ser Arg Ser Asp Val Leu Arg
        595                 600                 605

Ser Phe Leu Asp Asp
    610

<210> 24
<211> 611
<212> PRT
<213> Escherichia coli

<400> 24

Met Glu Gln Asn Pro Gln Ser Gln Leu Lys Leu Leu Val Thr Arg Gly
1               5                   10                  15

Lys Glu Gln Gly Tyr Leu Thr Tyr Ala Glu Val Asn Asp His Leu Pro
            20                  25                  30

Glu Asp Ile Val Asp Ser Asp Gln Ile Glu Asp Ile Ile Gln Met Ile
        35                  40                  45

Asn Asp Met Gly Ile Gln Val Met Glu Glu Ala Pro Asp Ala Asp Asp
    50                  55                  60

Leu Met Leu Ala Glu Asn Thr Ala Asp Glu Asp Ala Ala Glu Ala Ala
65                  70                  75                  80

Ala Gln Val Leu Ser Ser Val Glu Ser Glu Ile Gly Arg Thr Thr Asp
                85                  90                  95

Pro Val Arg Met Tyr Met Arg Glu Met Gly Thr Val Glu Leu Leu Thr
            100                 105                 110

Arg Glu Gly Glu Ile Asp Ile Ala Lys Arg Ile Glu Asp Gly Val Asn
        115                 120                 125

Gln Val Gln Cys Ser Val Ala Glu Tyr Pro Glu Ala Ile Thr Tyr Leu
    130                 135                 140

Leu Glu Gln Tyr Asp Arg Val Glu Ala Glu Glu Ala Arg Leu Ser Asp
145                 150                 155                 160

Leu Ile Thr Gly Phe Val Asp Pro Asn Ala Glu Glu Asp Leu Ala Pro
                165                 170                 175

Thr Ala Thr His Val Gly Ser Glu Leu Ser Gln Glu Asp Leu Asp Asp

            180                 185                 190

Asp Glu Asp Glu Asp Glu Glu Asp Gly Asp Asp Asp Ser Ala Asp Asp
        195                 200                 205

Asp Asn Ser Ile Asp Pro Glu Leu Ala Arg Glu Lys Phe Ala Glu Leu
    210                 215                 220

Arg Ala Gln Tyr Val Val Thr Arg Asp Thr Ile Ala Lys Gly Arg Ser
225                 230                 235                 240

His Ala Thr Ala Gln Glu Glu Ile Leu Lys Leu Ser Glu Val Phe Lys
                245                 250                 255

Gln Phe Arg Leu Val Pro Lys Gln Phe Asp Tyr Leu Val Asn Ser Met
            260                 265                 270

Arg Val Met Met Asp Arg Val Arg Thr Gln Glu Arg Leu Ile Met Lys
        275                 280                 285

Leu Cys Val Glu Gln Cys Lys Met Pro Lys Lys Asn Phe Ile Thr Leu
    290                 295                 300

Phe Thr Gly Asn Glu Thr Ser Asp Thr Trp Phe Asn Ala Ala Ile Ala
305                 310                 315                 320

Met Asn Lys Pro Trp Ser Glu Lys Leu His Asp Val Ser Glu Glu Val
                325                 330                 335

His Arg Ala Leu Gln Lys Leu Gln Gln Ile Glu Glu Glu Thr Gly Leu
            340                 345                 350

Thr Ile Glu Gln Val Lys Asp Ile Asn Arg Arg Met Ser Ile Gly Glu
        355                 360                 365

Ala Lys Ala Arg Arg Ala Lys Lys Glu Met Val Glu Ala Asn Leu Arg
    370                 375                 380

Leu Val Ile Ser Ile Ala Lys Lys Phe Thr Asn Arg Gly Leu Gln Phe
385                 390                 395                 400

Leu Asp Leu Ile Gln Glu Gly Asn Ile Gly Leu Val Lys Ala Val Asp
                405                 410                 415

Lys Phe Glu Tyr Arg Arg Gly Tyr Lys Phe Ser Thr Tyr Ala Thr Trp

420                 425                 430

Trp Ile Arg Gln Ala Ile Thr Arg Ser Ile Ala Asp Gln Ala Arg Thr
        435                 440                 445

Ile Arg Ile Pro Val His Met Ile Glu Thr Ile Asn Leu Asn Arg Ile
    450                 455                 460

Ser Arg Gln Met Leu Gln Glu Met Gly Arg Glu Pro Thr Pro Glu Glu
465                 470                 475                 480

Leu Ala Glu Arg Met Leu Met Gln Glu Asp Lys Ile Arg Lys Val Leu
                485                 490                 495

Lys Ile Ala Asn Glu Pro Ile Ser Met Glu Thr Pro Ile Gly Asp Asp
            500                 505                 510

Glu Asp Ser His Leu Gly Asp Phe Ile Glu Asp Thr Thr Leu Glu Leu
        515                 520                 525

Pro Leu Asp Ser Ala Thr Thr Glu Ser Leu Arg Ala Ala Thr His Asp
    530                 535                 540

Val Leu Ala Gly Leu Thr Ala Arg Glu Ala Lys Val Leu Arg Met Arg
545                 550                 555                 560

Phe Gly Ile Asp Met Asn Thr Asp Tyr Thr Leu Glu Glu Val Gly Lys
                565                 570                 575

Gln Phe Asp Val Thr Arg Glu Arg Ile Arg Gln Ile Glu Ala Lys Ala
            580                 585                 590

Leu Arg Lys Leu Arg His Pro Ser Arg Pro Glu Val Leu Arg Ser Phe
        595                 600                 605

Leu Asp Asp
    610

<210>  25
<211>  613
<212>  PRT
<213>  Escherichia coli

<400>  25

Met Glu Gln Asn Pro Gln Ser Gln Leu Lys Leu Leu Val Thr Arg Gly
1               5                   10                  15

Lys Glu Gln Gly Tyr Leu Thr Tyr Ala Glu Val Asn Asp His Leu Pro
            20                  25                  30

Glu Asp Ile Val Asp Ser Asp Gln Ile Glu Asp Ile Ile Gln Met Ile
        35                  40                  45

Asn Asp Met Gly Ile Gln Val Met Glu Glu Ala Pro Asp Ala Asp Asp
    50                  55                  60

Leu Met Leu Ala Glu Asn Thr Ala Asp Glu Asp Ala Ala Glu Ala Ala
65                  70                  75                  80

Ala Gln Val Leu Ser Ser Val Glu Ser Glu Ile Gly Arg Thr Thr Asp
                85                  90                  95

Pro Val Arg Met Tyr Met Arg Glu Met Gly Thr Val Glu Leu Leu Thr
            100                 105                 110

Arg Glu Gly Glu Ile Asp Ile Ala Lys Arg Ile Glu Asp Gly Ile Asn
        115                 120                 125

Gln Val Gln Cys Ser Val Ala Glu Tyr Pro Glu Ala Ile Thr Tyr Leu
    130                 135                 140

Leu Glu Gln Tyr Asp Arg Val Glu Ala Glu Glu Ala Arg Leu Ser Asp
145                 150                 155                 160

Leu Ile Thr Gly Phe Val Asp Pro Asn Ala Glu Glu Asp Leu Ala Pro
                165                 170                 175

Thr Ala Thr His Val Gly Ser Glu Leu Ser Gln Glu Asp Leu Asp Asp
            180                 185                 190

Asp Glu Asp Glu Asp Glu Glu Asp Gly Asp Asp Asp Ser Ala Asp Asp
        195                 200                 205

Asp Asn Ser Ile Asp Pro Glu Leu Ala Arg Glu Lys Phe Ala Glu Leu
    210                 215                 220

Arg Ala Gln Tyr Val Val Thr Arg Asp Thr Ile Lys Ala Lys Gly Arg
225                 230                 235                 240

Ser His Ala Thr Ala Gln Glu Glu Ile Leu Lys Leu Ser Glu Val Phe
                245                 250                 255

Lys Gln Phe Arg Leu Val Pro Lys Gln Phe Asp Tyr Leu Val Asn Ser
            260                 265                 270

Met Arg Val Met Met Asp Arg Val Arg Thr Gln Glu Arg Leu Ile Met
        275                 280                 285

Lys Leu Cys Val Glu Gln Cys Lys Met Pro Lys Lys Asn Phe Ile Thr
    290                 295                 300

Leu Phe Thr Gly Asn Glu Thr Ser Asp Thr Trp Phe Asn Ala Ala Ile
305                 310                 315                 320

Ala Met Asn Lys Pro Trp Ser Glu Lys Leu His Asp Val Ser Glu Glu
                325                 330                 335

Val His Arg Ala Leu Gln Lys Leu Gln Gln Ile Glu Glu Glu Thr Gly
            340                 345                 350

Leu Thr Ile Glu Gln Val Lys Asp Ile Asn Arg Arg Met Ser Ile Gly
        355                 360                 365

Glu Ala Lys Ala Arg Arg Ala Lys Lys Glu Met Val Glu Ala Asn Leu
    370                 375                 380

Arg Leu Val Ile Ser Ile Ala Lys Lys Tyr Thr Asn Arg Gly Leu Gln
385                 390                 395                 400

Phe Leu Asp Leu Ile Gln Glu Gly Tyr Ile Gly Leu Met Lys Ala Val
                405                 410                 415

Asp Lys Phe Glu Tyr Arg Arg Gly Tyr Lys Phe Ser Thr Tyr Ala Thr
            420                 425                 430

Trp Trp Ile Arg Gln Ala Ile Thr Arg Ser Ile Ala Asp Gln Ala Arg
        435                 440                 445

Thr Ile Arg Ile Pro Val His Met Ile Glu Thr Ile Asp Lys Leu Asn
    450                 455                 460

Arg Ile Ser Arg Gln Met Leu Gln Glu Met Gly Arg Glu Pro Thr Pro
465                 470                 475                 480

Glu Glu Leu Ala Glu Arg Met Leu Met Pro Glu Asp Lys Ile Arg Lys
                485                 490                 495

Val Leu Lys Ile Ala Lys Glu Pro Ile Ser Met Glu Thr Pro Ile Gly
            500                 505                 510

Asp Asp Glu Asp Ser His Leu Gly Asp Phe Ile Glu Asp Thr Thr Leu
        515                 520                 525

Glu Leu Pro Leu Asp Ser Ala Thr Thr Glu Ser Leu Arg Ala Ala Thr
    530                 535                 540

His Asp Val Leu Ala Gly Leu Thr Ala Arg Glu Ala Lys Val Leu Arg
545                 550                 555                 560

Met Arg Phe Gly Ile Asp Met Asn Thr Asp Tyr Thr Leu Glu Glu Val
                565                 570                 575

Gly Lys Gln Phe Asp Val Thr Arg Glu Arg Ile Arg Gln Ile Glu Ala
            580                 585                 590

Lys Ala Leu Arg Lys Leu Arg His Pro Ser Arg Ser Glu Val Leu Arg
        595                 600                 605

Ser Phe Leu Asp Asp
    610

<210> 26
<211> 107
<212> PRT
<213> Escherichia coli

<400> 26

Met Glu Thr Pro Ile Gly Asp Asp Glu Asp Pro His Leu Gly Asp Phe
1               5                   10                  15

Ile Glu Asp Thr Thr Leu Glu Leu Pro Leu Val Ser Ala Thr Thr Val
            20                  25                  30

Ser Leu Arg Ala Ala Thr His Asp Val Leu Ala Gly Gln Thr Ala Arg
        35                  40                  45

Glu Ala Lys Val Leu Arg Met Arg Phe Gly Val Asp Met Asn Thr Asp
    50                  55                  60

Tyr Thr Leu Glu Glu Val Gly Lys Gln Phe Asp Val Thr Arg Val Arg
65                  70                  75                  80

Ile Arg Gln Ile Glu Ala Lys Ala Leu Arg Lys Leu Arg His Pro Ser
                85                  90                  95

Arg Ser Glu Val Leu Arg Ser Phe Leu Asp Asp
            100                 105

<210>  27
<211>  2062
<212>  DNA
<213>  Escherichia coli

<400>  27
tgatttaacg gcttaagtgc cgaagagcga tcgggaagcc cccgacagcc gcactgagag      60

gcagcggcaa atatataagt acgccctcgt aattatcgtt ggcggtaaac aaccgttgga     120

tttcagcgtt aacggctgaa ggacatcggg tcaatcgccc aacaccaacc tcatgaaata     180

agtgtggata ccgtcttatg gagcaaaacc cgcagtcaca gctgaaactt cttgtcaccc     240

gtggtaagga gcaaggctat ctgacctatg ccgaggtcaa tgaccatctg ccggaagata     300

tcgtcgattc agatcagatc gaagacatca tccaaatgat caacgacatg ggcattcagg     360

tgatggaaga agcaccggat gccgatgatc tgatgctggc tgaaaacacc gcggacgaag     420

atgctgccga agccgccgcg caggtgcttt ccagcgtgga atctgaaatc gggcgcacga     480

ctgacccggt acgcatgtac atgcgtgaaa tgggcaccgt tgaactgttg acccgcgaag     540

gcgaaattga catcgctaag cgtattgaag acgggatcaa ccaggttcaa tgctccgttg     600

ctgaatatcc ggaagcgatc acctatctgc tggaacagta cgatcgtgtt gaagcagaag     660

aagcgcgtct gtccgatctg atcaccggct ttgttgaccc gaacgcagaa gaagatctgg     720

cacctaccgc cactcacgtc ggttctgagc tttcccagga agatctggac gatgacgaag     780

atgaagacga agaagatggc gatgacgaca gcgccgatga tgacaacagc atcgacccgg     840

aactggctcg cgaaaaattt gcggaactac gcgctcagta cgttgtaacg cgtgacacca     900

tcaaagcgaa aggtcgcagt cacgctaccg ctcaggaaga gatcctgaaa ctgtctgaag     960

tattcaaaca gttccgcctg gtgccgaagc agtttgacta cctggtcaac agcatgcgcg    1020

tcatgatgga ccgcgttcgt acgcaagaac gtctgatcat gaagctctgc gttgagcagt    1080

gcaaaatgcc gaagaaaaac ttcattaccc tgtttaccgg caacgaaacc agcgatacct    1140

ggttcaacgc ggcaattgcg atgaacaagc cgtggtcgga aaaactgcac gatgtctctg    1200

aagaagtgca tcgcgccctg caaaaactgc agcagattga agaagaaacc ggcctgacca    1260

tcgagcaggt taaagatatc aaccgtcgta tgtccatcgg tgaagcgaaa gcccgccgtg    1320

cgaagaaaga gatggttgaa gcgaacttac gtctggttat ttctatcgct aagaaataca    1380

ccaaccgtgg cttgcagttc cttgacctga ttcaggaagg caacatcggt ctgatgaaag 1440

cggttgataa attcgaatac cgccgtggtt acaagttctc cacctacgca acctggtgga 1500

tccgtcaggc gatcacccgc tctatcgcgg atcaggcgcg caccatccgt attccggtgc 1560

atatgattga gaccatcaac aagctcaacc gtatttctcg ccagatgctg caagagatgg 1620

gccgtgaacc gacgccggaa gaactggctg aacgtatgct gatgccggaa gacaagatcc 1680

gcaaagtgct gaagatcgcc aaagagccaa tctccatgga aacgccgatc ggtgatgatg 1740

aagattcgca tctgggggat ttcatcgagg ataccaccct cgagctgccg ctggattctg 1800

cgaccaccga aagcctgcgt gcggcaacgc acgacgtgct ggctggcctg accgcgcgtg 1860

aagcaaaagt tctgcgtatg cgtttcggta tcgatatgaa caccgactac acgctggaag 1920

aagtgggtaa acagttcgac gttacccgcg aacgtatccg tcagatcgaa gcgaaggcgc 1980

tgcgcaaact gcgtcacccg agccgttctg aagtgctgcg tagcttcctg gacgattaat 2040

cggtaggccg gatcaggcgt ta 2062

<210> 28
<211> 39
<212> DNA
<213> Artificial

<220>
<223> Primer TAF25_Sense

<400> 28
tcgagtgcta gcaaaatgga ttttgaggaa gattacgat 39

<210> 29
<211> 37
<212> DNA
<213> Artificial

<220>
<223> Primer TAF25_Anti

<400> 29
ctagcggtcg acctaacgat aaaagtctgg gcgacct 37

<210> 30
<211> 40
<212> DNA
<213> Artificial

<220>
<223> Primer SPT15_Sense

<400> 30
tcgagtgcta gcaaaatggc cgatgaggaa cgtttaaagg 40

<210>  31
<211>  40
<212>  DNA
<213>  Artificial

<220>
<223>  Primer SPT15_Anti

<400>  31
ctagcggtcg actcacattt ttctaaattc acttagcaca                              40


<210>  32
<211>  20
<212>  DNA
<213>  Artificial

<220>
<223>  Primer Seq_Forward

<400>  32
tcactcagta gaacgggagc                                                    20


<210>  33
<211>  20
<212>  DNA
<213>  Artificial

<220>
<223>  Primer Seq_Reverse

<400>  33
aatagggacc tagacttcag                                                    20


<210>  34
<211>  49
<212>  DNA
<213>  Artificial

<220>
<223>  primer HA350-F177S_sen

<400>  34
cgtctagaag ggttagcatc cagtcatggt actttctcct cctatgagc                    49


<210>  35
<211>  49
<212>  DNA
<213>  Artificial

<220>
<223>  primer HA351-F177S_ant

<400>  35
gctcatagga ggagaaagta ccatgactgg atgctaaccc ttctagacg                    49

<210>  36
<211>  41
<212>  DNA
<213>  Artificial

<220>
<223>  primer HA352-Y195H_sen

<400>  36
ccagaattgt ttcctggttt gatccataga atggtgaagc c                          41


<210>  37
<211>  41
<212>  DNA
<213>  Artificial

<220>
<223>  primer HA353-Y195H_ant

<400>  37
ggcttcacca ttctatggat caaaccagga aacaattctg g                          41


<210>  38
<211>  46
<212>  DNA
<213>  Artificial

<220>
<223>  primer HA354-K218R_sen

<400>  38
ggaaagattg ttcttactgg tgcaaggcaa agggaagaaa tttacc                     46


<210>  39
<211>  46
<212>  DNA
<213>  Artificial

<220>
<223>  primer HA355-K218R_ant

<400>  39
ggtaaatttc ttccctttgc cttgcaccag taagaacaat ctttcc                     46


<210>  40
<211>  49
<212>  DNA
<213>  Artificial

<220>
<223>  primer HA356-F177-Revert_sen

<400>  40
cgtctagaag ggttagcatt cagtcatggt actttctcct cctatgagc                  49

<210>  41
<211>  49
<212>  DNA
<213>  Artificial

<220>
<223>  primer HA357-F177-Revert_ant

<400>  41
gctcatagga ggagaaagta ccatgactga atgctaaccc ttctagacg                    49


<210>  42
<211>  41
<212>  DNA
<213>  Artificial

<220>
<223>  primer HA358-Y195H-revert_sen

<400>  42
ccagaattgt ttcctggttt gatctataga atggtgaagc c                            41


<210>  43
<211>  41
<212>  DNA
<213>  Artificial

<220>
<223>  primer HA359-Y195H-revert_ant

<400>  43
ggcttcacca ttctatagat caaaccagga aacaattctg g                            41


<210>  44
<211>  46
<212>  DNA
<213>  Artificial

<220>
<223>  primer HA360-K218R-revert_sen

<400>  44
ggaaagattg ttcttactgg tgcaaagcaa agggaagaaa tttacc                       46


<210>  45
<211>  46
<212>  DNA
<213>  Artificial

<220>
<223>  primer HA361-K218R-revert_ant

<400>  45
ggtaaatttc ttccctttgc tttgcaccag taagaacaat ctttcc                       46

<210>  46
<211>  39
<212>  DNA
<213>  Artificial

<220>
<223>  primer PHO5_sen-XhoI

<400>  46
ccgctcgagc aaaactattg tctcaataga ctggcgttg                              39


<210>  47
<211>  38
<212>  DNA
<213>  Artificial

<220>
<223>  primer PHO5_anti-XbaI

<400>  47
gctctagacc aatgtttaaa tctgttgttt attcaatt                               38


<210>  48
<211>  40
<212>  DNA
<213>  Artificial

<220>
<223>  primer PHM6_sen-SalI

<400>  48
acgcgtcgac attattaaaa caaaaacttc gtcatcgtca                             40


<210>  49
<211>  38
<212>  DNA
<213>  Artificial

<220>
<223>  primer PHM6_anti-XbaI

<400>  49
gctctagacc aagatggaag atacctcgag gtgcatcg                               38


<210>  50
<211>  39
<212>  DNA
<213>  Artificial

<220>
<223>  primer FMP16_sen-XhoI

<400>  50
ccgctcgagg tgcttcttaa taaacaccgt catctggcc                              39

<210>  51
<211>  38
<212>  DNA
<213>  Artificial

<220>
<223>  primer FMP16_anti-XbaI

<400>  51
gctctagaat aatgttgaga accacttttt tgcgcact                              38


<210>  52
<211>  240
<212>  PRT
<213>  Saccharomyces cerevisiae

<400>  52

Met Ala Asp Glu Glu Arg Leu Lys Glu Phe Lys Glu Ala Asn Lys Ile
1               5                   10                  15

Val Phe Asp Pro Asn Thr Arg Gln Val Trp Glu Asn Gln Asn Arg Asp
            20                  25                  30

Gly Thr Lys Pro Ala Thr Thr Phe Gln Ser Glu Glu Asp Ile Lys Arg
        35                  40                  45

Ala Ala Pro Glu Ser Glu Lys Asp Thr Ser Ala Thr Ser Gly Ile Val
    50                  55                  60

Pro Thr Leu Gln Asn Ile Val Ala Thr Val Thr Leu Gly Cys Arg Leu
65                  70                  75                  80

Asp Leu Lys Thr Val Ala Leu His Ala Arg Asn Ala Glu Tyr Asn Pro
                85                  90                  95

Lys Arg Phe Ala Ala Val Ile Met Arg Ile Arg Glu Pro Lys Thr Thr
            100                 105                 110

Ala Leu Ile Phe Ala Ser Gly Lys Met Val Val Thr Gly Ala Lys Ser
        115                 120                 125

Glu Asp Asp Ser Lys Leu Ala Ser Arg Lys Tyr Ala Arg Ile Ile Gln
    130                 135                 140

Lys Ile Gly Phe Ala Ala Lys Phe Thr Asp Phe Lys Ile Gln Asn Ile
145                 150                 155                 160

Val Gly Ser Cys Asp Val Lys Phe Pro Ile Arg Leu Glu Gly Leu Ala
                165                 170                 175

Phe Ser His Gly Thr Phe Ser Ser Tyr Glu Pro Glu Leu Phe Pro Gly
            180                 185                 190

Leu Ile Tyr Arg Met Val Lys Pro Lys Ile Val Leu Leu Ile Phe Val
        195                 200                 205

Ser Gly Lys Ile Val Leu Thr Gly Ala Lys Gln Arg Glu Glu Ile Tyr
    210                 215                 220

Gln Ala Phe Glu Ala Ile Tyr Pro Val Leu Ser Glu Phe Arg Lys Met
225                 230                 235                 240

<210>  53
<211>  723
<212>  DNA
<213>  Saccharomyces cerevisiae

<400>  53
atggccgatg aggaacgttt aaaggagttt aaagaggcaa acaagatagt gtttgatcca      60

aataccagac aagtatggga aaaccagaat cgagatggta caaaaccagc aactactttc     120

cagagtgaag aggacataaa aagagctgcc ccagaatctg aaaaagacac ctccgccaca     180

tcaggtattg ttccaacact acaaaacatt gtggcaactg tgactttggg gtgcaggtta     240

gatctgaaaa cagttgcgct acatgcccgt aatgcagaat ataaccccaa gcgttttgct     300

gctgtcatca tgcgtattag agagccaaaa actacagctt taatttttgc ctcagggaaa     360

atggttgtta ccggtgcaaa aagtgaggat gactcaaagc tggccagtag aaaatatgca     420

agaattatcc aaaaaatcgg gtttgctgct aaattcacag acttcaaaat acaaaatatt     480

gtcggttcgt gtgacgttaa attccctata cgtctagaag ggttagcatt cagtcatggt     540

actttctcct cctatgagcc agaattgttt cctggtttga tctatagaat ggtgaagccg     600

aaaattgtgt tgttaatttt tgtttcagga aagattgttc ttactggtgc aaagcaaagg     660

gaagaaattt accaagcttt tgaagctata taccctgtgc taagtgaatt tagaaaaatg     720

tga                                                                   723

<210>  54
<211>  723
<212>  DNA
<213>  Saccharomyces cerevisiae

<400>  54

atggccgatg aggaacgttt aaaggagttt aaagaggcaa acaagatagt gtttgatcca 60

aataccagac aagtatggga aaaccagaat cgagatggta caaaaccagc aactactttc 120

cagagtgaag aggacataaa aagagctgcc ccagaatctg aaaaagacac ctccgccaca 180

tcaggtattg ttccaacact acaaaacatt gtggcaactg tgactttggg gtgcaggtta 240

gatctgaaaa cagttgcgct acatgcccgt aatgcagaat ataaccccaa gcgttttgct 300

gctgtcatca tgcgtattag agagccaaaa actacagctt taatttttgc ctcagggaaa 360

atggttgtta ccggtgcaaa aagtgaggat gactcaaagc tggccagtag aaaatatgca 420

agaattatcc aaaaaatcgg gtttgctgct aaattcacag acttcaaaat acaaaatatt 480

gtcggttcgt gtgacgttaa attccctata cgtctagaag ggttagcatc cagtcatggt 540

actttctcct cctatgagcc agaattgttt cctggtttga tccatagaat ggtgaagccg 600

aaaattgtgt tgttaatttt tgtttcagga aagattgttc ttactggtgc aaggcaaagg 660

gaagaaattt accaagcttt tgaagctata taccctgtgc taagtgaatt tagaaaaatg 720

tga 723

<210> 55
<211> 621
<212> DNA
<213> Saccharomyces cerevisiae

<400> 55
atggattttg aggaagatta cgatgcggag tttgatgata atcaagaagg acaattagaa 60

acaccttttc catcggttgc gggagccgat gatggggaca atgataatga tgactctgtc 120

gcagaaaaca tgaagaagaa gcaaaagaga gaggctgtag tggatgatgg gagtgaaaat 180

gcatttggta tacccgaatt tacaagaaaa gataagactc tggaggagat tctagagatg 240

atggacagta ctcctcctat cattcccgat gcagtaatag actactattt aaccaaaaac 300

gggtttaacg tagcagatgt acgagtgaaa cgacttttag cacttgctac tcagaaattt 360

gttagtgata tagctaagga tgcctacgaa tattccagga tcaggtcttc cgtagcggta 420

tctaatgcta acaacagtca ggcgagagct aggcagctat tgcaaggaca gcaacagcct 480

ggcgtgcagc agatttcaca acaacaacat caacagaatg agaagactac agcaagcaaa 540

gttgttctga cggtgaacga tctcagtagc gctgttgctg aatacgggct caatataggt 600

cgcccagact tttatcgtta g 621

<210> 56
<211> 621
<212> DNA
<213> Saccharomyces cerevisiae

<400>  56
atggattttg aggaagatta cgatgcggag tttgatgata atcaagaagg acaattagaa      60

acaccttttc catcggttgc gggagccgat ggtggggaca atgataatga tgactctgtc     120

gcagaaaaca tgaagaagaa gcaaaagaga gaggctgtag aggatgatgg gagtgaaaat     180

gcatttggta tacccgaatt tacaagaaaa gataagactc tggaggagat tctagagatg     240

atggacagta ctcctcctat cattcccgat gcagtaatag actactattt aaccaaaaac     300

gggtttaacg tagcagatgt acgagtgaaa cgacttttag cacttgctac tcagaaattt     360

gttagtgata tagctaagga tgcctacgaa tattccagga tcaggtcttc cgtagcggta     420

tctaatgcta acaacagtca ggcgagagct aggcagctat tgcaaggaca gcaacagcct     480

ggcgtgcagc agatttcaca acaacaacat caacagaatg agaagactac agcaagcaga     540

gttgttctga cggtgaacga tctcagtagc gctgttgctg aatacgggct caatataggt     600

cgcccagact tttatcgtta g                                                621

SEQUENCE LISTING

<110>  MASSACHUSETTS INSTITUTE OF TECHNOLOGY
       WHITEHEAD INSTITUTE FOR BIOMEDICAL RESEARCH
       ALPER, Hal S.
       STEPHANOPOULOS, Gregory
       FINK, Gerald

<120>  GLOBAL TRANSCRIPTION MACHINERY ENGINEERING

<130>  M0656.70138WO00

<150>  US 60/873,419
<151>  2006-12-07

<160>  56

<170>  PatentIn version 3.4

<210>  1
<211>  44
<212>  DNA
<213>  Artificial

<220>
<223>  Primer CM_sense_AhdI

<400>  1
gttgcctgac tccccgtcgc caggcgttta agggcaccaa taac                        44


<210>  2
<211>  32
<212>  DNA
<213>  Artificial

<220>
<223>  Primer CM_anti_AatII

<400>  2
cagaagccac tggagcacct caaaactgca gt                                     32


<210>  3
<211>  34
<212>  DNA
<213>  Artificial

<220>
<223>  Primer pSC_sense_AflIII

<400>  3
cccacatgtc ctagacctag ctgcaggtcg agga                                   34


<210>  4
<211>  53
<212>  DNA
<213>  Artificial

<220>
<223>  Primer pSC_anti_NotI

<400>  4
aaggaaaaaa gcggccgcac gggtaagcct gttgatgata ccgctgcctt act              53

<210>  5
<211>  41
<212>  DNA
<213>  Artificial

<220>
<223>  Primer rpoD_sense_SacI

<400>  5
aacctaggag ctctgattta acggcttaag tgccgaagag c                41


<210>  6
<211>  37
<212>  DNA
<213>  Artificial

<220>
<223>  Primer rpoD_anti_HindIII

<400>  6
tggaagcttt aacgcctgat ccggcctacc gattaat                37


<210>  7
<211>  23
<212>  DNA
<213>  Artificial

<220>
<223>  Sequencing primer S1

<400>  7
ccatatgcgg tgtgaaatac cgc                23


<210>  8
<211>  24
<212>  DNA
<213>  Artificial

<220>
<223>  Sequencing primer S2

<400>  8
cacagctgaa acttcttgtc accc                24


<210>  9
<211>  22
<212>  DNA
<213>  Artificial

<220>
<223>  Sequencing primer S3

<400>  9
ttgttgaccc gaacgcagaa ga                22


<210>  10
<211>  20
<212>  DNA

<213>  Artificial

<220>
<223>  Sequencing primer S4

<400>  10
agaaaccggc ctgaccatcg                                                    20


<210>  11
<211>  24
<212>  DNA
<213>  Artificial

<220>
<223>  Sequencing primer A1

<400>  11
gcttcgatct gacggatacg ttcg                                               24


<210>  12
<211>  24
<212>  DNA
<213>  Artificial

<220>
<223>  Sequencing primer A2

<400>  12
caggttgcgt aggtggagaa cttg                                               24


<210>  13
<211>  22
<212>  DNA
<213>  Artificial

<220>
<223>  Sequencing primer A3

<400>  13
gtgactgcga cctttcgctt tg                                                 22


<210>  14
<211>  20
<212>  DNA
<213>  Artificial

<220>
<223>  Sequencing primer A4

<400>  14
catcagatca tcggcatccg                                                    20


<210>  15
<211>  19
<212>  DNA
<213>  Artificial

<220>
<223>  Sequencing primer A5

<400>  15
gcttcggcag catcttcgt                                                    19


<210>  16
<211>  21
<212>  DNA
<213>  Artificial

<220>
<223>  Sequencing primer A6

<400>  16
cggaagcgat cacctatctg c                                                 21


<210>  17
<211>  613
<212>  PRT
<213>  Escherichia coli

<400>  17

Met Glu Gln Asn Pro Gln Ser Gln Leu Lys Leu Leu Val Thr Arg Gly
1               5                   10                  15


Lys Glu Gln Gly Tyr Leu Thr Tyr Ala Glu Val Asn Asp His Leu Pro
            20                  25                  30


Glu Asp Ile Val Asp Ser Asp Gln Ile Glu Asp Ile Ile Gln Met Ile
        35                  40                  45


Asn Asp Met Gly Ile Gln Val Met Glu Glu Ala Pro Asp Ala Asp Asp
    50                  55                  60


Leu Met Leu Ala Glu Asn Thr Ala Asp Glu Asp Ala Ala Glu Ala Ala
65                  70                  75                  80


Ala Gln Val Leu Ser Ser Val Glu Ser Glu Ile Gly Arg Thr Thr Asp
                85                  90                  95


Pro Val Arg Met Tyr Met Arg Glu Met Gly Thr Val Glu Leu Leu Thr
            100                 105                 110


Arg Glu Gly Glu Ile Asp Ile Ala Lys Arg Ile Glu Asp Gly Ile Asn
        115                 120                 125


Gln Val Gln Cys Ser Val Ala Glu Tyr Pro Glu Ala Ile Thr Tyr Leu
    130                 135                 140


Leu Glu Gln Tyr Asp Arg Val Glu Ala Glu Glu Ala Arg Leu Ser Asp
145                 150                 155                 160


Leu Ile Thr Gly Phe Val Asp Pro Asn Ala Glu Glu Asp Leu Ala Pro
                165                 170                 175

Thr Ala Thr His Val Gly Ser Glu Leu Ser Gln Glu Asp Leu Asp Asp
            180                 185                 190

Asp Glu Asp Glu Asp Glu Glu Asp Gly Asp Asp Asp Ser Ala Asp Asp
        195                 200                 205

Asp Asn Ser Ile Asp Pro Glu Leu Ala Arg Glu Lys Phe Ala Glu Leu
    210                 215                 220

Arg Ala Gln Tyr Val Val Thr Arg Asp Thr Ile Lys Ala Lys Gly Arg
225                 230                 235                 240

Ser His Ala Thr Ala Gln Glu Glu Ile Leu Lys Leu Ser Glu Val Phe
                245                 250                 255

Lys Gln Phe Arg Leu Val Pro Lys Gln Phe Asp Tyr Leu Val Asn Ser
            260                 265                 270

Met Arg Val Met Met Asp Arg Val Arg Thr Gln Glu Arg Leu Ile Met
        275                 280                 285

Lys Leu Cys Val Glu Gln Cys Lys Met Pro Lys Lys Asn Phe Ile Thr
    290                 295                 300

Leu Phe Thr Gly Asn Glu Thr Ser Asp Thr Trp Phe Asn Ala Ala Ile
305                 310                 315                 320

Ala Met Asn Lys Pro Trp Ser Glu Lys Leu His Asp Val Ser Glu Glu
                325                 330                 335

Val His Arg Ala Leu Gln Lys Leu Gln Gln Ile Glu Glu Glu Thr Gly
            340                 345                 350

Leu Thr Ile Glu Gln Val Lys Asp Ile Asn Arg Arg Met Ser Ile Gly
        355                 360                 365

Glu Ala Lys Ala Arg Arg Ala Lys Lys Glu Met Val Glu Ala Asn Leu
    370                 375                 380

Arg Leu Val Ile Ser Ile Ala Lys Lys Tyr Thr Asn Arg Gly Leu Gln
385                 390                 395                 400

Phe Leu Asp Leu Ile Gln Glu Gly Asn Ile Gly Leu Met Lys Ala Val
                405                 410                 415

Asp Lys Phe Glu Tyr Arg Arg Gly Tyr Lys Phe Ser Thr Tyr Ala Thr
            420                 425                 430

Trp Trp Ile Arg Gln Ala Ile Thr Arg Ser Ile Ala Asp Gln Ala Arg
        435                 440                 445

Thr Ile Arg Ile Pro Val His Met Ile Glu Thr Ile Asn Lys Leu Asn
    450                 455                 460

Arg Ile Ser Arg Gln Met Leu Gln Glu Met Gly Arg Glu Pro Thr Pro
465                 470                 475                 480

Glu Glu Leu Ala Glu Arg Met Leu Met Pro Glu Asp Lys Ile Arg Lys
                485                 490                 495

Val Leu Lys Ile Ala Lys Glu Pro Ile Ser Met Glu Thr Pro Ile Gly
            500                 505                 510

Asp Asp Glu Asp Ser His Leu Gly Asp Phe Ile Glu Asp Thr Thr Leu
        515                 520                 525

Glu Leu Pro Leu Asp Ser Ala Thr Thr Glu Ser Leu Arg Ala Ala Thr
    530                 535                 540

His Asp Val Leu Ala Gly Leu Thr Ala Arg Glu Ala Lys Val Leu Arg
545                 550                 555                 560

Met Arg Phe Gly Ile Asp Met Asn Thr Asp Tyr Thr Leu Glu Glu Val
                565                 570                 575

Gly Lys Gln Phe Asp Val Thr Arg Glu Arg Ile Arg Gln Ile Glu Ala
            580                 585                 590

Lys Ala Leu Arg Lys Leu Arg His Pro Ser Arg Ser Glu Val Leu Arg
        595                 600                 605

Ser Phe Leu Asp Asp
    610

<210>  18
<211>  612
<212>  PRT
<213>  Escherichia coli

<400>  18

Met Glu Gln Asn Pro Gln Ser Gln Leu Lys Leu Leu Val Thr Arg Gly
1               5                   10                  15

Lys Glu Gln Gly Tyr Leu Thr Tyr Ala Glu Val Asn Asp Leu Leu Pro
            20                  25                  30

Glu Asp Ile Val Asp Asp Gln Ile Glu Asp Ile Ile Gln Met Ile Asn

35                  40                  45

Asp Met Gly Ile Gln Val Met Glu Glu Ala Ser Asp Ala Asp Asp Leu
    50                  55                  60

Met Leu Ala Glu Asn Thr Ala Asp Glu Asp Ala Ala Glu Ala Ala Ala
65                  70                  75                  80

Gln Val Leu Ser Ser Val Glu Ser Glu Ile Gly Arg Thr Ile Asp Pro
                85                  90                  95

Val Arg Met Tyr Met Arg Glu Met Gly Thr Val Glu Leu Leu Thr Arg
            100                 105                 110

Glu Gly Glu Ile Asp Ile Ala Lys Arg Ile Glu Asp Gly Ile Asn Gln
        115                 120                 125

Val Gln Cys Ser Val Ala Glu Tyr Pro Glu Ala Ile Thr Tyr Leu Leu
    130                 135                 140

Glu Gln Tyr Asp Arg Val Glu Ala Glu Glu Ala Arg Leu Ser Asp Leu
145                 150                 155                 160

Ile Thr Gly Phe Val Asp Pro Asn Ala Glu Glu Asp Leu Ala Pro Thr
                165                 170                 175

Ala Thr His Val Gly Ser Glu Leu Ser Gln Glu Asp Leu Asp Asp Asp
            180                 185                 190

Glu Asp Glu Asp Glu Glu Asp Gly Asp Asp Gly Ser Ala Asp Asp Asp
        195                 200                 205

Asn Ser Ile Asp Pro Glu Leu Ala Arg Glu Lys Leu Ala Glu Leu Arg
    210                 215                 220

Ala Gln Tyr Val Val Thr Arg Asp Thr Ile Lys Ala Lys Gly Arg Ser
225                 230                 235                 240

His Ala Thr Ala Gln Glu Glu Phe Leu Lys Leu Ser Glu Val Phe Lys
                245                 250                 255

Gln Phe Arg Leu Val Pro Lys Gln Phe Asp Tyr Leu Val Asn Ser Met
            260                 265                 270

Arg Val Met Met Asp Arg Val Arg Thr Gln Glu Arg Leu Val Met Lys
        275                 280                 285

Leu Cys Val Glu Gln Cys Lys Met Pro Lys Lys Asn Phe Ile Thr Leu
    290                 295                 300

Phe Thr Gly Asn Glu Thr Ser Asp Thr Trp Phe Asn Ala Ala Ile Ala
305                 310                 315                 320

Met Asn Lys Pro Trp Ser Glu Lys Leu His Asp Val Ser Glu Glu Val
                325                 330                 335

His Arg Ala Leu Gln Lys Leu Gln Gln Ile Glu Glu Glu Thr Gly Leu
            340                 345                 350

Thr Ile Glu Gln Val Lys Asp Ile Asn Arg Arg Met Ser Ile Gly Glu
        355                 360                 365

Ala Lys Ala Arg Arg Ala Lys Lys Glu Met Val Glu Ala Asn Leu Arg
    370                 375                 380

Leu Val Ile Ser Ile Ala Lys Lys Tyr Thr Asn Arg Gly Leu Gln Phe
385                 390                 395                 400

Leu Asp Leu Ile Gln Glu Gly Asn Ile Gly Leu Met Lys Ala Val Asp
                405                 410                 415

Lys Phe Glu Tyr Arg Arg Gly Tyr Lys Phe Ser Thr Tyr Ala Thr Trp
            420                 425                 430

Trp Ile Arg Gln Ala Ile Thr Arg Ser Ile Ala Asp Gln Ala Arg Thr
        435                 440                 445

Ile Arg Ile Pro Val His Met Ile Glu Thr Ile Asn Lys Leu Asn Arg
    450                 455                 460

Ile Ser Arg Gln Met Leu Gln Glu Met Gly Arg Glu Pro Thr Pro Glu
465                 470                 475                 480

Glu Leu Ala Glu Arg Met Leu Met Pro Glu Asp Lys Ile Arg Lys Val
                485                 490                 495

Leu Lys Ile Ala Lys Glu Pro Ile Ser Met Glu Thr Pro Val Gly Asp
            500                 505                 510

Asp Glu Asp Ser His Leu Gly Asp Phe Ile Glu Asp Thr Thr Leu Glu
        515                 520                 525

Leu Pro Leu Asp Ser Ala Thr Thr Glu Ser Leu Arg Ala Ala Thr His
    530                 535                 540

Asp Val Leu Ala Gly Leu Thr Ala Arg Glu Ala Lys Val Leu Arg Met
545                 550                 555                 560

Arg Phe Gly Ile Asp Met Asn Thr Asp Tyr Thr Leu Glu Glu Val Gly
                565                 570                 575

Lys Gln Phe Asp Val Thr Arg Glu Arg Ile Arg Gln Ile Glu Ala Lys
            580                 585                 590

Ala Leu Arg Lys Leu Arg His Pro Ser Cys Ser Glu Val Leu Arg Ser
        595                 600                 605

Phe Leu Asp Asp
    610

<210>  19
<211>  107
<212>  PRT
<213>  Escherichia coli

<400>  19

Met Glu Thr Pro Ile Gly Asp Asp Glu Asp Ser His Leu Gly Asp Phe
1               5                   10                  15

Ile Glu Asp Thr Thr Leu Glu Leu Pro Leu Asp Ser Ala Thr Thr Glu
            20                  25                  30

Ser Leu Arg Ala Ala Thr His Asp Val Leu Ala Gly Leu Thr Ala Arg
        35                  40                  45

Glu Ala Lys Val Leu Arg Met Arg Phe Gly Ile Asp Met Asn Thr Asp
    50                  55                  60

Tyr Thr Leu Glu Glu Val Gly Lys Gln Phe Asp Val Thr Arg Glu Arg
65                  70                  75                  80

Ile Arg Gln Ile Glu Ala Lys Ala Leu Arg Lys Leu Arg His Pro Ser
                85                  90                  95

Cys Ser Glu Val Leu Arg Ser Phe Leu Asp Asp
            100                 105

<210>  20
<211>  107
<212>  PRT
<213>  Escherichia coli

<400>  20

Met Glu Thr Pro Ile Gly Asp Asp Glu Asp Ser Leu Leu Gly Asp Phe
1               5                   10                  15

Ile Glu Asp Thr Thr Leu Glu Leu Pro Leu Asp Ser Ala Thr Thr Val
            20                  25                  30

Ser Leu Arg Val Ala Thr His Asp Val Leu Ala Gly Leu Thr Ala Arg
        35                  40                  45

Val Ala Lys Val Leu Arg Met Arg Phe Gly Ile Asn Met Asn Thr Asp
    50                  55                  60

Tyr Thr Leu Glu Glu Val Gly Lys Gln Phe Asp Val Thr Arg Glu Arg
65                  70                  75                  80

Ile Arg Gln Ile Glu Ala Lys Ala Leu Arg Lys Leu Arg His Pro Ser
                85                  90                  95

Arg Ser Gly Val Gln Arg Ser Phe Leu Asp Asp
            100                 105

<210>  21
<211>  107
<212>  PRT
<213>  Escherichia coli

<400>  21

Met Glu Thr Pro Ile Gly Asp Asp Glu Asp Ser His Leu Gly Asp Tyr
1               5                   10                  15

Ile Glu Asp Thr Thr Leu Glu Leu Pro Leu Asp Ser Ala Thr Thr Glu
            20                  25                  30

Ser Leu Arg Ala Ala Thr His Asp Val Leu Ala Gly Met Thr Ala Arg
        35                  40                  45

Glu Ala Lys Val Leu Arg Met Arg Phe Gly Ile Asp Val Asn Thr Asp
    50                  55                  60

Tyr Thr Leu Glu Glu Val Gly Lys Gln Phe Asp Val Thr Arg Glu Arg
65                  70                  75                  80

Ile Ser Gln Ile Glu Ala Lys Ala Leu Arg Lys Leu Arg His Pro Ser
                85                  90                  95

Arg Ser Glu Val Leu Arg Ser Phe Leu Asp Asp
            100                 105

<210>  22
<211>  612
<212>  PRT
<213>  Escherichia coli

<400>  22

Met Glu Gln Asn Pro Gln Ser Gln Leu Lys Leu Leu Val Thr Arg Gly

1               5                   10                  15

Lys Glu Gly Tyr Leu Thr Tyr Ala Glu Val Asn Asp His Leu Pro Glu
            20                  25                  30

Asp Ile Val Asp Ser Asp Gln Ile Glu Asp Ile Ile Gln Met Ile Asn
        35                  40                  45

Asp Met Gly Ile Gln Val Met Glu Glu Ala Pro Asp Ala Asp Asp Leu
    50                  55                  60

Met Leu Ala Glu Asn Thr Ala Asp Glu Asp Ala Ala Glu Ala Ala Ala
65                  70                  75                  80

Gln Val Leu Ser Ser Val Glu Ser Gly Val Gly Arg Thr Thr Asp Pro
                85                  90                  95

Val Arg Met Tyr Met Arg Glu Met Gly Thr Val Glu Leu Leu Thr Arg
            100                 105                 110

Glu Gly Glu Ile Asp Ile Ala Lys Arg Ile Glu Asp Gly Asn Asn Gln
        115                 120                 125

Val Gln Cys Ser Val Ala Glu Tyr Pro Glu Ala Ile Thr Tyr Leu Leu
    130                 135                 140

Glu Gln Tyr Asp Arg Ala Glu Ala Glu Glu Ala Arg Leu Ser Asp Met
145                 150                 155                 160

Ile Thr Gly Phe Val Asp Pro Asn Ala Glu Glu Asp Leu Ala Pro Thr
                165                 170                 175

Ala Thr His Val Gly Ser Glu Leu Ser Gln Glu Asp Leu Asp Asp Asp
            180                 185                 190

Glu Asp Glu Asp Glu Val Asp Gly Asp Asp Asp Ser Ala Asp Asp Asp
        195                 200                 205

Asn Ser Ile Asp Pro Glu Leu Ala Arg Glu Lys Phe Ala Glu Leu Arg
    210                 215                 220

Ala Gln Tyr Val Val Thr Arg Asp Thr Ile Lys Ala Lys Gly Arg Ser
225                 230                 235                 240

His Ala Thr Ala Gln Glu Glu Ile Leu Lys Leu Ser Glu Val Phe Lys
                245                 250                 255

Gln Phe Arg Leu Val Pro Lys Gln Phe Asp Tyr Leu Val Asn Ser Met
            260                 265                 270

Arg Val Met Met Asp His Val Arg Thr Gln Glu Arg Leu Ile Met Lys
        275                 280                 285

Leu Cys Val Glu Gln Cys Lys Met Pro Lys Lys Asn Phe Ile Thr Leu
    290                 295                 300

Phe Thr Gly Asn Glu Thr Ser Asp Thr Trp Phe Asn Ala Ala Ile Ala
305                 310                 315                 320

Met Asn Arg Pro Trp Ser Glu Lys Leu His Asp Val Ser Glu Glu Val
                325                 330                 335

His Arg Ala Leu Gln Lys Leu Gln Gln Ile Glu Glu Glu Thr Gly Leu
            340                 345                 350

Thr Ile Glu Gln Val Lys Asp Ile Asn Arg Arg Met Ser Ile Gly Glu
        355                 360                 365

Ala Lys Ala Arg Arg Ala Lys Lys Glu Met Val Glu Ala Asn Leu Arg
    370                 375                 380

Leu Val Ile Ser Ile Ala Lys Lys Tyr Thr Asn Arg Gly Leu Gln Phe
385                 390                 395                 400

Leu Asp Leu Ile Gln Glu Gly Asn Ile Gly Leu Met Lys Ala Val Asp
                405                 410                 415

Lys Phe Glu Tyr Arg Arg Gly Tyr Lys Phe Ser Thr Tyr Ala Thr Trp
            420                 425                 430

Trp Ile Arg Gln Ala Ile Thr Arg Ser Ile Ala Asp Gln Ala Arg Thr
        435                 440                 445

Ile Arg Ile Pro Val His Met Ile Glu Thr Ile Asn Lys Leu Asn Arg
    450                 455                 460

Ile Ser Arg Gln Met Leu Gln Glu Met Gly Arg Glu Pro Thr Pro Glu
465                 470                 475                 480

Glu Leu Ala Glu Arg Met Leu Met Pro Glu Asp Lys Ile Arg Lys Val
                485                 490                 495

Leu Lys Ile Ala Lys Glu Pro Ile Ser Met Glu Thr Pro Ile Gly Asp
            500                 505                 510

Asp Glu Asp Ser His Leu Gly Asp Phe Ile Glu Asp Thr Thr Leu Glu
        515                 520                 525

Leu Pro Leu Asp Ser Ala Thr Thr Glu Ser Leu Arg Ala Ala Thr His
    530                 535                 540

Asp Val Leu Ala Gly Leu Thr Ala Arg Glu Ala Lys Val Leu Arg Met
545                 550                 555                 560

Arg Phe Gly Ile Asp Met Asn Thr Asp Tyr Thr Leu Glu Glu Val Gly
                565                 570                 575

Lys Gln Phe Asp Val Thr Arg Glu Arg Ile Arg Gln Ile Glu Ala Lys
            580                 585                 590

Ala Leu Arg Lys Leu Arg His Pro Ser Arg Ser Glu Val Leu Arg Ser
        595                 600                 605

Phe Leu Asp Asp
    610

<210>  23
<211>  613
<212>  PRT
<213>  Escherichia coli

<400>  23

Met Asp Gln Asn Pro Gln Ser Gln Leu Lys Leu Leu Val Thr Arg Gly
1               5                   10                  15

Lys Glu Gln Gly Tyr Leu Thr Tyr Ala Glu Val Asn Asp His Leu Pro
            20                  25                  30

Glu Asp Ile Val Asp Ser Asp Gln Ile Glu Asp Ile Ile Gln Met Ile
        35                  40                  45

Asn Asp Met Gly Ile Gln Val Met Glu Glu Ala Pro Asp Ala Asp Asp
    50                  55                  60

Leu Met Leu Ala Glu Asn Thr Ala Asp Glu Asp Ala Ala Glu Ala Val
65                  70                  75                  80

Ala Gln Val Leu Ser Ser Val Glu Ser Glu Ile Gly Arg Thr Thr Asp
                85                  90                  95

Pro Val Arg Met Tyr Met Arg Glu Met Gly Thr Val Glu Leu Leu Thr
            100                 105                 110

Arg Glu Gly Glu Ile Asp Ile Ala Lys Arg Ile Glu Asp Gly Ile Asn
        115                 120                 125

Gln Val Gln Cys Ser Val Ala Glu Tyr Pro Glu Ala Ile Thr Tyr Leu

130                 135                 140

Leu Glu Gln Tyr Asp Arg Val Glu Ala Glu Glu Ala Arg Leu Ser Asp
145                 150                 155                 160

Leu Ile Thr Gly Phe Val Asp Pro Asn Ala Glu Glu Asp Leu Ala Pro
                165                 170                 175

Thr Ala Thr His Val Gly Ser Glu Pro Ser Gln Glu Asp Leu Asp Asp
            180                 185                 190

Asp Glu Asp Glu Asp Glu Glu Asp Gly Asp Asp Asp Ser Ala Asp Asp
        195                 200                 205

Asp Asn Ser Ile Asp Pro Glu Leu Ala Arg Glu Lys Phe Ala Glu Leu
    210                 215                 220

Arg Ala Gln Tyr Val Val Thr Arg Asp Thr Ile Lys Ala Lys Gly Arg
225                 230                 235                 240

Ser His Ala Thr Ala Gln Glu Glu Ile Leu Lys Leu Ser Glu Val Phe
                245                 250                 255

Lys Gln Phe Arg Leu Val Pro Lys Gln Phe Asp Tyr Leu Val Asn Ser
            260                 265                 270

Met Arg Val Met Met Asp Arg Val Arg Thr Gln Glu Arg Leu Ile Met
        275                 280                 285

Lys Leu Cys Val Glu Gln Cys Lys Met Pro Lys Lys Asn Phe Ile Thr
    290                 295                 300

Leu Phe Thr Gly Asn Glu Thr Ser Asp Thr Trp Phe Asn Ala Ala Ile
305                 310                 315                 320

Ala Met Asn Lys Leu Trp Ser Glu Lys Leu His Asp Val Ser Glu Glu
                325                 330                 335

Val His Arg Ala Leu Gln Lys Leu Arg Gln Ile Glu Glu Glu Thr Gly
            340                 345                 350

Leu Thr Ile Glu Gln Val Lys Asp Ile Asn Arg Arg Met Ser Ile Gly
        355                 360                 365

Glu Ala Lys Ala Arg Arg Ala Lys Lys Glu Met Val Glu Ala Asn Leu
    370                 375                 380

Arg Leu Val Ile Ser Ile Ala Lys Lys Tyr Thr Asn Arg Gly Leu Gln
385                 390                 395                 400

Phe Leu Asp Leu Ile Gln Glu Gly Asn Ile Gly Leu Met Lys Ala Val
                405                 410                 415

Asp Lys Phe Glu Tyr Arg Arg Gly Tyr Lys Phe Ser Thr Tyr Ala Thr
            420                 425                 430

Trp Trp Ile Arg Gln Ala Ile Thr Arg Phe Ile Ala Asp Gln Ala Arg
        435                 440                 445

Thr Ile Arg Ile Pro Val His Met Ile Glu Thr Ile Asn Lys Leu Asn
    450                 455                 460

Arg Ile Ser Arg Gln Met Leu Gln Glu Met Gly Arg Glu Pro Thr Pro
465                 470                 475                 480

Glu Glu Leu Ala Glu Arg Met Leu Met Pro Glu Asp Lys Ile Arg Lys
                485                 490                 495

Val Leu Lys Ile Ala Lys Glu Pro Ile Ser Met Glu Thr Pro Ile Gly
            500                 505                 510

Asp Asp Glu Asp Ser His Leu Gly Asp Phe Ile Glu Asp Thr Thr Leu
        515                 520                 525

Glu Leu Pro Leu Asp Ser Ala Thr Thr Glu Ser Leu Arg Ala Ala Thr
    530                 535                 540

His Asp Val Leu Ala Gly Leu Thr Ala Arg Glu Ala Lys Val Leu Arg
545                 550                 555                 560

Met Arg Phe Gly Ile Asp Val Asn Thr Asp Tyr Thr Leu Glu Glu Val
                565                 570                 575

Ser Lys Gln Phe Asn Val Thr Arg Glu Arg Ile Arg Gln Ile Glu Ala
            580                 585                 590

Lys Ala Leu Arg Lys Leu Arg His Pro Ser Arg Ser Asp Val Leu Arg
        595                 600                 605

Ser Phe Leu Asp Asp
    610

<210>  24
<211>  611
<212>  PRT
<213>  Escherichia coli

<400>  24

Met Glu Gln Asn Pro Gln Ser Gln Leu Lys Leu Leu Val Thr Arg Gly
1               5                   10                  15

Lys Glu Gln Gly Tyr Leu Thr Tyr Ala Glu Val Asn Asp His Leu Pro
            20                  25                  30

Glu Asp Ile Val Asp Ser Asp Gln Ile Glu Asp Ile Ile Gln Met Ile
        35                  40                  45

Asn Asp Met Gly Ile Gln Val Met Glu Glu Ala Pro Asp Ala Asp Asp
    50                  55                  60

Leu Met Leu Ala Glu Asn Thr Ala Asp Glu Asp Ala Ala Glu Ala Ala
65                  70                  75                  80

Ala Gln Val Leu Ser Ser Val Glu Ser Glu Ile Gly Arg Thr Thr Asp
                85                  90                  95

Pro Val Arg Met Tyr Met Arg Glu Met Gly Thr Val Glu Leu Leu Thr
            100                 105                 110

Arg Glu Gly Glu Ile Asp Ile Ala Lys Arg Ile Glu Asp Gly Val Asn
        115                 120                 125

Gln Val Gln Cys Ser Val Ala Glu Tyr Pro Glu Ala Ile Thr Tyr Leu
    130                 135                 140

Leu Glu Gln Tyr Asp Arg Val Glu Ala Glu Glu Ala Arg Leu Ser Asp
145                 150                 155                 160

Leu Ile Thr Gly Phe Val Asp Pro Asn Ala Glu Glu Asp Leu Ala Pro
                165                 170                 175

Thr Ala Thr His Val Gly Ser Glu Leu Ser Gln Glu Asp Leu Asp Asp
            180                 185                 190

Asp Glu Asp Glu Asp Glu Glu Asp Gly Asp Asp Asp Ser Ala Asp Asp
        195                 200                 205

Asp Asn Ser Ile Asp Pro Glu Leu Ala Arg Glu Lys Phe Ala Glu Leu
    210                 215                 220

Arg Ala Gln Tyr Val Val Thr Arg Asp Thr Ile Ala Lys Gly Arg Ser
225                 230                 235                 240

His Ala Thr Ala Gln Glu Glu Ile Leu Lys Leu Ser Glu Val Phe Lys
                245                 250                 255

Gln Phe Arg Leu Val Pro Lys Gln Phe Asp Tyr Leu Val Asn Ser Met

260                 265                 270

Arg Val Met Met Asp Arg Val Arg Thr Gln Glu Arg Leu Ile Met Lys
        275                 280                 285

Leu Cys Val Glu Gln Cys Lys Met Pro Lys Lys Asn Phe Ile Thr Leu
    290                 295                 300

Phe Thr Gly Asn Glu Thr Ser Asp Thr Trp Phe Asn Ala Ala Ile Ala
305                 310                 315                 320

Met Asn Lys Pro Trp Ser Glu Lys Leu His Asp Val Ser Glu Glu Val
                325                 330                 335

His Arg Ala Leu Gln Lys Leu Gln Gln Ile Glu Glu Glu Thr Gly Leu
            340                 345                 350

Thr Ile Glu Gln Val Lys Asp Ile Asn Arg Arg Met Ser Ile Gly Glu
        355                 360                 365

Ala Lys Ala Arg Arg Ala Lys Lys Glu Met Val Glu Ala Asn Leu Arg
    370                 375                 380

Leu Val Ile Ser Ile Ala Lys Lys Phe Thr Asn Arg Gly Leu Gln Phe
385                 390                 395                 400

Leu Asp Leu Ile Gln Glu Gly Asn Ile Gly Leu Val Lys Ala Val Asp
                405                 410                 415

Lys Phe Glu Tyr Arg Arg Gly Tyr Lys Phe Ser Thr Tyr Ala Thr Trp
            420                 425                 430

Trp Ile Arg Gln Ala Ile Thr Arg Ser Ile Ala Asp Gln Ala Arg Thr
        435                 440                 445

Ile Arg Ile Pro Val His Met Ile Glu Thr Ile Asn Leu Asn Arg Ile
    450                 455                 460

Ser Arg Gln Met Leu Gln Glu Met Gly Arg Glu Pro Thr Pro Glu Glu
465                 470                 475                 480

Leu Ala Glu Arg Met Leu Met Gln Glu Asp Lys Ile Arg Lys Val Leu
                485                 490                 495

Lys Ile Ala Asn Glu Pro Ile Ser Met Glu Thr Pro Ile Gly Asp Asp
            500                 505                 510

Glu Asp Ser His Leu Gly Asp Phe Ile Glu Asp Thr Thr Leu Glu Leu
        515                 520                 525

Pro Leu Asp Ser Ala Thr Thr Glu Ser Leu Arg Ala Ala Thr His Asp
    530                 535                 540

Val Leu Ala Gly Leu Thr Ala Arg Glu Ala Lys Val Leu Arg Met Arg
545                 550                 555                 560

Phe Gly Ile Asp Met Asn Thr Asp Tyr Thr Leu Glu Glu Val Gly Lys
                565                 570                 575

Gln Phe Asp Val Thr Arg Glu Arg Ile Arg Gln Ile Glu Ala Lys Ala
            580                 585                 590

Leu Arg Lys Leu Arg His Pro Ser Arg Pro Glu Val Leu Arg Ser Phe
        595                 600                 605

Leu Asp Asp
    610

<210>  25
<211>  613
<212>  PRT
<213>  Escherichia coli

<400>  25

Met Glu Gln Asn Pro Gln Ser Gln Leu Lys Leu Leu Val Thr Arg Gly
1               5                   10                  15

Lys Glu Gln Gly Tyr Leu Thr Tyr Ala Glu Val Asn Asp His Leu Pro
            20                  25                  30

Glu Asp Ile Val Asp Ser Asp Gln Ile Glu Asp Ile Ile Gln Met Ile
        35                  40                  45

Asn Asp Met Gly Ile Gln Val Met Glu Glu Ala Pro Asp Ala Asp Asp
    50                  55                  60

Leu Met Leu Ala Glu Asn Thr Ala Asp Glu Asp Ala Ala Glu Ala Ala
65                  70                  75                  80

Ala Gln Val Leu Ser Ser Val Glu Ser Glu Ile Gly Arg Thr Thr Asp
                85                  90                  95

Pro Val Arg Met Tyr Met Arg Glu Met Gly Thr Val Glu Leu Leu Thr
            100                 105                 110

Arg Glu Gly Glu Ile Asp Ile Ala Lys Arg Ile Glu Asp Gly Ile Asn
        115                 120                 125

Gln Val Gln Cys Ser Val Ala Glu Tyr Pro Glu Ala Ile Thr Tyr Leu
    130                 135                 140

Leu Glu Gln Tyr Asp Arg Val Glu Ala Glu Glu Ala Arg Leu Ser Asp
145                 150                 155                 160

Leu Ile Thr Gly Phe Val Asp Pro Asn Ala Glu Glu Asp Leu Ala Pro
                165                 170                 175

Thr Ala Thr His Val Gly Ser Glu Leu Ser Gln Glu Asp Leu Asp Asp
            180                 185                 190

Asp Glu Asp Glu Asp Glu Glu Asp Gly Asp Asp Asp Ser Ala Asp Asp
        195                 200                 205

Asp Asn Ser Ile Asp Pro Glu Leu Ala Arg Glu Lys Phe Ala Glu Leu
    210                 215                 220

Arg Ala Gln Tyr Val Val Thr Arg Asp Thr Ile Lys Ala Lys Gly Arg
225                 230                 235                 240

Ser His Ala Thr Ala Gln Glu Glu Ile Leu Lys Leu Ser Glu Val Phe
                245                 250                 255

Lys Gln Phe Arg Leu Val Pro Lys Gln Phe Asp Tyr Leu Val Asn Ser
            260                 265                 270

Met Arg Val Met Met Asp Arg Val Arg Thr Gln Glu Arg Leu Ile Met
        275                 280                 285

Lys Leu Cys Val Glu Gln Cys Lys Met Pro Lys Lys Asn Phe Ile Thr
    290                 295                 300

Leu Phe Thr Gly Asn Glu Thr Ser Asp Thr Trp Phe Asn Ala Ala Ile
305                 310                 315                 320

Ala Met Asn Lys Pro Trp Ser Glu Lys Leu His Asp Val Ser Glu Glu
                325                 330                 335

Val His Arg Ala Leu Gln Lys Leu Gln Gln Ile Glu Glu Glu Thr Gly
            340                 345                 350

Leu Thr Ile Glu Gln Val Lys Asp Ile Asn Arg Arg Met Ser Ile Gly
        355                 360                 365

Glu Ala Lys Ala Arg Arg Ala Lys Lys Glu Met Val Glu Ala Asn Leu
    370                 375                 380

Arg Leu Val Ile Ser Ile Ala Lys Lys Tyr Thr Asn Arg Gly Leu Gln

385                 390                 395                 400

Phe Leu Asp Leu Ile Gln Glu Gly Tyr Ile Gly Leu Met Lys Ala Val
                405                 410                 415

Asp Lys Phe Glu Tyr Arg Arg Gly Tyr Lys Phe Ser Thr Tyr Ala Thr
            420                 425                 430

Trp Trp Ile Arg Gln Ala Ile Thr Arg Ser Ile Ala Asp Gln Ala Arg
        435                 440                 445

Thr Ile Arg Ile Pro Val His Met Ile Glu Thr Ile Asp Lys Leu Asn
    450                 455                 460

Arg Ile Ser Arg Gln Met Leu Gln Glu Met Gly Arg Glu Pro Thr Pro
465                 470                 475                 480

Glu Glu Leu Ala Glu Arg Met Leu Met Pro Glu Asp Lys Ile Arg Lys
                485                 490                 495

Val Leu Lys Ile Ala Lys Glu Pro Ile Ser Met Glu Thr Pro Ile Gly
            500                 505                 510

Asp Asp Glu Asp Ser His Leu Gly Asp Phe Ile Glu Asp Thr Thr Leu
        515                 520                 525

Glu Leu Pro Leu Asp Ser Ala Thr Thr Glu Ser Leu Arg Ala Ala Thr
    530                 535                 540

His Asp Val Leu Ala Gly Leu Thr Ala Arg Glu Ala Lys Val Leu Arg
545                 550                 555                 560

Met Arg Phe Gly Ile Asp Met Asn Thr Asp Tyr Thr Leu Glu Glu Val
                565                 570                 575

Gly Lys Gln Phe Asp Val Thr Arg Glu Arg Ile Arg Gln Ile Glu Ala
            580                 585                 590

Lys Ala Leu Arg Lys Leu Arg His Pro Ser Arg Ser Glu Val Leu Arg
        595                 600                 605

Ser Phe Leu Asp Asp
    610

<210>  26
<211>  107
<212>  PRT
<213>  Escherichia coli

<400>  26

Met Glu Thr Pro Ile Gly Asp Asp Glu Asp Pro His Leu Gly Asp Phe
1               5                   10                  15

Ile Glu Asp Thr Thr Leu Glu Leu Pro Leu Val Ser Ala Thr Thr Val
            20                  25                  30

Ser Leu Arg Ala Ala Thr His Asp Val Leu Ala Gly Gln Thr Ala Arg
        35                  40                  45

Glu Ala Lys Val Leu Arg Met Arg Phe Gly Val Asp Met Asn Thr Asp
    50                  55                  60

Tyr Thr Leu Glu Glu Val Gly Lys Gln Phe Asp Val Thr Arg Val Arg
65                  70                  75                  80

Ile Arg Gln Ile Glu Ala Lys Ala Leu Arg Lys Leu Arg His Pro Ser
                85                  90                  95

Arg Ser Glu Val Leu Arg Ser Phe Leu Asp Asp
            100                 105

<210>  27
<211>  2062
<212>  DNA
<213>  Escherichia coli

<400>  27
tgatttaacg gcttaagtgc cgaagagcga tcgggaagcc cccgacagcc gcactgagag      60

gcagcggcaa atatataagt acgccctcgt aattatcgtt ggcggtaaac aaccgttgga     120

tttcagcgtt aacggctgaa ggacatcggg tcaatcgccc aacaccaacc tcatgaaata     180

agtgtggata ccgtcttatg gagcaaaacc cgcagtcaca gctgaaactt cttgtcaccc     240

gtggtaagga gcaaggctat ctgacctatg ccgaggtcaa tgaccatctg ccggaagata     300

tcgtcgattc agatcagatc gaagacatca tccaaatgat caacgacatg ggcattcagg     360

tgatggaaga agcaccggat gccgatgatc tgatgctggc tgaaaacacc gcggacgaag     420

atgctgccga agccgccgcg caggtgcttt ccagcgtgga atctgaaatc gggcgcacga     480

ctgacccggt acgcatgtac atgcgtgaaa tgggcaccgt tgaactgttg acccgcgaag     540

gcgaaattga catcgctaag cgtattgaag acgggatcaa ccaggttcaa tgctccgttg     600

ctgaatatcc ggaagcgatc acctatctgc tggaacagta cgatcgtgtt gaagcagaag     660

aagcgcgtct gtccgatctg atcaccggct ttgttgaccc gaacgcagaa gaagatctgg     720

cacctaccgc cactcacgtc ggttctgagc tttcccagga agatctggac gatgacgaag     780

atgaagacga agaagatggc gatgacgaca gcgccgatga tgacaacagc atcgacccgg     840

aactggctcg cgaaaaattt gcggaactac gcgctcagta cgttgtaacg cgtgacacca     900

tcaaagcgaa aggtcgcagt cacgctaccg ctcaggaaga gatcctgaaa ctgtctgaag 960

tattcaaaca gttccgcctg gtgccgaagc agtttgacta cctggtcaac agcatgcgcg 1020

tcatgatgga ccgcgttcgt acgcaagaac gtctgatcat gaagctctgc gttgagcagt 1080

gcaaaatgcc gaagaaaaac ttcattaccc tgtttaccgg caacgaaacc agcgatacct 1140

ggttcaacgc ggcaattgcg atgaacaagc cgtggtcgga aaaactgcac gatgtctctg 1200

aagaagtgca tcgcgccctg caaaaactgc agcagattga agaagaaacc ggcctgacca 1260

tcgagcaggt taaagatatc aaccgtcgta tgtccatcgg tgaagcgaaa gcccgccgtg 1320

cgaagaaaga gatggttgaa gcgaacttac gtctggttat ttctatcgct aagaaataca 1380

ccaaccgtgg cttgcagttc cttgacctga ttcaggaagg caacatcggt ctgatgaaag 1440

cggttgataa attcgaatac cgccgtggtt acaagttctc cacctacgca acctggtgga 1500

tccgtcaggc gatcacccgc tctatcgcgg atcaggcgcg caccatccgt attccggtgc 1560

atatgattga gaccatcaac aagctcaacc gtatttctcg ccagatgctg caagagatgg 1620

gccgtgaacc gacgccggaa gaactggctg aacgtatgct gatgccggaa gacaagatcc 1680

gcaaagtgct gaagatcgcc aaagagccaa tctccatgga aacgccgatc ggtgatgatg 1740

aagattcgca tctgggggat ttcatcgagg ataccaccct cgagctgccg ctggattctg 1800

cgaccaccga aagcctgcgt gcggcaacgc acgacgtgct ggctggcctg accgcgcgtg 1860

aagcaaaagt tctgcgtatg cgtttcggta tcgatatgaa caccgactac acgctggaag 1920

aagtgggtaa acagttcgac gttacccgcg aacgtatccg tcagatcgaa gcgaaggcgc 1980

tgcgcaaact gcgtcacccg agccgttctg aagtgctgcg tagcttcctg gacgattaat 2040

cggtaggccg gatcaggcgt ta 2062

<210> 28
<211> 39
<212> DNA
<213> Artificial

<220>
<223> Primer TAF25_Sense

<400> 28
tcgagtgcta gcaaaatgga ttttgaggaa gattacgat 39

<210> 29
<211> 37
<212> DNA
<213> Artificial

<220>
<223> Primer TAF25_Anti

<400> 29
ctagcggtcg acctaacgat aaaagtctgg gcgacct 37

<210>  30
<211>  40
<212>  DNA
<213>  Artificial

<220>
<223>  Primer SPT15_Sense

<400>  30
tcgagtgcta gcaaaatggc cgatgaggaa cgtttaaagg                                40


<210>  31
<211>  40
<212>  DNA
<213>  Artificial

<220>
<223>  Primer SPT15_Anti

<400>  31
ctagcggtcg actcacattt ttctaaattc acttagcaca                                40


<210>  32
<211>  20
<212>  DNA
<213>  Artificial

<220>
<223>  Primer Seq_Forward

<400>  32
tcactcagta gaacgggagc                                                      20


<210>  33
<211>  20
<212>  DNA
<213>  Artificial

<220>
<223>  Primer Seq_Reverse

<400>  33
aatagggacc tagacttcag                                                      20


<210>  34
<211>  49
<212>  DNA
<213>  Artificial

<220>
<223>  primer HA350-F177S_sen

<400>  34
cgtctagaag ggttagcatc cagtcatggt actttctcct cctatgagc                      49


<210>  35
<211>  49
<212>  DNA
<213>  Artificial

<220>
<223>  primer HA351-F177S_ant

<400>  35
gctcatagga ggagaaagta ccatgactgg atgctaaccc ttctagacg                49


<210>  36
<211>  41
<212>  DNA
<213>  Artificial

<220>
<223>  primer HA352-Y195H_sen

<400>  36
ccagaattgt ttcctggttt gatccataga atggtgaagc c                        41


<210>  37
<211>  41
<212>  DNA
<213>  Artificial

<220>
<223>  primer HA353-Y195H_ant

<400>  37
ggcttcacca ttctatggat caaaccagga aacaattctg g                        41


<210>  38
<211>  46
<212>  DNA
<213>  Artificial

<220>
<223>  primer HA354-K218R_sen

<400>  38
ggaaagattg ttcttactgg tgcaaggcaa agggaagaaa tttacc                   46


<210>  39
<211>  46
<212>  DNA
<213>  Artificial

<220>
<223>  primer HA355-K218R_ant

<400>  39
ggtaaatttc ttccctttgc cttgcaccag taagaacaat ctttcc                   46


<210>  40
<211>  49
<212>  DNA
<213>  Artificial

<220>
<223>  primer HA356-F177-Revert_sen

<400>  40
cgtctagaag ggttagcatt cagtcatggt actttctcct cctatgagc                49

<210>  41
<211>  49
<212>  DNA
<213>  Artificial

<220>
<223>  primer HA357-F177-Revert_ant

<400>  41
gctcatagga ggagaaagta ccatgactga atgctaaccc ttctagacg                49


<210>  42
<211>  41
<212>  DNA
<213>  Artificial

<220>
<223>  primer HA358-Y195H-revert_sen

<400>  42
ccagaattgt ttcctggttt gatctataga atggtgaagc c                        41


<210>  43
<211>  41
<212>  DNA
<213>  Artificial

<220>
<223>  primer HA359-Y195H-revert_ant

<400>  43
ggcttcacca ttctatagat caaaccagga aacaattctg g                        41


<210>  44
<211>  46
<212>  DNA
<213>  Artificial

<220>
<223>  primer HA360-K218R-revert_sen

<400>  44
ggaaagattg ttcttactgg tgcaaagcaa agggaagaaa tttacc                   46


<210>  45
<211>  46
<212>  DNA
<213>  Artificial

<220>
<223>  primer HA361-K218R-revert_ant

<400>  45
ggtaaatttc ttccctttgc tttgcaccag taagaacaat ctttcc                   46


<210>  46
<211>  39
<212>  DNA

<213>  Artificial

<220>
<223>  primer PHO5_sen-XhoI

<400>  46
ccgctcgagc aaaactattg tctcaataga ctggcgttg                              39


<210>  47
<211>  38
<212>  DNA
<213>  Artificial

<220>
<223>  primer PHO5_anti-XbaI

<400>  47
gctctagacc aatgtttaaa tctgttgttt attcaatt                               38


<210>  48
<211>  40
<212>  DNA
<213>  Artificial

<220>
<223>  primer PHM6_sen-SalI

<400>  48
acgcgtcgac attattaaaa caaaaacttc gtcatcgtca                             40


<210>  49
<211>  38
<212>  DNA
<213>  Artificial

<220>
<223>  primer PHM6_anti-XbaI

<400>  49
gctctagacc aagatggaag atacctcgag gtgcatcg                               38


<210>  50
<211>  39
<212>  DNA
<213>  Artificial

<220>
<223>  primer FMP16_sen-XhoI

<400>  50
ccgctcgagg tgcttcttaa taaacaccgt catctggcc                              39


<210>  51
<211>  38
<212>  DNA
<213>  Artificial

<220>
<223>  primer FMP16_anti-XbaI

<400>  51
gctctagaat aatgttgaga accacttttt tgcgcact                              38


<210>  52
<211>  240
<212>  PRT
<213>  Saccharomyces cerevisiae

<400>  52

Met Ala Asp Glu Glu Arg Leu Lys Glu Phe Lys Glu Ala Asn Lys Ile
1               5                   10                  15


Val Phe Asp Pro Asn Thr Arg Gln Val Trp Glu Asn Gln Asn Arg Asp
            20                  25                  30


Gly Thr Lys Pro Ala Thr Thr Phe Gln Ser Glu Glu Asp Ile Lys Arg
        35                  40                  45


Ala Ala Pro Glu Ser Glu Lys Asp Thr Ser Ala Thr Ser Gly Ile Val
    50                  55                  60


Pro Thr Leu Gln Asn Ile Val Ala Thr Val Thr Leu Gly Cys Arg Leu
65                  70                  75                  80


Asp Leu Lys Thr Val Ala Leu His Ala Arg Asn Ala Glu Tyr Asn Pro
                85                  90                  95


Lys Arg Phe Ala Ala Val Ile Met Arg Ile Arg Glu Pro Lys Thr Thr
            100                 105                 110


Ala Leu Ile Phe Ala Ser Gly Lys Met Val Val Thr Gly Ala Lys Ser
        115                 120                 125


Glu Asp Asp Ser Lys Leu Ala Ser Arg Lys Tyr Ala Arg Ile Ile Gln
    130                 135                 140


Lys Ile Gly Phe Ala Ala Lys Phe Thr Asp Phe Lys Ile Gln Asn Ile
145                 150                 155                 160


Val Gly Ser Cys Asp Val Lys Phe Pro Ile Arg Leu Glu Gly Leu Ala
                165                 170                 175


Phe Ser His Gly Thr Phe Ser Ser Tyr Glu Pro Glu Leu Phe Pro Gly
            180                 185                 190


Leu Ile Tyr Arg Met Val Lys Pro Lys Ile Val Leu Leu Ile Phe Val
        195                 200                 205


Ser Gly Lys Ile Val Leu Thr Gly Ala Lys Gln Arg Glu Glu Ile Tyr
    210                 215                 220

Gln Ala Phe Glu Ala Ile Tyr Pro Val Leu Ser Glu Phe Arg Lys Met
225                 230                 235                 240

<210>  53
<211>  723
<212>  DNA
<213>  Saccharomyces cerevisiae

<400>  53
atggccgatg aggaacgttt aaaggagttt aaagaggcaa acaagatagt gtttgatcca      60

aataccagac aagtatggga aaaccagaat cgagatggta caaaaccagc aactactttc     120

cagagtgaag aggacataaa aagagctgcc ccagaatctg aaaaagacac ctccgccaca     180

tcaggtattg ttccaacact acaaaacatt gtggcaactg tgactttggg gtgcaggtta     240

gatctgaaaa cagttgcgct acatgcccgt aatgcagaat ataaccccaa gcgttttgct     300

gctgtcatca tgcgtattag agagccaaaa actacagctt taatttttgc ctcagggaaa     360

atggttgtta ccggtgcaaa aagtgaggat gactcaaagc tggccagtag aaaatatgca     420

agaattatcc aaaaaatcgg gtttgctgct aaattcacag acttcaaaat acaaaatatt     480

gtcggttcgt gtgacgttaa attccctata cgtctagaag ggttagcatt cagtcatggt     540

actttctcct cctatgagcc agaattgttt cctggtttga tctatagaat ggtgaagccg     600

aaaattgtgt tgttaatttt tgtttcagga aagattgttc ttactggtgc aaagcaaagg     660

gaagaaattt accaagcttt tgaagctata taccctgtgc taagtgaatt tagaaaaatg     720

tga                                                                   723

<210>  54
<211>  723
<212>  DNA
<213>  Saccharomyces cerevisiae

<400>  54
atggccgatg aggaacgttt aaaggagttt aaagaggcaa acaagatagt gtttgatcca      60

aataccagac aagtatggga aaaccagaat cgagatggta caaaaccagc aactactttc     120

cagagtgaag aggacataaa aagagctgcc ccagaatctg aaaaagacac ctccgccaca     180

tcaggtattg ttccaacact acaaaacatt gtggcaactg tgactttggg gtgcaggtta     240

gatctgaaaa cagttgcgct acatgcccgt aatgcagaat ataaccccaa gcgttttgct     300

gctgtcatca tgcgtattag agagccaaaa actacagctt taatttttgc ctcagggaaa     360

atggttgtta ccggtgcaaa aagtgaggat gactcaaagc tggccagtag aaaatatgca     420

agaattatcc aaaaaatcgg gtttgctgct aaattcacag acttcaaaat acaaaatatt     480

gtcggttcgt gtgacgttaa attccctata cgtctagaag ggttagcatc cagtcatggt     540

actttctcct cctatgagcc agaattgttt cctggtttga tccatagaat ggtgaagccg     600

aaaattgtgt tgttaatttt tgtttcagga aagattgttc ttactggtgc aaggcaaagg 660

gaagaaattt accaagcttt tgaagctata taccctgtgc taagtgaatt tagaaaaatg 720

tga 723

<210> 55
<211> 621
<212> DNA
<213> Saccharomyces cerevisiae

<400> 55
atggattttg aggaagatta cgatgcggag tttgatgata atcaagaagg acaattagaa 60

acaccttttc catcggttgc gggagccgat gatggggaca atgataatga tgactctgtc 120

gcagaaaaca tgaagaagaa gcaaaagaga gaggctgtag tggatgatgg gagtgaaaat 180

gcatttggta tacccgaatt tacaagaaaa gataagactc tggaggagat tctagagatg 240

atggacagta ctcctcctat cattcccgat gcagtaatag actactattt aaccaaaaac 300

gggtttaacg tagcagatgt acgagtgaaa cgacttttag cacttgctac tcagaaattt 360

gttagtgata tagctaagga tgcctacgaa tattccagga tcaggtcttc cgtagcggta 420

tctaatgcta acaacagtca ggcgagagct aggcagctat tgcaaggaca gcaacagcct 480

ggcgtgcagc agatttcaca acaacaacat caacagaatg agaagactac agcaagcaaa 540

gttgttctga cggtgaacga tctcagtagc gctgttgctg aatacgggct caatataggt 600

cgcccagact tttatcgtta g 621

<210> 56
<211> 621
<212> DNA
<213> Saccharomyces cerevisiae

<400> 56
atggattttg aggaagatta cgatgcggag tttgatgata atcaagaagg acaattagaa 60

acaccttttc catcggttgc gggagccgat ggtggggaca atgataatga tgactctgtc 120

gcagaaaaca tgaagaagaa gcaaaagaga gaggctgtag aggatgatgg gagtgaaaat 180

gcatttggta tacccgaatt tacaagaaaa gataagactc tggaggagat tctagagatg 240

atggacagta ctcctcctat cattcccgat gcagtaatag actactattt aaccaaaaac 300

gggtttaacg tagcagatgt acgagtgaaa cgacttttag cacttgctac tcagaaattt 360

gttagtgata tagctaagga tgcctacgaa tattccagga tcaggtcttc cgtagcggta 420

tctaatgcta acaacagtca ggcgagagct aggcagctat tgcaaggaca gcaacagcct 480

ggcgtgcagc agatttcaca acaacaacat caacagaatg agaagactac agcaagcaga 540

gttgttctga cggtgaacga tctcagtagc gctgttgctg aatacgggct caatataggt 600

cgcccagact tttatcgtta g 621

## Description

### Government Interest

This work was funded in part by the Department of Energy under grant number DE-FG02-94ER14487. The government has certain rights in this invention.

### Related Application

This application claims the benefit under 35 U.S.C. § 119(e) of U.S. provisional application 60/873,419, filed December 7, 2006, the entire disclosure of which is incorporated herein by reference.

### Field of the Invention

The invention relates to global transcription machinery engineering to produce altered cells having improved phenotypes, and the use of such cells in production processes.

### Background of the Invention

It is now generally accepted that many important cellular phenotypes, from disease states to metabolite overproduction, are affected by many genes. Yet, most cell and metabolic engineering approaches rely almost exclusively on the deletion or over-expression of single genes due to experimental limitations in vector construction and transformation efficiencies. These limitations preclude the simultaneous exploration of multiple gene modifications and confine gene modification searches to restricted sequential approaches where a single gene is modified at a time.

U.S. patent 5,686,283 described the use of a sigma factor encoded by *rpoS* to activate the expression of other bacterial genes that are latent or expressed at low levels in bacterial cells. This patent did not, however, describe mutating the sigma factor in order to change globally the transcription of genes.

U.S. patent 5,200,341 provides a mutated *rpoH* gene identified as a suppressor of a temperature sensitive *rpoD* gene by selection of temperature-resistant mutants of a bacterial strain having the temperature sensitive *rpoD* gene. No mutagenesis of the bacteria was undertaken, nor was the suppressor strain selected for a phenotype other than temperature resistance. When the mutant *rpoH* gene is added to other bacteria that are modified to express heterologous proteins, the heterologous proteins are accumulated at increased levels in the bacteria.

U.S. patent 6,156,532 describes microorganisms that are modified by introduction of a gene coding for a heat shock protein and a gene coding for a sigma factor (*rpoH*) that specifically functions for the heat shock protein gene to enhance expression amount of the heat shock protein in cells. The modified microorganisms are useful for producing fermentative products such as amino acids. The sigma factor used in the microorganisms was not mutated.

Directed evolution has been applied to microorganisms by shuffling of bacterial genomes for antibiotic (tylosin) production by *Streptomyces* (Zhang et al., Nature, 415, 644-646 (2002)) and acid tolerance of *Lactobacillus* (Patnaik et al., Nature Biotech. 20, 707-712 (2002)). These methods did not target mutations in any specific gene or genes, but instead non-recombinantly shuffled the genomes of strains having a desired phenotype using protoplast fusion, followed by selection of strains having improvements in the desired phenotype.

### Summary of the Invention

The invention utilizes global transcription machinery engineering to produce altered cells having improved phenotypes. In particular, the invention is demonstrated through the generation of mutated yeast RNA polymerase II factors, such as the TATA binding protein (SPT15), with varying preferences for promoters on a genome-wide level. The cells resulting from introduction of the mutated RNA polymerase II factors have rapid and marked improvements in phenotypes, such as tolerance of deleterious culture conditions or improved production of metabolites.

The introduction of mutant transcription machinery into a cell, combined with methods and concepts of directed evolution, allows one to explore a vastly expanded search space in a high throughput manner by evaluating multiple, simultaneous gene alterations in order to improve complex cellular phenotypes.

Directed evolution through iterative rounds of mutagenesis and selection has been successful in broadening properties of antibodies and enzymes (W. P. Stemmer, *Nature* **370**, 389-91 (1994)). These concepts have been recently extended and applied to non-coding, functional regions of DNA in the search for libraries of promoter activity spanning a broad dynamic range of strength as measured by different metrics (H. Alper, C. Fischer, E. Nevoigt, G. Stephanopoulos, Proc Natl Acad Sci U S A 102, 12678-12683 (2005)). However, no evolution-inspired approaches have been directed towards the systematic modification of the global transcription machinery as a means of improving cellular phenotype. Yet, detailed biochemical studies suggest that both the transcription rate and *in vitro* preference for a given promoter sequence can be altered by modifying key residues on bacterial sigma factors (D. A. Siegele, J. C. Hu, W. A. Walter, C. A. Gross, J Mol Biol 206, 591-603 (1989); T. Gardella, H. Moyle, M. M. Susskind, J Mol Biol 206, 579-590 (1989)). Such modified transcription machinery units offer the unique opportunity to introduce simultaneous global transcription-level alterations that have the potential to impact cellular properties in a very profound way.

The invention is described herein in relation to yeast and SPT15, but the invention is broadly applicable to other eukaryotic cells, particularly fungi as it pertains to ethanol production, and related RNA polymerase II factors in such eukaryotic cells. The specific mutations described herein can be replicated in the corresponding amino acid positions of orthologs of SPT15 in other cells with substantially similar results. Likewise, other amino acids (preferably amino acids that are conservative substitutions of the mutant amino acids) can be used in place of the specific amino acid substitutions used in the mutant SPT15 gene herein, with substantially similar results. Therefore, the invention embraces the use of other eukaryotic cells and the corresponding global transcription machinery of such cells for the improvement of phenotypic characteristics, particularly tolerance of glucose (and other sugars) and/or ethanol in culture media, and/or ethanol production by the cells from a variety of feedstocks known in the art.

According to one aspect of the invention, genetically modified yeast strains are provided. The strains include a mutated *SPT15* gene. Optionally, prior to introduction of the mutated *SPT15* gene or mutation of an endogenous *SPT15* gene, the yeast strain without the mutated *SPT15* gene had improved ethanol and/or glucose tolerance and/or ethanol production relative to a wild type yeast strain. The mutated *SPT15* gene further improves ethanol and/or glucose tolerance and/or ethanol production relative to the wild type yeast and the yeast strain without the mutated *SPT15* gene.

In some embodiments, the mutated *SPT15* gene includes mutations at two or more of positions F177, Y195 and K218, preferably at all three positions (F177, Y195 and K218). In some preferred embodiments, the mutated *SPT15* gene includes two or more of the mutations F177S, Y195H and K218R, or conservative substitutions of the mutant amino acids, preferably all of F177S, Y195H and K218R, or conservative substitutions of the mutant amino acids.

In other embodiments, the mutated *SPT15* gene is recombinantly expressed in the genetically modified yeast strains. In some embodiments, the mutated *SPT15* gene is introduced into the yeast cell on a plasmid, or is introduced into the genomic DNA of the yeast cell. In other embodiments, the mutated *SPT15* gene is an endogenous gene in the genomic DNA of the yeast cell that is mutated *in situ.*

In further embodiments, the yeast strain is selected from *Saccharomyces* spp., *Schizosaccharomyces* spp., *Pichia* spp., *Paffia* spp., *Kluyveromyces* spp., *Candida* spp., *Talaromyces* spp., *Brettanomyces* spp., *Pachysolen* spp., *Debaryomyces* spp., and industrial polyploid yeast strains. Preferably the yeast strain is a *S*. *cerevisiae* strain.

In some embodiments, the yeast strain without the mutated *SPT15* gene is a yeast strain that is genetically engineered, selected, or known to have one or more desirable phenotypes for enhanced ethanol production. Preferably the one or more desirable phenotypes are ethanol tolerance and/or increased fermentation of C5 and C6 sugars. The phenotype of increased fermentation of C5 and C6 sugars preferably is increased fermentation of xylose. In certain of these latter embodiments, the genetically modified yeast strain is transformed with an exogenous xylose isomerase gene, an exogenous xylose reductase gene, and exogenous xylitol dehydrogenase gene and/or an exogenous xylulose kinase gene. In further embodiments, the genetically modified yeast strain comprises a further genetic modification that is deletion of non-specific or specific aldose reductase gene(s), deletion of xylitol dehydrogenase gene(s) and/or overexpression of xylulokinase.

In still other embodiments, the yeast strain without the mutated *SPT15* gene is a yeast strain that is respiration-deficient. In some embodiments, the yeast strain displays normal expression or increased expression of Spt3 and/or is not an Spt3 knockout or null mutant.

According to another aspect of the invention, methods for making the foregoing genetically modified yeast strains are provided. The methods include introducing into a yeast strain one or more copies of the mutated *SPT15* gene and/or mutating *in situ* an endogenous gene in the genomic DNA of the yeast cell.

In a further aspect of the invention, methods for producing ethanol are provided. The methods include culturing the foregoing genetically modified yeast strains in a culture medium that has one or more substrates that are metabolizable into ethanol, for a time sufficient to produce a fermentation product that contains ethanol. In some embodiments, the one or more substrates that are metabolizable into ethanol comprise C5 and/or C6 sugars. Preferably the one or more C5 and/or C6 sugars comprise glucose and/or xylose.

According to another aspect of the invention, methods for producing ethanol are provided. The methods include culturing the genetically modified yeast strain comprising a mutated *SPT15* gene having mutations at F177S, Y195H and K218R, in a culture medium that has one or more substrates that are metabolizable into ethanol, for a time sufficient to produce a fermentation product that contains ethanol. In some embodiments, the one or more substrates that are metabolizable into ethanol comprise C5 and/or C6 sugars. Preferably the one or more C5 and/or C6 sugars comprise glucose and/or xylose.

In another aspect of the invention, fermentation products of the foregoing methods are provided, as is ethanol isolated from the fermentation products. Preferably the ethanol is isolated by distillation of the fermentation products.

According to another aspect of the invention, methods for producing a yeast strain having improved ethanol and/or glucose tolerance and/or ethanol production are provided. The methods include providing a yeast strain comprising a mutated *SPT15* gene, and performing genetic engineering and/or selection for improved ethanol and/or glucose tolerance and/or improved ethanol production.

In some embodiments, the mutated *SPT15* gene includes mutations at two or more of positions F177, Y195 and K218, preferably at all three positions (F177, Y195 and K218). In some preferred embodiments, the mutated *SPT15* gene includes two or more of the mutations F177S, Y195H and K218R, or conservative substitutions of the mutant amino acids, preferably all of F177S, Y195H and K218R, or conservative substitutions of the mutant amino acids.

In other embodiments, the mutated *SPT15* gene is recombinantly expressed in the genetically modified yeast strains. In some embodiments, the mutated *SPT15* gene is introduced into the yeast cell on a plasmid, or is introduced into the genomic DNA of the yeast cell. In other embodiments, the mutated *SPT15* gene is an endogenous gene in the genomic DNA of the yeast cell that is mutated *in situ.*

In further embodiments, the yeast strain is selected from *Saccharomyces* spp., *Schizosaccharomyces* spp., *Pichia* spp., *Paffia* spp., *Kluyveromyces* spp., *Candida* spp., *Talaromyces* spp., *Brettanomyces* spp., *Pachysolen* spp., *Debaryomyces* spp., and industrial polyploid yeast strains. Preferably the yeast strain is a *S*. *cerevisiae* strain. More preferably the yeast strain is Spt15-300

According to another aspect of the invention, yeast strains produced by the foregoing methods are provided.

Still another aspect of the invention provides methods for producing ethanol. The methods include culturing the foregoing yeast strains in a culture medium that has one or more substrates that are metabolizable into ethanol, for a time sufficient to produce a fermentation product that contains ethanol. In some embodiments, the one or more substrates that are metabolizable into ethanol comprise C5 and/or C6 sugars; preferably the one or more C5 and/or C6 sugars comprise glucose and/or xylose.

In another aspect of the invention, fermentation products of the foregoing methods are provided, as is ethanol isolated from the fermentation products. Preferably the ethanol is isolated by distillation of the fermentation products.

According to another aspect of the invention, yeast strains are provided that overexpress any combination of at least 2, 3, 4, 5, 6, 7, 8, 9, 10, 11, 12, 13, or all 14 genes listed in Table 5, or genes with one or more substantially similar or redundant biological/biochemical activities or functions.

According to a further aspect of the invention, genetically modified yeast strains are provided. The strains, when cultured in a culture medium containing an elevated level of ethanol, achieve a cell density at least 4 times as great as a wild type strain cultured in the culture medium containing an elevated level of ethanol. In some embodiments, the strain achieves a cell density between 4-5 times as great as a wild type strain. In further embodiments, the elevated level of ethanol is at least about 5% or at least about 6%.

In some embodiments of the foregoing genetically modified yeast strains, the culture medium comprises one or more sugars at a concentration of at least about 20 g/L, preferably at least about 60 g/L, more preferably at least about 100 g/L, and still more preferably at least about 120 g/L.

These and other aspects of the invention, as well as various embodiments thereof, will become more apparent in reference to the drawings and detailed description of the invention.

### Brief Description of the Drawings

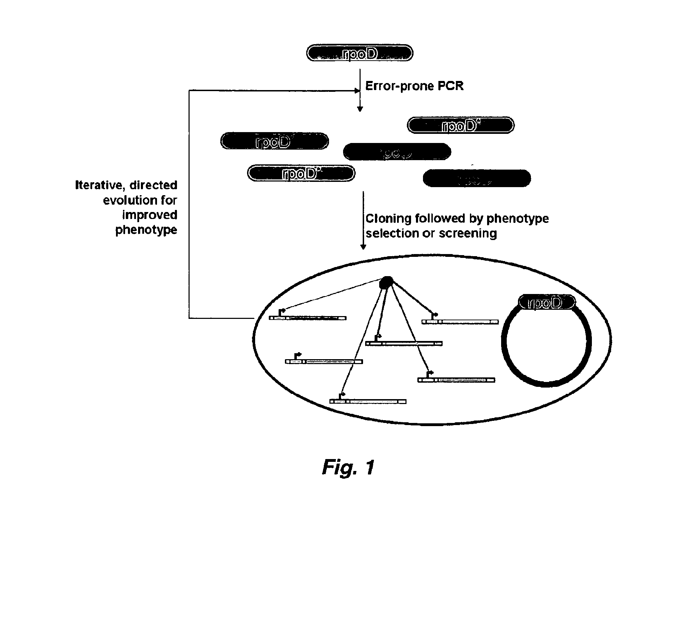

Figure 1 depicts the basic methodology of global transcription machinery engineering. By introducing altered global transcription machinery into a cell, the transcriptome is altered and the expression level of genes changes in a global manner. In this study, the bacterial sigma factor 70 (encoded by *rpoD*) was subjected to error-prone PCR to generate various mutants. The mutants were then cloned into a low-copy expression vector, during which the possibility arose for a truncated form of the sigma factor due to the presence of a nearly complete internal restriction enzyme site. The vectors were then transformed into *E. coli* and screened based on the desired phenotype. Isolated mutants can then be subjected to subsequent rounds of mutagenesis and selection to further improve phenotypes.

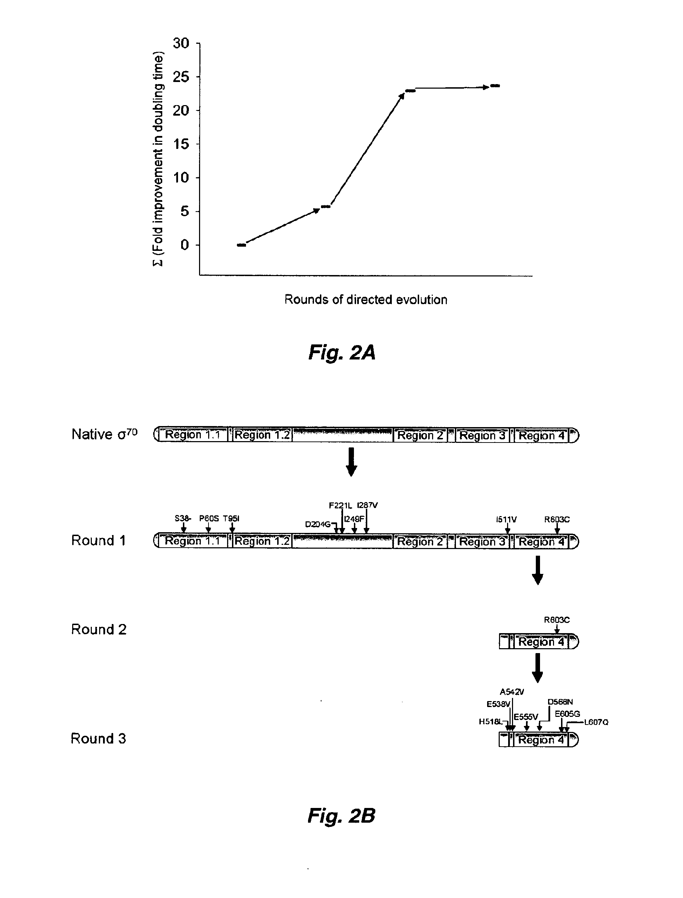

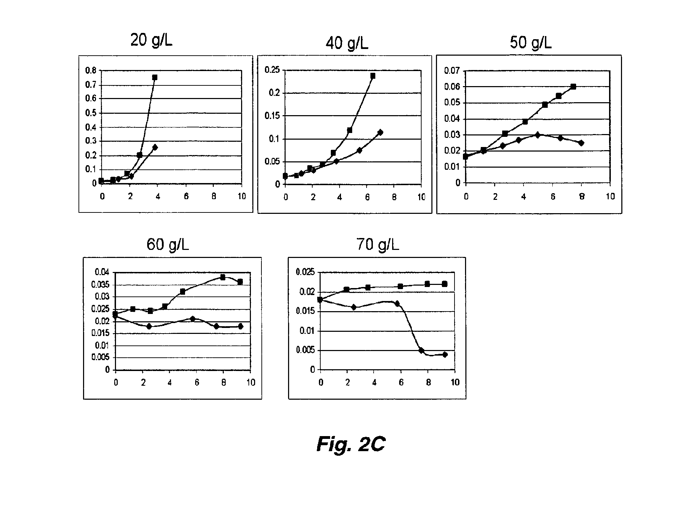

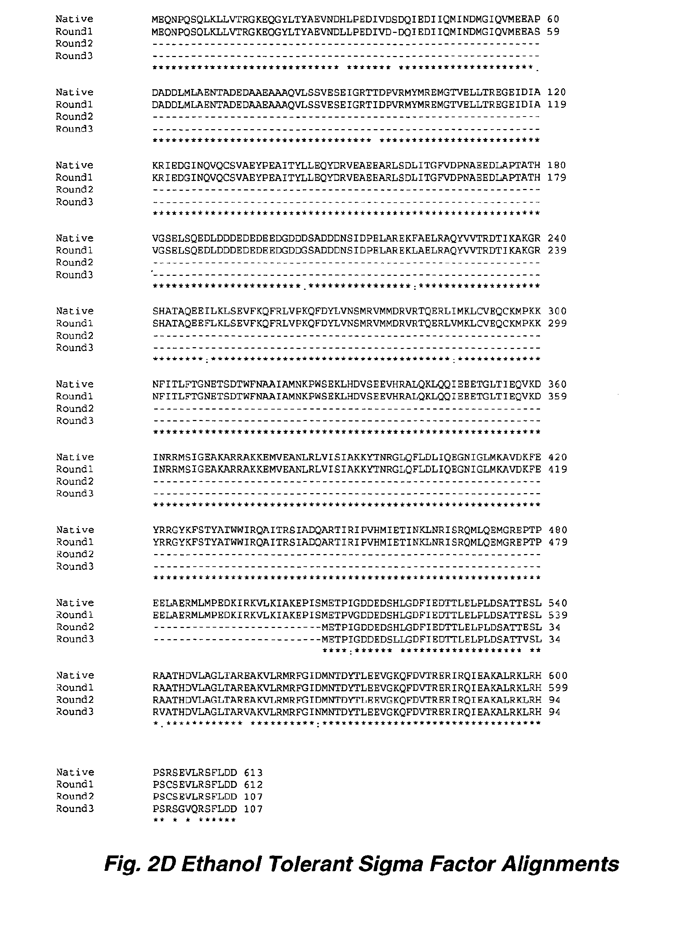

Figure 2 shows the isolation of ethanol tolerant sigma factor mutants. Strains were isolated containing mutant sigma factors which increased the tolerance to ethanol. Fig. 2A: The overall enhancement of phenotype through the various round of directed evolution of the mutant factor. Overall enhancement (y-axis) is assessed by taking the summation of the fold reduction of doubling time for the mutant over the control at 0, 20, 40, 50, 60, 70 and 80 g/L of ethanol. By the third round, the improvement in growth rate seems to be small and incremental. Fig. 2B: The location of mutations on the σ⁷⁰ protein are indicated in relation to previously identified critical functional regions. The second round mutagenesis resulted in the identification of a truncated factor containing only one of the two prior mutations in that region. Fig. 2C: Growth curves are presented for the Round 3 mutant (Red) and control (Blue) strains. The round 3 mutant has significantly improved growth rates at all tested ethanol concentrations. Fig. 2D: Amino acid sequence alignments of the ethanol tolerant mutant sigma factors (Native, SEQ ID NO: 17; Round 1, SEQ ID NO: 18; Round 2, SEQ ID NO: 19; Round 3, SEQ ID NO:20).

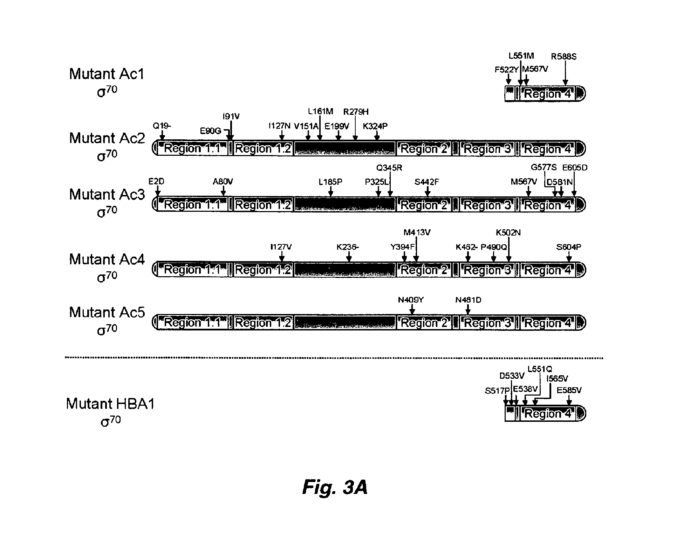

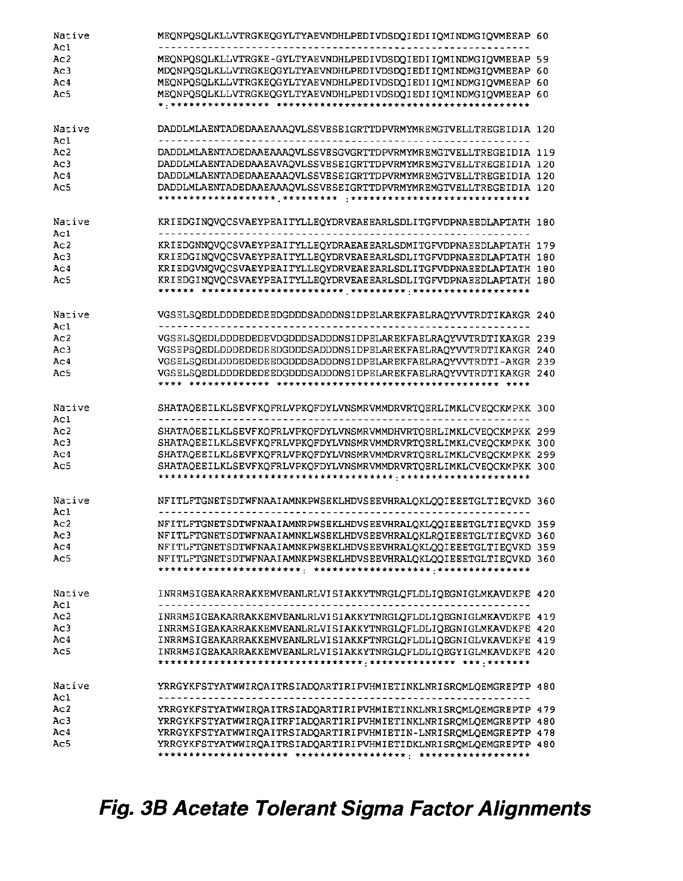

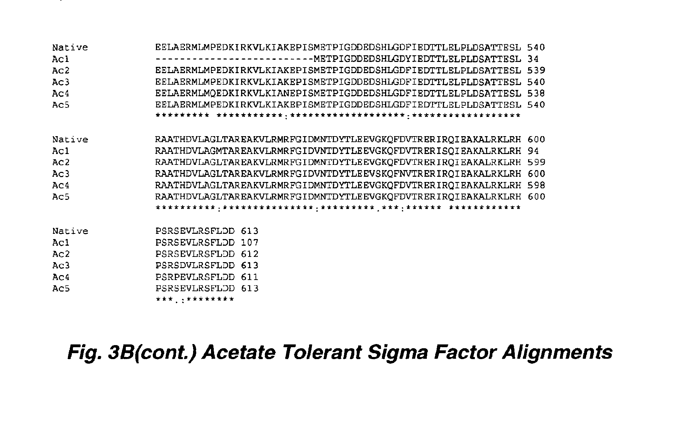

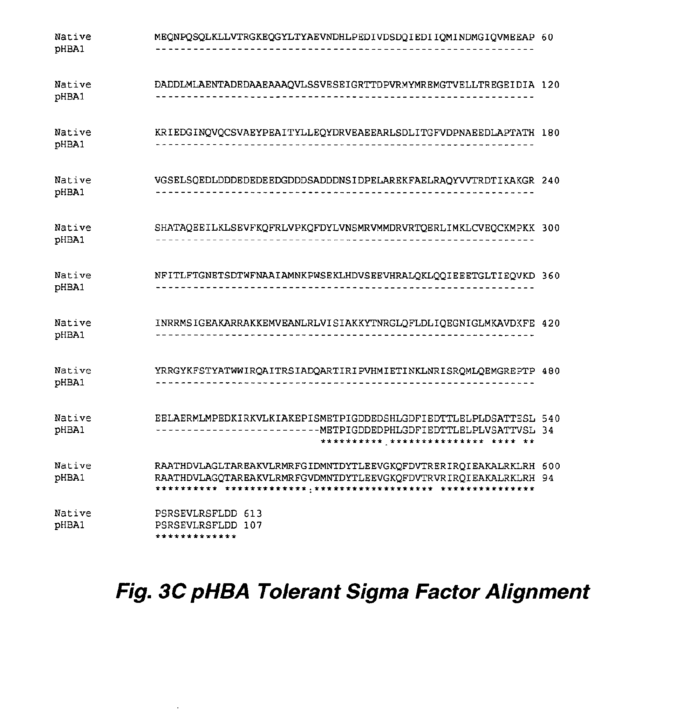

Figure 3 shows sequence analysis of sigma factors for additional phenotypes. Fig. 3A: The location of the mutations in the acetate and pHBA mutants of the σ⁷⁰ protein area indicated in relation to previously identified critical functional regions. The vast majority of the acetate mutants were full-length sigma factors. The identified mutant for pHBA was a truncated factor which is expected to act as an inhibitor to specific gene transcription. Fig. 3B: Amino acid sequence alignments of the acetate tolerant mutant sigma factors (Native, SEQ ID NO:17; Ac1, SEQ ID NO:21; Ac2, SEQ ID NO:22; Ac3, SEQ ID NO:23; Ac4, SEQ ID NO:24; Ac5, SEQ ID NO:25). Fig. 3C: Amino acid sequence alignments of the pHBA tolerant mutant sigma factors (Native, SEQ ID NO:17; pHBA1, SEQ ID NO:26).

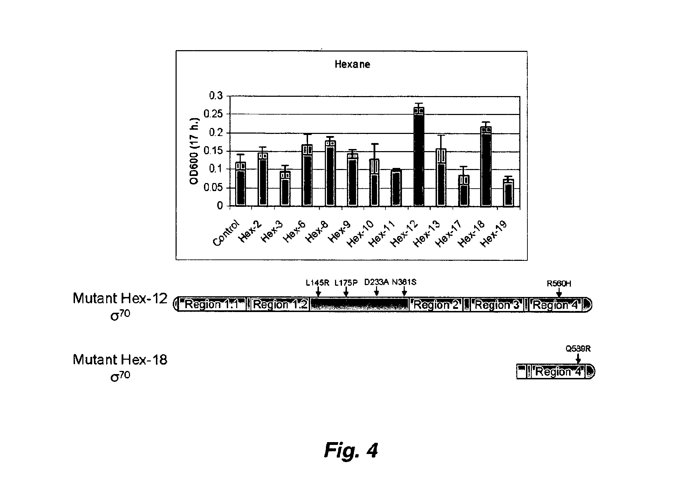

Figure 4 depicts cell densities of cultures of isolated strains with hexane tolerant sigma factor mutants. Fig. 4 also shows the sequences of the best hexane-tolerant mutants, Hex-12 and Hex-18.

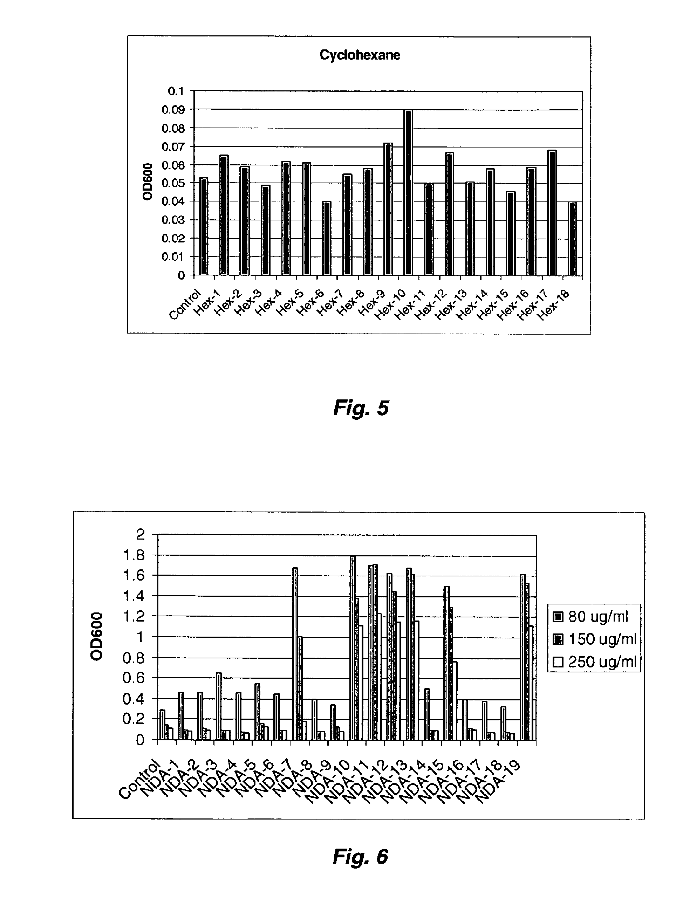

Figure 5 shows cell densities of cultures of isolated strains with cyclohexane tolerant sigma factor mutants.
Figure 6 depicts cell densities of cultures of isolated strains of antibiotic resistant sigma factor mutants at increasing concentrations of nalidixic acid.

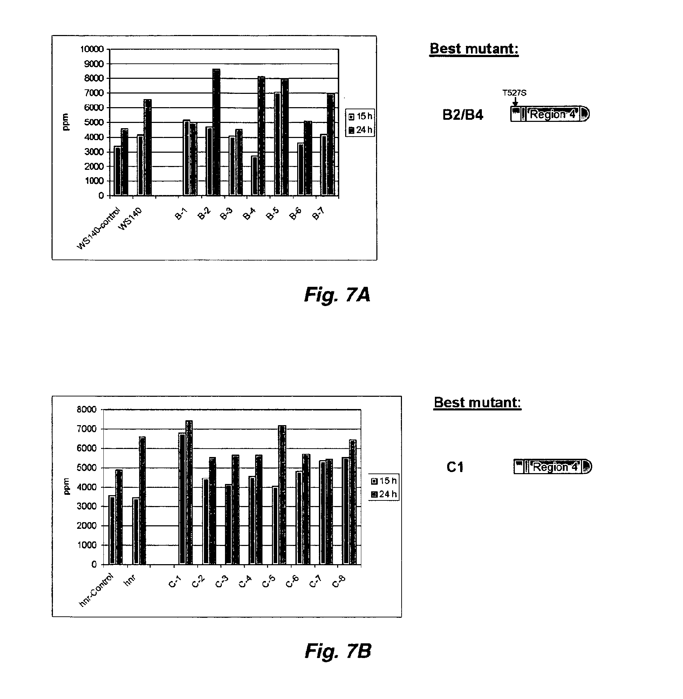

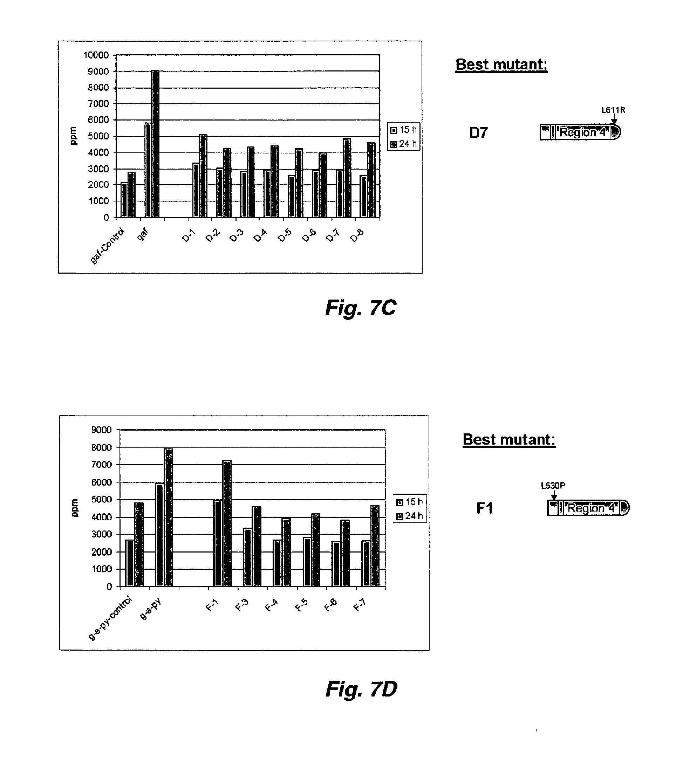

Figures 7A - 7D show the results of culturing and assaying selected strains for lycopene production at 15 and 24 hours, along with the sequence of the sigma factor mutant from the best strain.

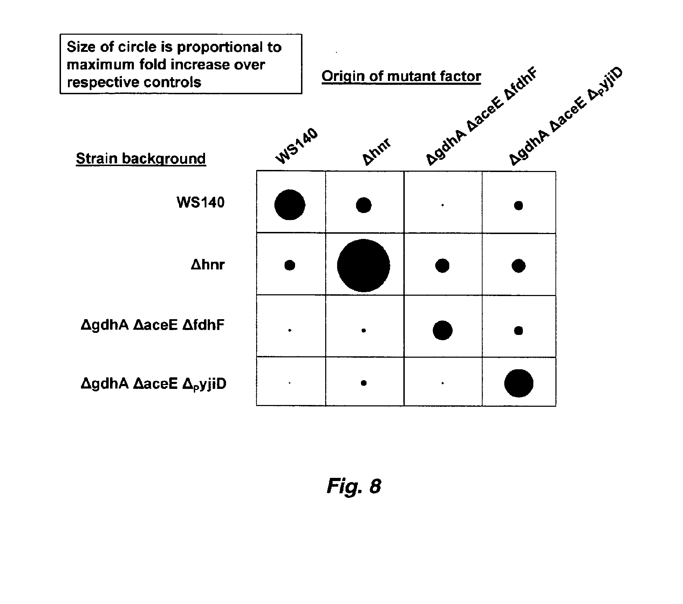

Figure 8 is a dot plot that depicts the maximum fold increase in lycopene production achieved over the control during the fermentation. The size of the circle is proportional to the fold increase.

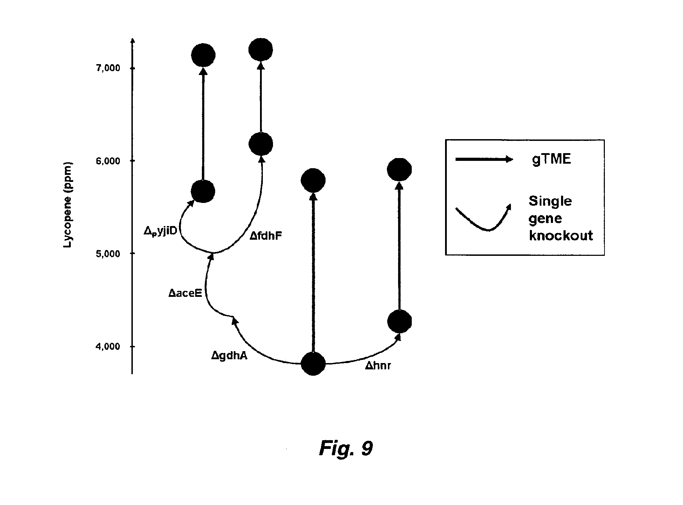

Figure 9 illustrates the lycopene content after 15 hours for several strains of interest. This figure compares the improvement provided by global transcription machinery engineering to traditional methods of strain improvement by sequential gene knockouts. In this example, the method of global transcription machinery engineering was more potent in increasing the phenotype than a series of multiple gene knockouts. Furthermore, improvements were achieved in pre-engineered strains.

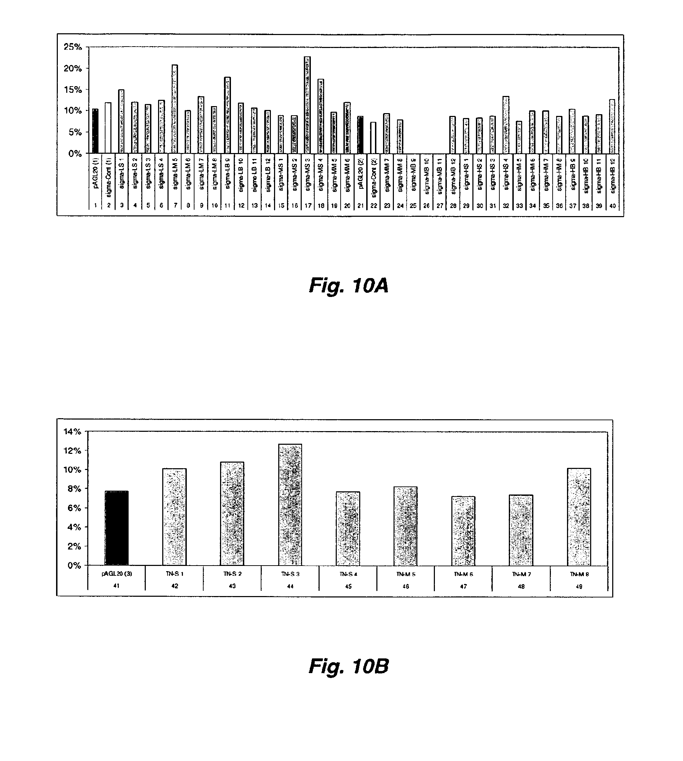

Figure 10 shows strains selected for increased exponential phase PHB in a glucose-minimal media. Fig. 7A presents the results for various strains (bars in red and yellow represent controls) obtained using sigma factor engineering. Fig. 7B presents the results of selected strains from a random knockout library created using transposon mutagenesis.

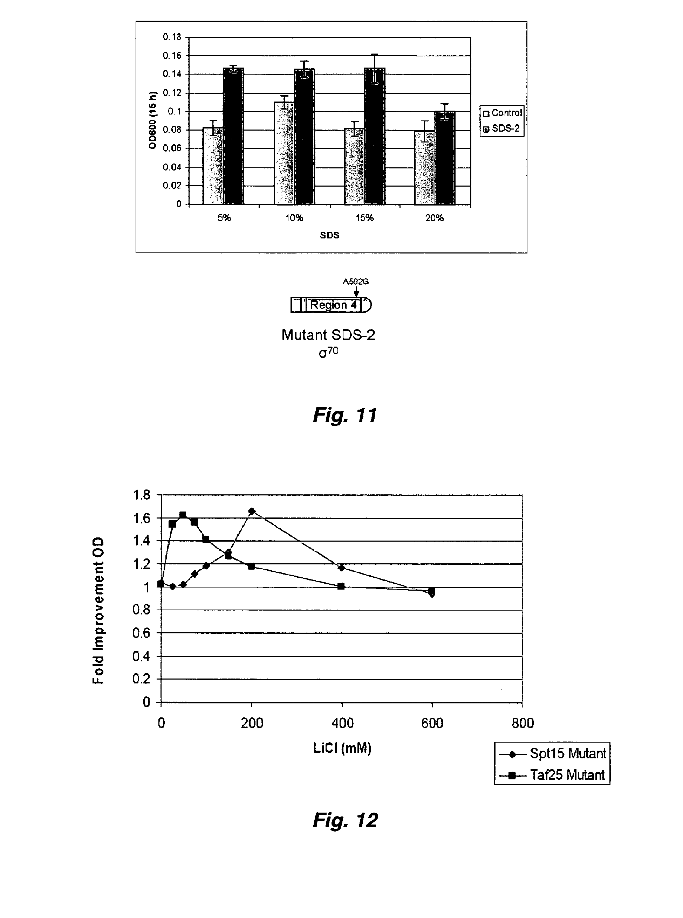

Figure 11 depicts cell densities of cultures of isolated strains of SDS-tolerant sigma factor mutants at increasing concentrations of SDS, along with the sequence of the sigma factor mutant from the best strain.
Figure 12 shows a growth analysis of LiCl gTME mutants in yeast. Strains harboring mutant *Taf25* or *Spt15* were isolated with through serial subculturing in elevated levels of LiCl in a synthetic minimal medium. The growth yield (as measured by OD600) is shown for mutant and control strains after 16 hours. The *Taf25* outperformed the control at lower concentrations of LiCl, while the *Spt15* mutant was more effective at higher concentrations.

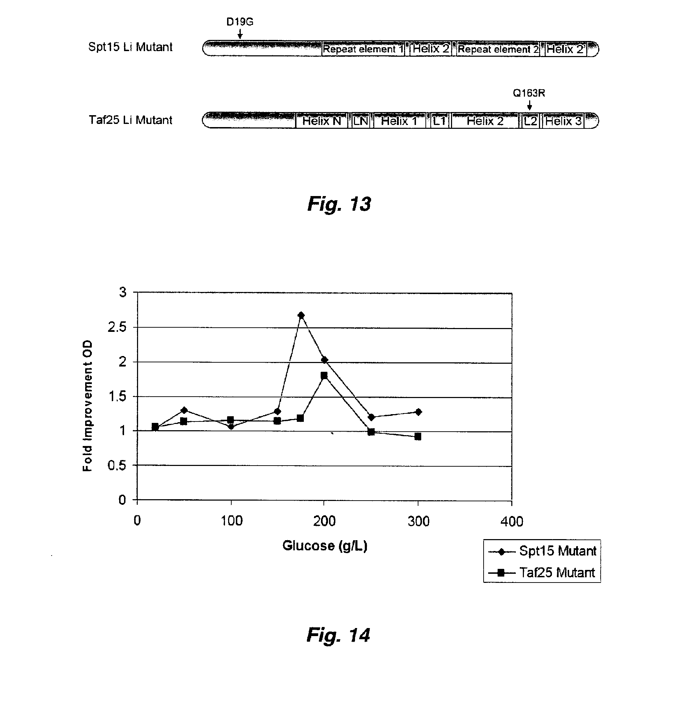

Figure 13 depicts sequence analysis of LiCl gTME mutants in yeast. Mutations are shown mapped onto a schematic showing critical functional components of the respective factor. Each mutant was seen to possess only a single amino acid substitution.
Figure 14 shows a growth analysis of glucose gTME mutants in yeast. Strains harboring mutant *Taf25* or *Spt15* were isolated with through serial subculturing in elevated levels of glucose in a synthetic minimal medium. Here, both proteins show an improvement across a similar range of concentrations, with the SPT15 protein giving the largest improvement.

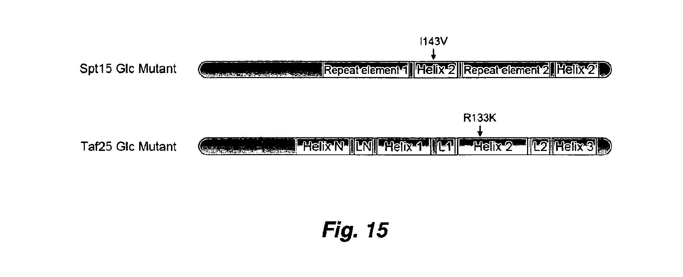

Figure 15 depicts sequence analysis of glucose gTME mutants in yeast. Mutations are shown mapped onto a schematic showing critical functional components of the respective factor. Each mutant was seen to possess only a single amino acid substitution, however several other SPT15 proteins were isolated, some possessing many mutations.

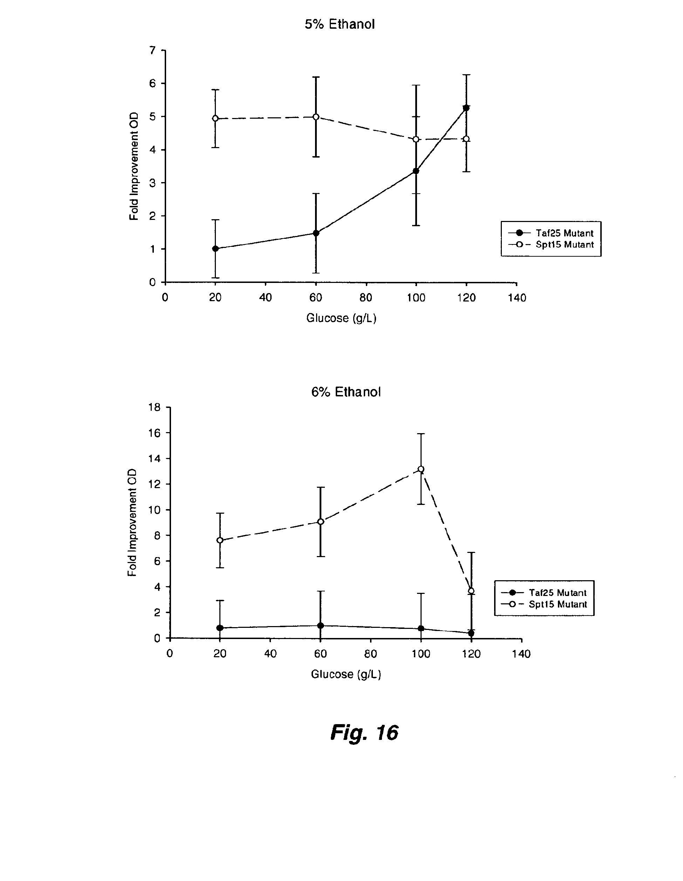

Figure 16 shows a growth analysis of ethanol-glucose gTME mutants in yeast. Strains harboring mutant *Taf25* or *Spt15* were isolated with through serial subculturing in elevated levels of ethanol and glucose in a synthetic minimal medium and assayed for growth at 20 hours. Here, the SPT15 protein far exceeded the impact of the TAF25 mutant.

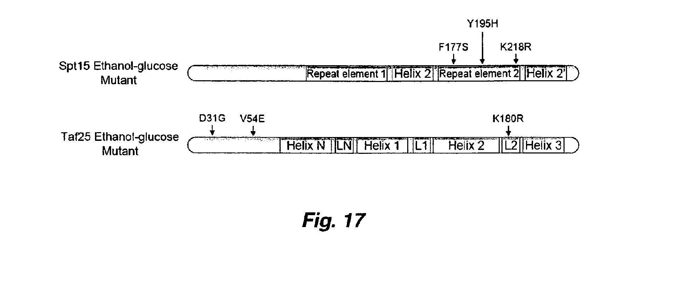

Figure 17 depicts sequence analysis of ethanol-glucose gTME mutants in yeast. Mutations are shown mapped onto a schematic showing critical functional components of the respective factor. Each mutant was seen to possess several single amino acid substitutions in critical regions for DNA or protein contacts.

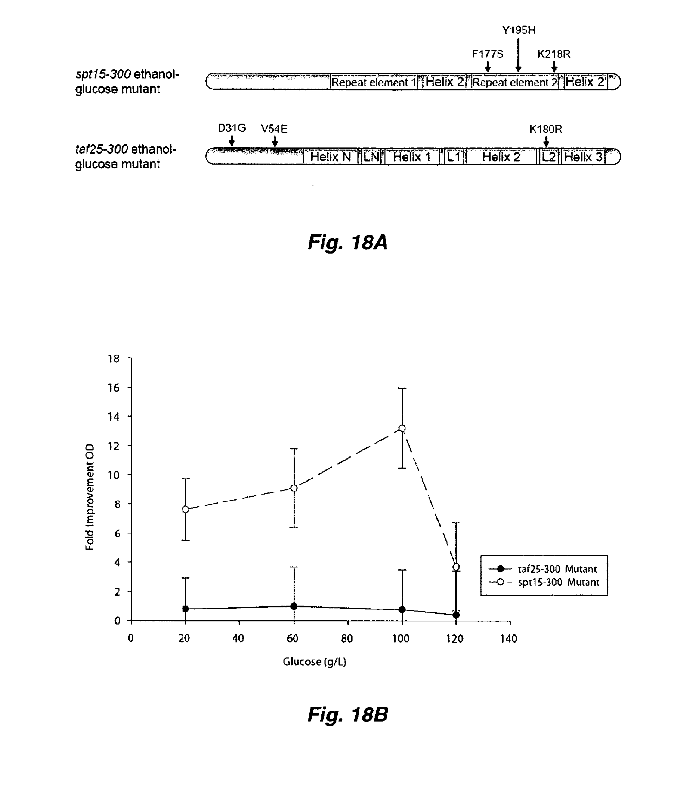

Figure 18 shows yeast gTME mutants with increased tolerance to elevated ethanol and glucose concentrations. (**A**) Mutations for the best clone isolated from either the *spt15-300* or *taf25-300* mutant library are shown mapped onto a schematic of critical functional components of the respective factor (Supplemental text, part a). (**B**) Growth yields of the clones from (**A**), were assayed in synthetic minimal medium containing elevated levels (6% by volume) of ethanol and glucose after 20 hours. Under these conditions, the *spt15-300* mutant far exceeded the performance of the *taf25-300* mutant. Fold improvements of growth yields are compared to an isogenic strain that harbors a plasmid-borne, wild-type version of either *SPT15* or *TAF25.*

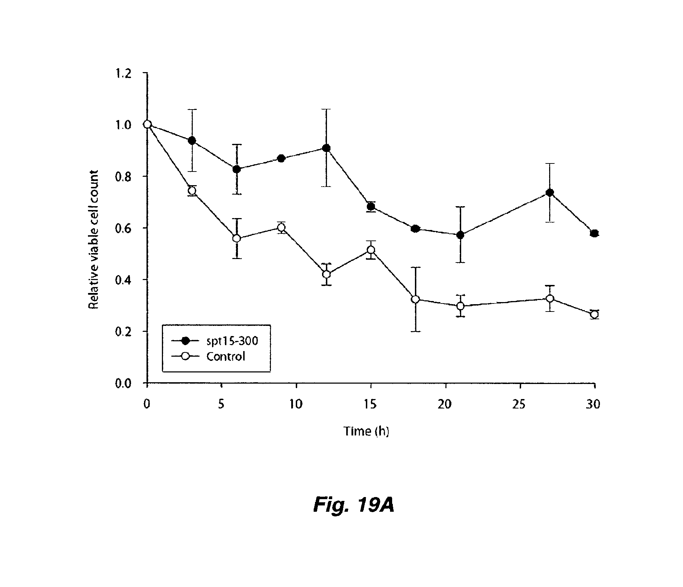

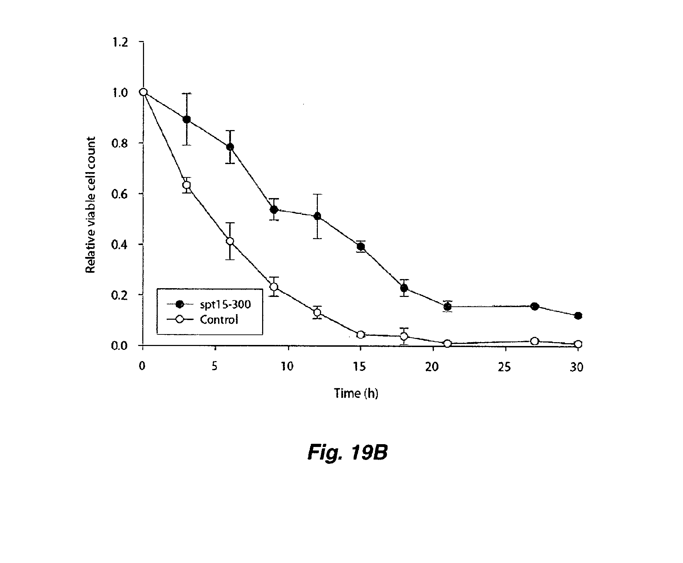

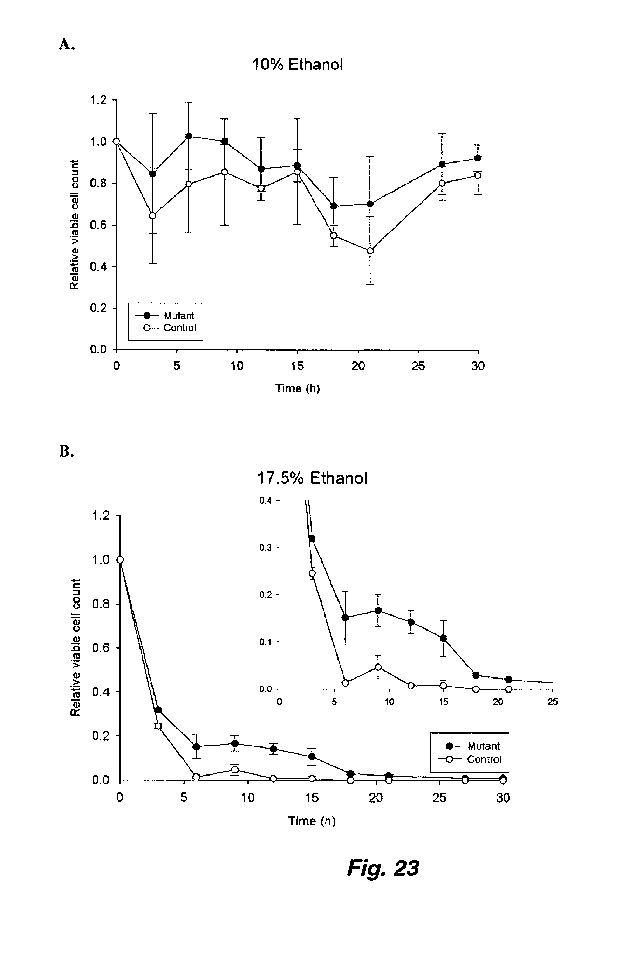

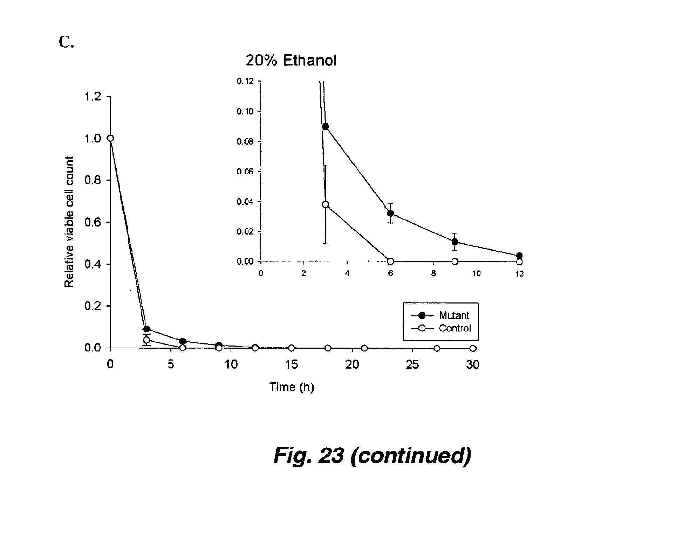

Figure 19 depicts cellular viability curves evaluating the tolerance of the mutant under ethanol stress. Viability of the *spt15-300* mutant strain compared with the control is measured as a function of time (hours) and expressed as the relative number of colony forming units compared with colony count at 0 hours for stationary phase cells treated and incubated in standard medium in the presence of (**A**) 12.5% and (**B**) 15% ethanol by volume. The *spt15-300* mutation confers a significantly enhanced viability at all concentrations tested above 10% ethanol by volume (Fig. 23). Error bars represent the standard deviation between biological replicate experiments. Initial cell counts were approximately 3.5x10⁶ cells/ml.

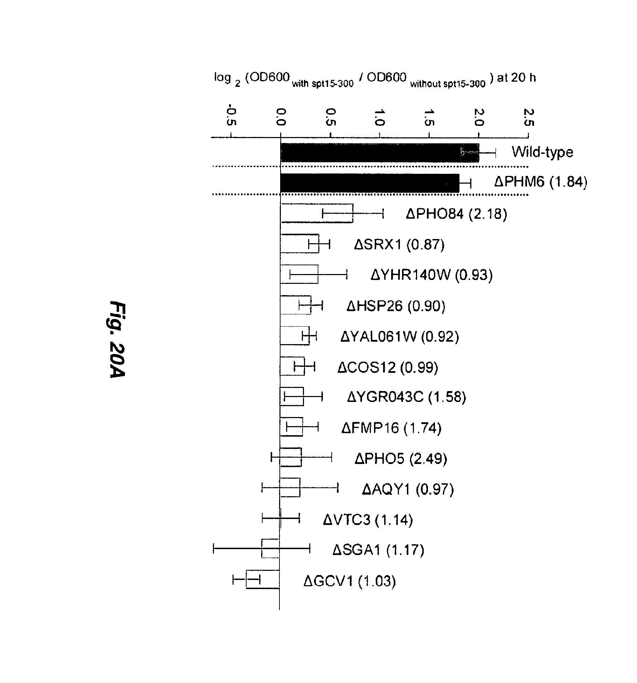

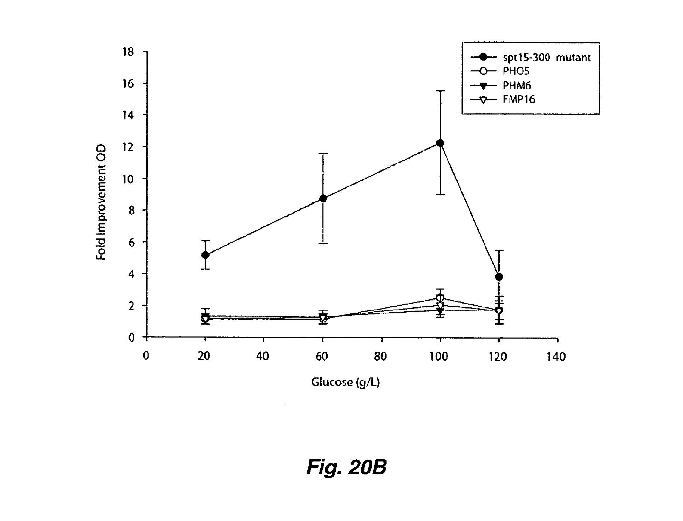

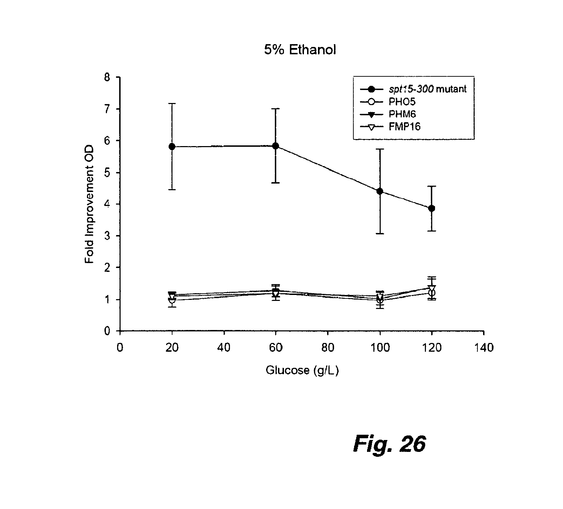

Figure 20 shows gene knockout and overexpression analysis to probe the transcriptome-level response elicited by the mutant *spt15.* (**A**) Loss-of-phenotype analysis was performed using twelve of the most highly expressed genes in this mutant (log2 differential gene expression given in parenthesis), as well as 2 additional genes were chosen for further study (Supplemental text, part c). The tolerance (to 5% ethanol, 60 g/L glucose) of 14 strains deleted in one of the 14 genes, respectively, was tested by comparing the knockout strain containing the *spt15-300* mutation on a plasmid to a strain containing the wild-type *SPT15.* All gene knockouts, except *PHM6,* resulted in slight to full loss of phenotype. Control mutants for all of the gene knockout targets exhibited similar growth yields. (**B**) Gene overexpression studies are provided for the top 3 candidate genes from the microarray (*PHO5, PHM6,* and *FMP16*) and assayed under 6% ethanol by volume as previously assayed (see also Fig. 26). The overexpression of these genes failed to impart a tolerance phenotype.

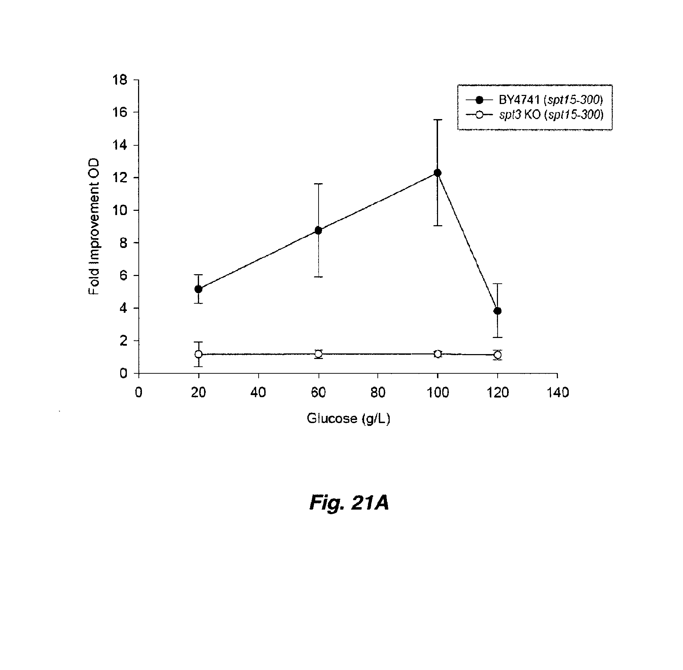

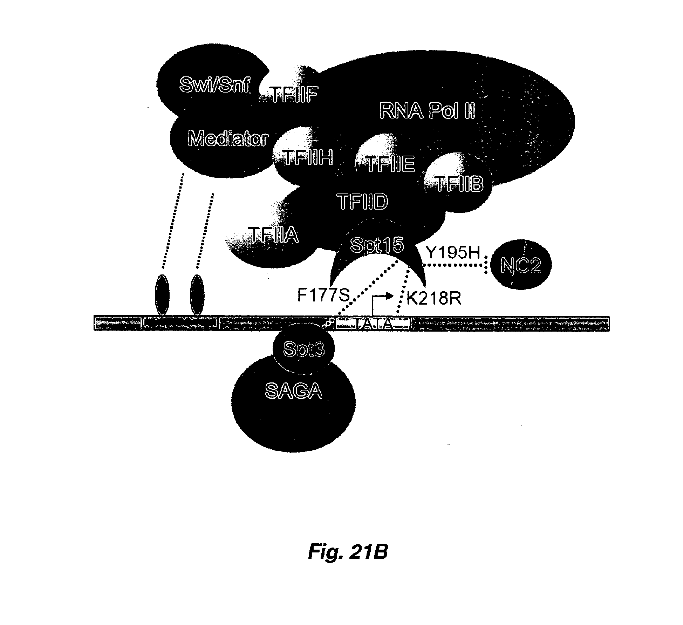

Figure 21 shows the elucidation and validation of a mechanism partially mediated by the *SPT3*/SAGA complex. (A) The impact of an *spt3* knockout was evaluated through the introduction of the *spt15-300* mutant and assaying in the presence of 6% ethanol by volume. The incapacity of the mutant to impart the phenotype illustrates the essentiality of *SPT3* as a part of the mechanism provided. (B) The three mutations (F177S, Y195H, and K218R) are mapped on the global transcription machinery molecular mechanism proposed by prior studies with each of these mutation sites (*22-24*, *27*, *28*). Collectively, these three mutations lead to a mechanism involving Spt3p.

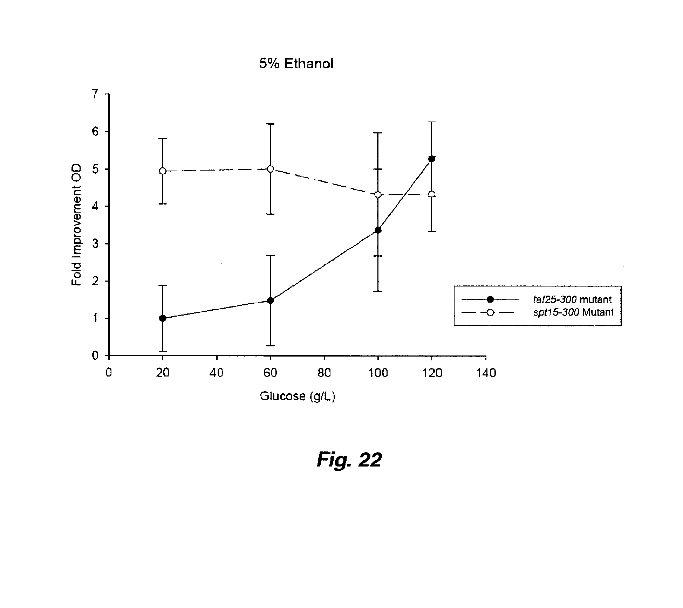

Figure 22 shows growth yields of the best clones isolated from the *taf25* and *spt15* mutant library, respectively, in a synthetic minimal medium containing elevated levels (5% by volume) of ethanol and glucose were measured after 20 hours.
Figure 23: Viability of the *spt15-300* mutant strain compared with the control is measured as a function of time (hours) and expressed as the relative number of colony forming units compared with colony count at 0 hours for stationary phase cells treated and incubated in standard medium in the presence of (A) 10%, (B) 17% and (C) 20% ethanol by volume. Insets are provided for 17.5% and 20% ethanol to better depict the differences between the mutant and the control harboring the wild-type version of the SPT15. The *spt15-300* mutation confers a significantly enhanced viability at all concentrations tested above 10% ethanol by volume (see also Fig. 2A, 2B). Error bars represent the standard deviation between biological replicate experiments. Initial cell counts were approximately 3.5x10⁶ cells/ml.

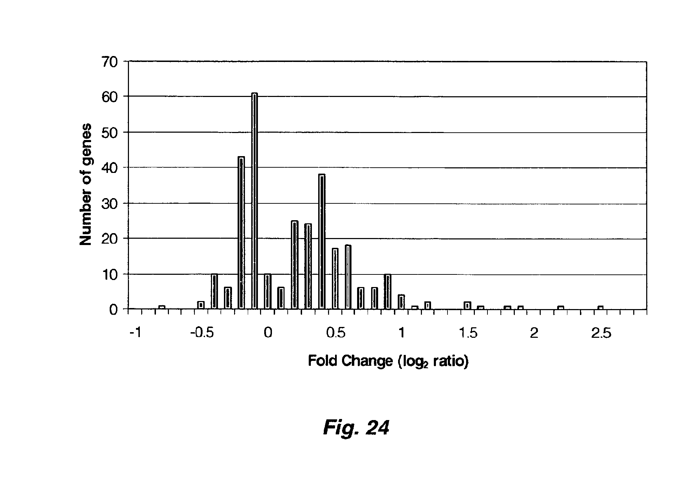

Figure 24 provides a histogram of differentially expressed genes in the *spt15-300* mutant strain compared with the control at a statistical threshold of p-value ≤ 0.001. This *spt15-300* has a bias for imparting an upregulation over a downregulation of genes.

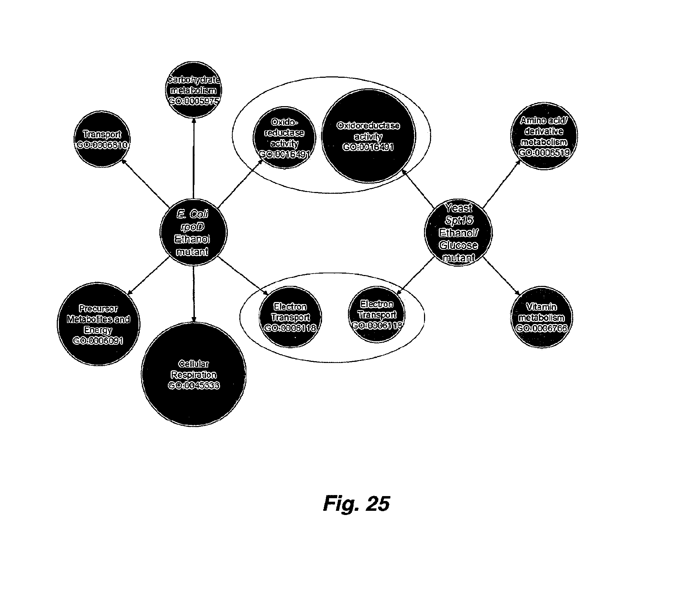

Figure 25: Gene ontology enrichment of altered genes was compared between the *E*. *coli* ethanol tolerant sigma factor mutant and the yeast *spt15-300* mutant tolerant to elevated ethanol and glucose. This comparison illustrates that despite differences in the transcription machinery, both were able to elicit a similar, conserved response of altered oxidoreductase and electron transport genes. These protein functions play an important role in ethanol tolerance in these strains. The size of the circle is proportional to the p-value of functional enrichment.
Figure 26: Gene overexpression studies are provided for the top 3 candidate genes from the microarray (*PHO5, PHM6,* and *FMP16*) and assayed under 5% ethanol by (see also Fig. 3B).

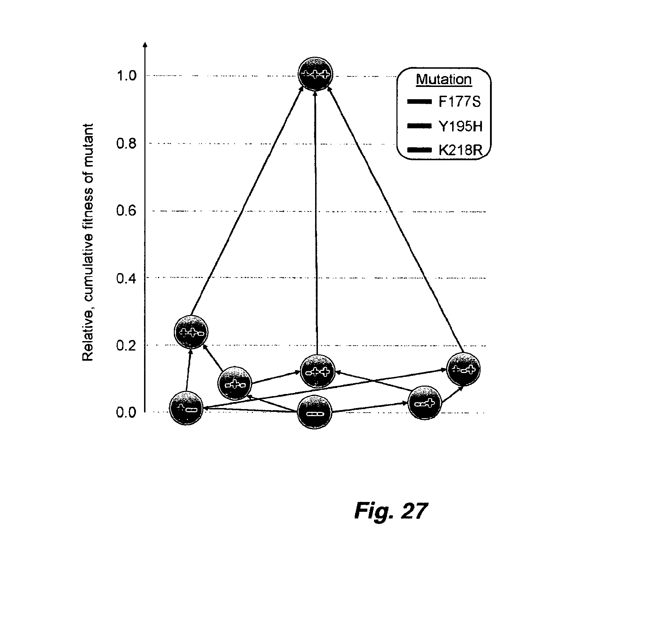

Figure 27: An exhaustive evaluation of single and double mutations leading to the triple *spt15* mutant illustrates that no single mutation or combination of doubles performs as well as the identified triple mutant. A cumulative, relative fitness is plotted on the y-axis as well as trajectories (by color) for each of the modifications. Supplemental text, part d provides data for each of the mutants and an explanation of the fitness metric.

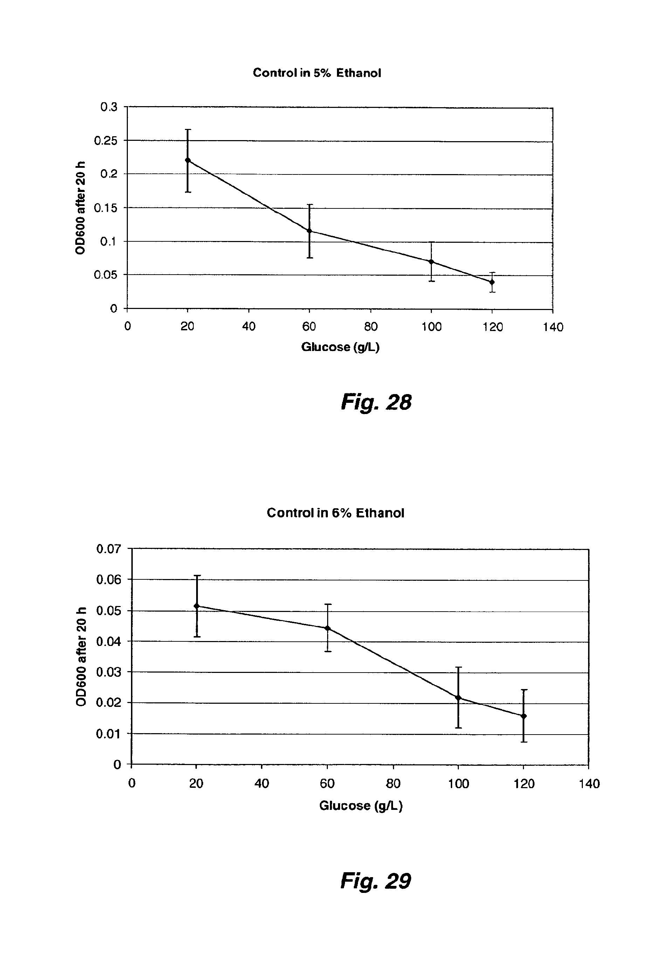

Figure 28 depicts the growth of control strain in the presence of 5% ethanol and various glucose concentrations after 20 hours of incubation.
Figure 29 depicts the growth of control strain in the presence of 6% ethanol and various glucose concentrations after 20 hours of incubation.

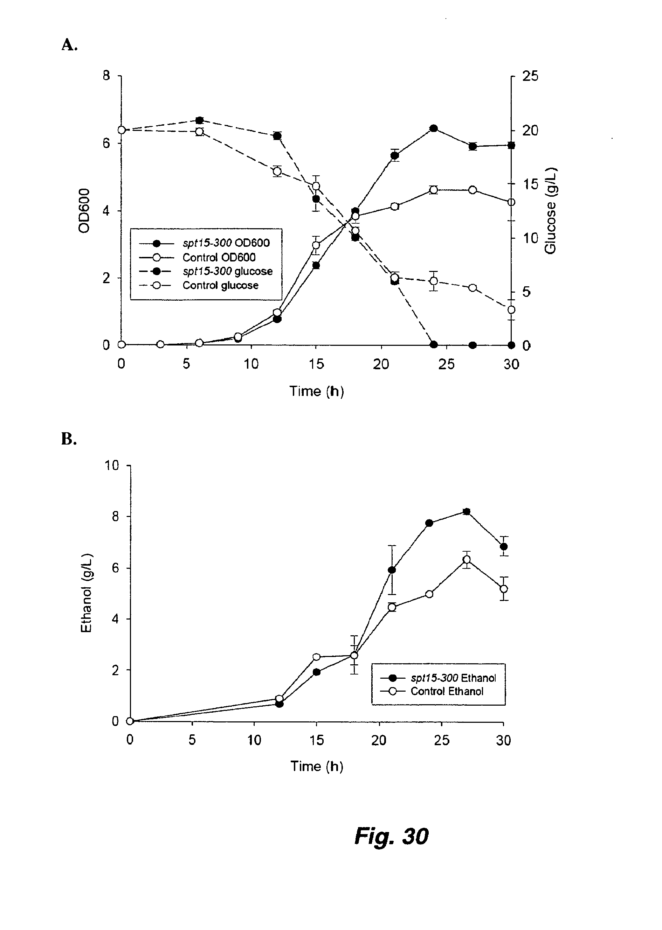

Figure 30 shows the glucose, cell density, and ethanol profile for the mutant and control in a low inoculum fermentation with 20 g/L of glucose. Growth rate was similar between the mutant and control, but growth continued with an extended growth phase (A). Ethanol yield was also higher in the mutant (B).

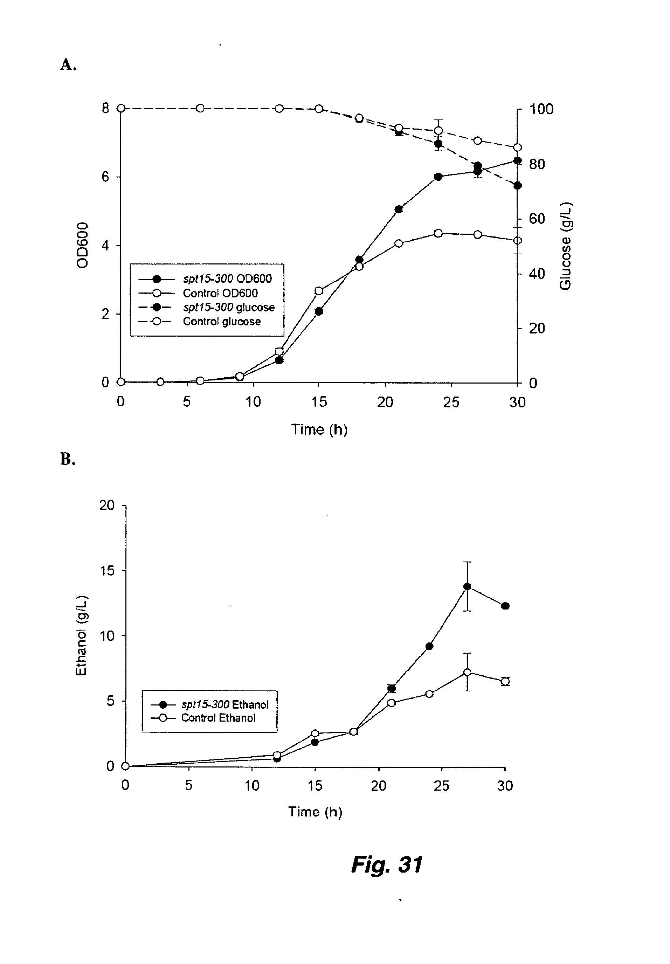

Figure 31 shows the glucose, cell density, and ethanol profile for the mutant and control in a low inoculum fermentation with 100 g/L of glucose. Glucose utilization rates and growth in the mutant strain exceed that of the control. Additionally, ethanol yield was higher in the mutant.

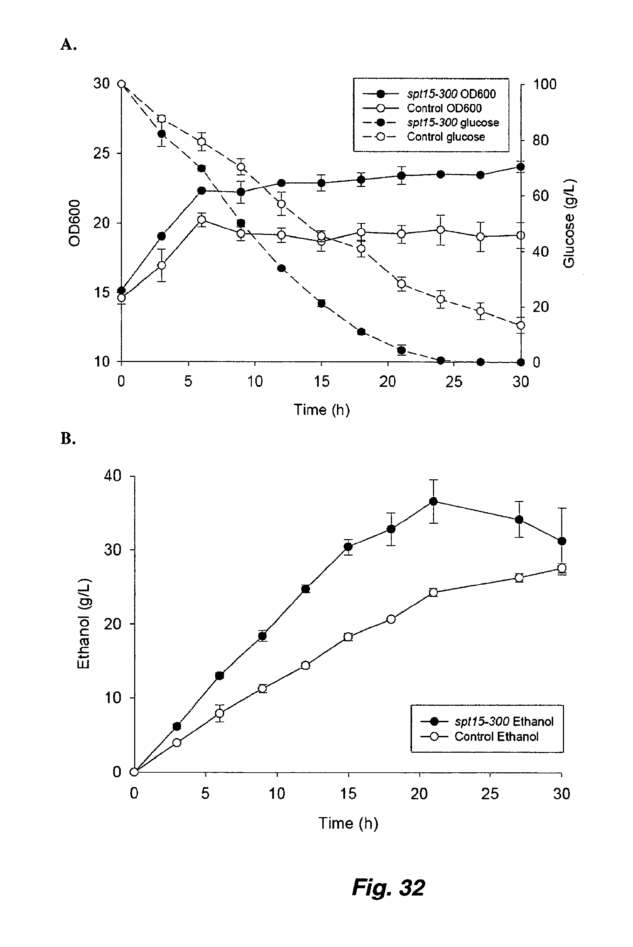

Figure 32: Cells were cultured in biological replicate u in 100 g/L of glucose with a high inoculum of initial cell density of OD 15 (∼4 g DCW/L). Exhibited by the profiles above, the mutant exhibits a more robust growth (higher growth yields), a complete utilization of glucose, and a higher ethanol productivity.

### Detailed Description of the Invention

Global transcription machinery is responsible for controlling the transcriptome in all cellular systems (prokaryotic and eukaryotic). In bacterial systems, the sigma factors play a critical role in orchestrating global transcription by focusing the promoter preferences of the RNA polymerase holoenzyme (R. R. Burgess, L. Anthony, Curr. Opin. Microbiol 4, 126-131 (2001)). *Escherichia coli* contains six alternative sigma factors and one principal factor, σ⁷⁰, encoded by the gene *rpoD.* On the protein level, regions of residues have been analyzed for contacts with promoter sites and the holoenzyme (J. T. Owens et al., PNAS 95, 6021-6026 (1998)). Crystal structure analysis and site specific mutagenesis of σ⁷⁰ in *E. coli* and other bacteria, have demonstrated the ability to alter the *in vitro* promoter preference of the RNA polymerase holoenzyme evidenced by increased or decreased transcription of a reporter gene (A. Malhotra, E. Severinova, S. A. Darst, Cell 87, 127-36 (1996)). This invention exploits the ability to generate mutant sigma factors with varying preferences for promoters on a genome-wide level.

Traditional strain improvement paradigms rely predominantly on making sequential, single-gene modifications and often fail to reach the global maxima. The reason is that metabolic landscapes are complex (H. Alper, K. Miyaoku, G. Stephanopoulos, Nat Biotechnol 23, 612-616 (2005); H. Alper, Y.-S. Jin, J. F. Moxley, G. Stephanopoulos, Metab Eng 7, 155-164 (2005)) and incremental or greedy search algorithms fail to uncover synthetic mutants that are beneficial only when all mutations are simultaneously introduced. Protein engineering on the other hand can quickly improve fitness, through randomized mutagenesis and selection for enhanced antibody affinity, enzyme specificity, or catalytic activity (E. T. Boder, K. S. Midelfort, K. D. Wittrup, Proc Natl Acad Sci U S A 97, 10701-5 (2000); A. Glieder, E. T. Farinas, F. H. Arnold, Nat Biotechnol 20, 1135-9 (2002); N. Varadarajan, J. Gam, M. J. Olsen, G. Georgiou, B. L. Iverson, Proc Natl Acad Sci U S A 102, 6855-60 (2005)). An important reason for the drastic enhancement obtained in these examples is the ability of these methods to probe a significant subset of the huge amino acid combinatorial space by evaluating many simultaneous mutations. Using the invention, we exploit the global regulatory functions of the σ⁷⁰ sigma factor to similarly introduce multiple simultaneous gene expression changes and thus facilitate whole-cell engineering by selecting mutants responsible for improved cellular phenotype.

The invention provides methods for altering the phenotype of a cell. In the methods include mutating a nucleic acid encoding a global transcription machinery protein and, optionally, its promoter, expressing the nucleic acid in a cell to provide an altered cell that includes a mutated global transcription machinery protein, and culturing the altered cell. As used herein, "global transcription machinery" is one or more molecules that modulates the transcription of a plurality of genes. The global transcription machinery can be proteins that affect gene transcription by interacting with and modulating the activity of a RNA polymerase molecule. The global transcription machinery also can be proteins that alter the ability of the genome of a cell to be transcribed (e.g., methyltransferases, histone methyltransferases, histone acetylases and deacetylases). Further, global transcription machinery can be molecules other than proteins (e.g., micro RNAs) that alter transcription of a plurality of genes.

Global transcription machinery useful in accordance with the invention include bacterial sigma factors and anti-sigma factors. Exemplary genes that encode sigma factors include *rpoD,* encoding σ⁷⁰; *rpoF,* encoding σ²⁸; *rpoS,* encoding σ³⁸; *rpoH,* encoding σ³²; *rpoN,* encoding σ⁵⁴; *rpoE,* encoding σ²⁴; and *fecI,* encoding σ¹⁹. Anti-sigma factors bind to the sigma factors and control their availability and consequently transcription. In *E. coli,* anti-sigma factors are encoded by *rsd* (for sigma factor 70) or *flgM*, among others. The anti-sigma factors can be mutated to control their impact in transcription for normal cells. In addition, novel pairings of mutant sigma factors with mutant anti-sigma factors can be created to create further control of transcription in cells. For example, the anti-sigma factor can be expressed using an inducible promoter, which allows for tunable control of the phenotype imparted by the mutant sigma factor.

Global transcription machinery also includes polypeptides that bind to and modulate the activity of eukaryotic RNA polymerases, such as RNA polymerase I, RNA polymerase II or RNA polymerase III, or a promoter of RNA polymerase I, RNA polymerase II or RNA polymerase III. Examples of such eukaryotic global transcription machinery are TFIID or a subunit thereof, such as TATA-binding protein (TBP) or a TBP-associated factor (TAF) such as TAF25, and elongation factors. Examples of TBPs from various species include. NP_011075.1; AAA35146.1; XP_447540.1; NP_986800.1; XP_454405.1; 1YTB; 1TBP; XP_462043.1; AAA79367.1; XP_501249.1; NP_594566.1; AAA79368.1; AAY23352.1; Q12731; BAE57713.1; XP_001213720.1; XP_364033.1; XP_960219.1; CAJ41964.1; EAT85966.1; XP_754608.1; XP_388603.1; P26354; EAU88086.1; XP_758541.1; XP_662580.1; XP_710759.1; XP_572300.1; and BAB92075.1. Further examples of global transcription machinery from yeast include GAL11, SIN4, RGR1, HRS 1, PAF1, MED2, SNF6, SNF2, and SWI1.

Global transcription machinery also includes polypeptides that alter the ability of chromosomal DNA to be transcribed, such as nucleic acid methyltransferases (e.g., DamMT, DNMT1, Dnmt3a); histone methyltransferases (e.g., Set1, MLL1); histone acetylases (e.g., PCAF, GCN5, Sas2p and other MYST-type histone acetylases, TIP60); and histone deacetylases (e.g., HDAC1, HDA1, HDAC2, HDAC3, RPD3, HDAC8, Sir2p), as well as associated factors (e.g., HDACs are associated with mSin3A, Mi-2/NRD, CoREST/kiaa0071, N-CoR and SMRT).

Still other global transcription machinery is encoded by nucleic acid molecules of an organelle of a eukaryotic cell, such as a mitochondrion or a chloroplast.

The foregoing examples of global transcription machinery are only meant to be illustrative. As will be known to the person of skill in the art, many other examples of global transcription machinery are known and many other examples of the aforementioned examples from other species are known. The invention included the use of all of the foregoing.

In addition, the global transcription machinery useful in accordance with the invention includes sequences that are at least X% identical to molecules of interest. For example, molecules that share identical sequences with the *S*. *cerevisiae* TBP SPT15, i.e., homologs of SPT15, are contemplated for use in accordance with the invention. Such homologs are at least about 70% identical, preferably at least about 75% identical, more preferably at least about 80% identical, still more preferably at least about 85% identical, still more preferably at least about 90% identical, still more preferably at least about 95% identical, still more preferably at least about 97% identical, and most preferably at least about 99% identical.

In many instances, the process of mutating the global transcription machinery will include iteratively making a plurality of mutations of the global transcription machinery, but it need not, as even a single mutation of the global transcription machinery can result in dramatic alteration of phenotype, as is demonstrated herein.

While the methods of the invention typically are carried out by mutating the global transcription machinery followed by introducing the mutated global transcription machinery into a cell to create an altered cell, it is also possible to mutate endogenous global transcription machinery genes, e.g., by replacement with mutant global transcription machinery or by *in situ* mutation of the endogenous global transcription machinery. As used herein, "endogenous" means native to the cell; in the case of mutating global transcription machinery, endogenous refers to the gene or genes of the global transcription machinery that are in the cell. In contrast, the more typical methodology includes mutation of a global transcription machinery gene or genes outside of the cell, followed by introduction of the mutated gene(s) into the cell.

The global transcription machinery genes can be of the same species or different species as the cell into which they are introduced. For example, as shown herein, *E. coli* sigma factor 70 was mutated and introduced into *E. coli* to alter the phenotype of the *E coli* cells. Other global transcription machinery of *E. coli* also could be used in the same fashion. Similarly, global transcription machinery of a particular yeast species, e.g., *S*. *cerevisiae* or *S*. *pombe,* could be mutated and introduced into the same yeast species. Likewise, global transcription machinery of a nematode species, e.g., *C*. *elegans,* or a mammalian species, e.g., *M. musculus, R. norvegicus* or *H. sapiens,* can be mutated and introduced into the same species in a manner similar to the specific examples provided herein, using standard recombinant genetic techniques.

Alternatively, global transcription machinery from different species can be utilized to provide additional variation in the transcriptional control of genes. For example, global transcription machinery of a *Streptomyces* bacterium could be mutated and introduced into *E*. *coli.* The different global transcription machinery also could be sourced from different kingdoms or phyla of organisms. Depending on the method of mutation used, same and different global transcription machinery can be combined for use in the methods of the invention, e.g., by gene shuffling.

Optionally, the transcriptional control sequences of global transcription machinery can be mutated, rather than the coding sequence itself. Transcriptional control sequences include promoter and enhancer sequences. The mutated promoter and/or enhancer sequences, linked to the global transcription machinery coding sequence, can then be introduced into the cell.

After the mutant global transcription machinery is introduced into the cell to make an altered cell, then the phenotype of the altered cell is determined/assayed. This can be done by selecting altered cells for the presence (or absence) of a particular phenotype. Examples of phenotypes are described in greater detail below. The phenotype also can be determined by comparing the phenotype of the altered cell with the phenotype of the cell prior to alteration.

In preferred embodiments, the mutation of the global transcription machinery and introduction of the mutated global transcription machinery are repeated one or more times to produce an "n^{th} generation" altered cell, where "n" is the number of iterations of the mutation and introduction of the global transcription machinery. For example, repeating the mutation and introduction of the global transcription machinery once (after the initial mutation and introduction of the global transcription machinery) results in a second generation altered cell. The next iteration results in a third generation altered cell, and so on. The phenotypes of the cells containing iteratively mutated global transcription machinery then are determined (or compared with a cell containing non-mutated global transcription machinery or a previous iteration of the mutant global transcription machinery) as described elsewhere herein.

The process of iteratively mutating the global transcription machinery allows for improvement of phenotype over sequential mutation steps, each of which may result in multiple mutations of the global transcription machinery. It is also possible that the iterative mutation may result in mutations of particular amino acid residues "appearing" and "disappearing" in the global transcription machinery over the iterative process. Examples of such mutations are provided in the working examples.

In a typical use of the methodology, the global transcription machinery is subjected to directed evolution by mutating a nucleic acid molecule that encodes the global transcription machinery. A preferred method to mutate the nucleic acid molecule is to subject the coding sequence to mutagenesis, and then to insert the nucleic acid molecule into a vector (e.g., a plasmid). This process may be inverted if desired, i.e., first insert the nucleic acid molecule into a vector, and then subject the sequence to mutagenesis, although it is preferred to mutate the coding sequence prior to inserting it in a vector.

When the directed evolution of the global transcription machinery is repeated, i.e., in the iterative processes of the invention, a preferred method includes the isolation of a nucleic acid encoding the mutated global transcription machinery and optionally, its promoter, from the altered cell. The isolated nucleic acid molecule is then mutated (producing a nucleic acid encoding a second generation mutated global transcription machinery), and subsequently introduced into another cell.

The isolated nucleic acid molecule when mutated, forms a collection of mutated nucleic acid molecules that have different mutations or sets of mutations. For example, the nucleic acid molecule when mutated randomly can have set of mutations that includes mutations at one or more positions along the length of the nucleic acid molecule. Thus, a first member of the set may have one mutation at nucleotide *n1* (wherein *nx* represents a number of the nucleotide sequence of the nucleic acid molecule, with *x* being the position of the nucleotide from the first to the last nucleotide of the molecule). A second member of the set may have one mutation at nucleotide *n2.* A third member of the set may have two mutations at nucleotides *n1* and *n3.* A fourth member of the set may have two mutations at positions *n4* and *n5.* A fifth member of the set may have three mutations: two point mutations at nucleotides *n4* and *n5,* and a deletion of nucleotides *n6-n7.* A sixth member of the set may have point mutations at nucleotides *n1, n5* and *n8,* and a truncation of the 3' terminal nucleotides. A seventh member of the set may have nucleotides *n9-n10* switched with nucleotides *n11-n12.* Various other combinations can be readily envisioned by one of ordinary skill in the art, including combinations of random and directed mutations.

The collection of nucleic acid molecules can be a library of nucleic acids, such as a number of different mutated nucleic acid molecules inserted in a vector. Such a library can be stored, replicated, aliquotted and/or introduced into cells to produce altered cells in accordance with standard methods of molecular biology.

Mutation of the global transcription machinery for directed evolution preferably is random. However, it also is possible to limit the randomness of the mutations introduced into the global transcription machinery, to make a non-random or partially random mutation to the global transcription machinery, or some combination of these mutations. For example, for a partially random mutation, the mutation(s) may be confined to a certain portion of the nucleic acid molecule encoding the global transcription machinery.

The method of mutation can be selected based on the type of mutations that are desired. For example, for random mutations, methods such as error-prone PCR amplification of the nucleic acid molecule can be used. Site-directed mutagenesis can be used to introduce specific mutations at specific nucleotides of the nucleic acid molecule. Synthesis of the nucleic acid molecules can be used to introduce specific mutations and/or random mutations, the latter at one or more specific nucleotides, or across the entire length of the nucleic acid molecule. Methods for synthesis of nucleic acids are well known in the art (e.g., Tian et al., Nature 432: 1050-1053 (2004)).

DNA shuffling (also known as gene shuffling) can be used to introduce still other mutations by switching segments of nucleic acid molecules. See, e.g., US patent 6,518,065, related patents, and references cited therein. The nucleic acid molecules used as the source material to be shuffled can be nucleic acid molecule(s) that encode(s) a single type of global transcription machinery (e.g., σ⁷⁰), or more than one type of global transcription machinery. For example, nucleic acid molecules encoding different global transcription machinery, such as different sigma factors of a single species (e.g., σ⁷⁰ and σ²⁸ of *E. coli*), or sigma factors from different species can be shuffled. Likewise, nucleic acid molecules encoding different types of global transcription machinery, e.g., sigma factor 70 and TFIID, can be shuffled.

A variety of other methods of mutating nucleic acid molecules, in a random or non-random fashion, are well known to one of ordinary skill in the art. One or more different methods can be used combinatorially to make mutations in nucleic acid molecules encoding global transcription machinery. In this aspect, "combinatorially" means that different types of mutations are combined in a single nucleic acid molecule, and assorted in a set of nucleic acid molecules. Different types of mutations include point mutations, truncations of nucleotides, deletions of nucleotides, additions of nucleotides, substitutions of nucleotides, and shuffling (e.g., re-assortment) of segments of nucleotides. Thus, any single nucleic acid molecule can have one or more types of mutations, and these can be randomly or non-randomly assorted in a set of nucleic acid molecules. For example, a set of nucleic acid molecules can have a mutation common to each nucleic acid molecule in the set, and a variable number of mutations that are not common to each nucleic acid molecule in the set.

The common mutation, for example, may be one that is found to be advantageous to a desired altered phenotype of the cell.

Preferably a promoter binding region of the global transcription machinery is not disrupted or removed by the one or more truncations or deletions.

The mutated global transcription machinery can exhibit increased or decreased transcription of genes relative to the unmutated global transcription machinery. In addition, the mutated global transcription machinery can exhibit increased or decreased repression of transcription of genes relative to the unmutated global transcription machinery.

As used herein, a "vector" may be any of a number of nucleic acids into which a desired sequence may be inserted by restriction and ligation for transport between different genetic environments or for expression in a host cell. Vectors are typically composed of DNA although RNA vectors are also available. Vectors include, but are not limited to: plasmids, phagemids, virus genomes and artificial chromosomes.

A cloning vector is one which is able to replicate autonomously or integrated in the genome in a host cell, and which is further characterized by one or more endonuclease restriction sites at which the vector may be cut in a determinable fashion and into which a desired DNA sequence may be ligated such that the new recombinant vector retains its ability to replicate in the host cell. In the case of plasmids, replication of the desired sequence may occur many times as the plasmid increases in copy number within the host bacterium or just a single time per host before the host reproduces by mitosis. In the case of phage, replication may occur actively during a lytic phase or passively during a lysogenic phase.

An expression vector is one into which a desired DNA sequence may be inserted by restriction and ligation such that it is operably joined to regulatory sequences and may be expressed as an RNA transcript. Vectors may further contain one or more marker sequences suitable for use in the identification of cells which have or have not been transformed or transfected with the vector. Markers include, for example, genes encoding proteins which increase or decrease either resistance or sensitivity to antibiotics or other compounds, genes which encode enzymes whose activities are detectable by standard assays known in the art (e.g., β-galactosidase, luciferase or alkaline phosphatase), and genes which visibly affect the phenotype of transformed or transfected cells, hosts, colonies or plaques (e.g., green fluorescent protein). Preferred vectors are those capable of autonomous replication and expression of the structural gene products present in the DNA segments to which they are operably joined.

As used herein, a coding sequence and regulatory sequences are said to be "operably" joined when they are covalently linked in such a way as to place the expression or transcription of the coding sequence under the influence or control of the regulatory sequences. If it is desired that the coding sequences be translated into a functional protein, two DNA sequences are said to be operably joined if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein. Thus, a promoter region would be operably joined to a coding sequence if the promoter region were capable of effecting transcription of that DNA sequence such that the resulting transcript might be translated into the desired protein or polypeptide.

The precise nature of the regulatory sequences needed for gene expression may vary between species or cell types, but shall in general include, as necessary, 5' non-transcribed and 5' non-translated sequences involved with the initiation of transcription and translation respectively, such as a TATA box, capping sequence, CAAT sequence, and the like. In particular, such 5' non-transcribed regulatory sequences will include a promoter region which includes a promoter sequence for transcriptional control of the operably joined gene. Regulatory sequences may also include enhancer sequences or upstream activator sequences as desired. The vectors of the invention may optionally include 5' leader or signal sequences. The choice and design of an appropriate vector is within the ability and discretion of one of ordinary skill in the art.

Expression vectors containing all the necessary elements for expression are commercially available and known to those skilled in the art. See, e.g., Sambrook et al., Molecular Cloning: A Laboratory Manual, Second Edition, Cold Spring Harbor Laboratory Press, 1989. Cells are genetically engineered by the introduction into the cells of heterologous DNA (RNA) encoding a CT antigen polypeptide or fragment or variant thereof. That heterologous DNA (RNA) is placed under operable control of transcriptional elements to permit the expression of the heterologous DNA in the host cell.

Preferred systems for mRNA expression in mammalian cells are those such as pRc/CMV or pcDNA3.1 (available from Invitrogen, Carlsbad, CA) that contain a selectable marker such as a gene that confers G418 resistance (which facilitates the selection of stably transfected cell lines) and the human cytomegalovirus (CMV) enhancer-promoter sequences. Additionally, suitable for expression in primate or canine cell lines is the pCEP4 vector (Invitrogen), which contains an Epstein Barr Virus (EBV) origin of replication, facilitating the maintenance of plasmid as a multicopy extrachromosomal element.

When the nucleic acid molecule that encodes mutated global transcription machinery is expressed in a cell, a variety of transcription control sequences (e.g., promoter/enhancer sequences) can be used to direct expression of the global transcription machinery. The promoter can be a native promoter, i.e., the promoter of the global transcription machinery gene, which provides normal regulation of expression of the global transcription machinery. The promoter also can be one that is ubiquitously expressed, such as beta-actin, ubiquitin B, phage promoters or the cytomegalovirus promoter. A promoter useful in the invention also can be one that does not ubiquitously express the global transcription machinery. For example, the global transcription machinery can be expressed in a cell using a tissue-specific promoter, a cell-specific promoter, or an organelle-specific promoter. A variety of conditional promoters also can be used, such as promoters controlled by the presence or absence of a molecule, such as the tetracycline-responsive promoter (M. Gossen and H. Bujard, Proc. Natl Acad. Sci. USA, 89, 5547-5551 (1992)).

A nucleic acid molecule that encodes mutated global transcription machinery can be introduced into a cell or cells using methods and techniques that are standard in the art. For example, nucleic acid molecules can be introduced by various transfection methods, transduction, electroporation, particle bombardment, injection (including microinjection of cells and injection into multicellular organisms), lipofection, yeast spheroplast/cell fusion for YACs (yeast artificial chromosomes), *Agrobacterium*-mediated transformation for plant cells, etc..

Expressing the nucleic acid molecule encoding mutated global transcription machinery also may be accomplished by integrating the nucleic acid molecule into the genome or by replacing a nucleic acid sequence that encodes the endogenous global transcription machinery.

By mutating global transcription machinery, novel compositions are provided, including nucleic acid molecules encoding global transcription machinery produced by a plurality of rounds of mutation. The plurality of rounds of mutation can include directed evolution, in which each round of mutation is followed by a selection process to select the mutated global transcription machinery that confer a desired phenotype. The methods of mutation and selection of the mutated global transcription machinery are as described elsewhere herein. Global transcription machinery produced by these nucleic acid molecules also are provided.

In certain cases, it has been found that mutated global transcription machinery are truncated forms of the unmutated global transcription machinery. In particular, for sigma factor 70, it has been found that an amino-terminal truncation of σ⁷⁰ that leaves only the carboxyl-terminus of the σ⁷⁰ protein confers advantageous phenotypes to bacteria in which it is introduced. Thus, fragments of global transcription machinery are provided, particularly fragments that retain the promoter binding properties of the unmutated global transcription machinery, more particularly σ⁷⁰ fragments that include region 4. Nucleic acid molecules encoding the truncated global transcription machinery also are provided, including nucleic acid molecules as contained in vectors and/or cells.

The cells useful in the invention include prokaryotic cells and eukaryotic cells. Prokaryotic cells include bacterial cells and archaeal cells. Eukaryotic cells include yeast cells, mammalian cells, plant cells, insect cells, stem cells, and fungus cells. Eukaryotic cells may be contained in, e.g., part of or all of, a multicellular organism. Multicellular organisms include mammals, nematodes such as *Caenorhabditis elegans,* plants such as *Arabidopsis thaliana, Bombyx mori, Xenopus laevis,* zebrafish (*Danio rerio*), sea urchin and *Drosophila melanogaster.*

Examples of bacteria include *Escherichia* spp., *Streptomyces* spp., *Zymonas* spp., *Acetobacter* spp., *Citrobacter* spp., *Synechocystis* spp., *Rhizobium* spp., *Clostridium* spp., *Corynebacterium* spp., *Streptococcus* spp., *Xanthomonas* spp., *Lactobacillus* spp., *Lactococcus* spp., *Bacillus* spp., *Alcaligenes* spp., *Pseudomonas* spp., *Aeromonas* spp., *Azotobacter* spp., *Comamonas* spp., *Mycobacterium* spp., *Rhodococcus* spp., *Gluconobacter* spp., *Ralstonia* spp., *Acidithiobacillus* spp., *Microlunatus* spp., *Geobacter* spp., *Geobacillus* spp., *Arthrobacter* spp., *Flavobacterium* spp., *Serratia* spp., *Saccharopolyspora* spp., *Thermus* spp., *Stenotrophomonas* spp., *Chromobacterium* spp., *Sinorhizobium* spp., *Saccharopolyspora* spp., *Agrobacterium* spp. and *Pantoea* spp.

Examples of archaea (also known as archaebacteria) include *Methylomonas* spp., *Sulfolobus* spp., *Methylobacterium* spp. *Halobacterium* spp., *Methanobacterium* spp., *Methanococci* spp., *Methanopyri* spp., *Archaeoglobus* spp., *Ferroglobus* spp., *Thermoplasmata* spp. and *Thermococci* spp.

Examples of yeast include *Saccharomyces* spp., *Schizosaccharomyces* spp., *Pichia* spp., *Paffia* spp., *Kluyveromyces* spp., *Candida* spp., *Talaromyces* spp., *Brettanomyces* spp., *Pachysolen* spp., *Debaryomyces* spp., and industrial polyploid yeast strains.

Examples of fungi include *Aspergillus* spp., *Pennicilium* spp., *Fusarium* spp., *Rhizopus* spp., *Acremonium* spp., *Neurospora* spp., *Sordaria* spp., *Magnaporthe* spp., *Allomyces* spp., *Ustilago* spp., *Botrytis* spp., and *Trichoderma* spp.

Examples of insect cells include *Spodoptera frugiperda* cell lines such as Sf9 and Sf21, *Drosophila melanogaster* cell lines such as Kc, Ca, 311, DH14, DH15, DH33P1, P2, P4 and SCHNEIDER-2 (D. Mel- S2) and *Lymantria dispar* cedll lines such as 652Y.

Examples of mammalian cells include primary cells, such as stem cells and dendritic cells, and mammalian cell lines such as Vero, HEK 293, Sp2/0, P3UI, CHO, COS, HeLa, BAE-1, MRC-5, NIH 3T3, L929, HEPG2, NS0, U937, HL60, YAC1, BHK, ROS, Y79, Neuro2a, NRK, MCF-10, RAW 264.7, and TBY-2.

Stem cell lines include hESC BG01, hESC BG01V, ES-C57BL/6, ES-D3 GL, J1, R1, RW.4, 7AC5/EYFP, and R1/E. Additional human stem cell lines include (NIH designations) CH01, CH02, GE01, GE07, GE09, GE13, GE14, GE91, GE92, SA19, MB01, MB02, MB03, NC01, NC02, NC03, RL05, RL07, RL10, RL13, RL15, RL20, and RL21.

Directed evolution of global transcription machinery produces altered cells, some of which have altered phenotypes. Thus the invention also includes selecting altered cells for a predetermined phenotype or phenotypes. Selecting for a predetermined phenotype can be accomplished by culturing the altered cells under selective conditions. Selecting for a predetermined phenotype also can be accomplished by high-throughput assays of individual cells for the phenotype. For example, cells can be selected for tolerance to deleterious conditions and/or for increased production of metabolites. Tolerance phenotypes include tolerance of solvents such as ethanol, and organic solvents such as hexane or cyclohexane; tolerance of toxic metabolites such as acetate, para-hydroxybenzoic acid (pHBA), para-hydroxycinnamic acid, hydroxypropionaldehyde, overexpressed proteins, organic solvents and immuno-suppressant molecules; tolerance of surfactants; tolerance of osmotic stress; tolerance of high sugar concentrations; tolerance of high temperatures; tolerance of extreme pH conditions (high or low); resistance to apoptosis; tolerance of toxic substrates such as hazardous waste; tolerance of industrial media; increased antibiotic resistance, etc. Selection for ethanol tolerance, organic solvent tolerance, acetate tolerance, para-hydroxybenzoic acid tolerance, SDS tolerance and antibiotic resistance are exemplified in the working examples. In other working examples, selection for increased production of lycopene and polyhydroxybutyrate are exemplified. In working examples with yeast cells, selection for high sugar (glucose) tolerance, osmotic stress (LiCl) tolerance, and multiple tolerance to both high glucose and ethanol concentrations are exemplified.

Additional phenotypes that are manifested in multicellular organisms also can be selected. Mutant versions of global transcription machinery can be introduced into mammalian or other eukaryotic cell lines, or even introduced into whole organism (e.g., through introduction into germ cells lines or injections into oocytes) to allow for a screening of phenotypes. Such phenotypes may or may not be manifested in a single cell of the organism, and include: one or more growth characteristics, generation time, resistance to one or more pests or diseases, production of fruit or other parts of a plant, one or more developmental changes, one or more lifespan alterations, gain or loss of function, increased robustness, etc.

As used herein with respect to altered cells containing mutated global transcription machinery, "tolerance" means that an altered cell is able to withstand the deleterious conditions to a greater extent than an unaltered cell, or a previously altered cell. For example, the unaltered or previously altered cell is a "parent" of the "child" altered cell, or the unaltered or previously altered cell is the (n-1)^{th} generation as compared to the cell being tested, which is n^{th} generation. "Withstanding the deleterious conditions" means that the altered cell has increased growth and/or survival relative to the unaltered or previously altered cell. This concept also includes increased production of metabolites that are toxic to cells.

With respect to tolerance of high sugar concentrations, such concentrations can be ≥ 100 g/L, ≥ 120 g/L, ≥ 140 g/L, ≥ 160 g/L,≥ 180 g/L, ≥ 200 g/L, ≥ 250 g/L, ≥ 300 g/L, ≥ 350 g/L, ≥ 400 g/L, ≥ 450 g/L, ≥ 500 g/L, etc. With respect to tolerance of high salt concentrations, such concentrations can be ≥ 1 M, ≥ 2 M, ≥ 3 M, ≥ 4 M, ≥ 5 M, etc. With respect to tolerance of high temperatures, the temperatures can be, e.g., ≥42°C, ≥44°C, ≥46°C, ≥48°C, ≥50°C for bacterial cells. Other temperature cutoffs may be selected according to the cell type used. With respect to tolerance of extreme pH, exemplary pH cutoffs are, e.g., ≥pH10, ≥pH11, ≥pH12, ≥pH13, or ≤pH4.0, ≤pH3.0, ≤pH2.0, ≤pH1.0. With respect to tolerance of surfactants, exemplary surfactant concentrations are ≥5% w/v, ≥6% w/v, ≥7% w/v, ≥8% w/v, ≥9% w/v, ≥10% w/v, ≥12% w/v, ≥15% w/v, etc. With respect to tolerance of ethanol, exemplary ethanol concentrations are ≥4% v/v, ≥5% v/v, ≥6% v/v, ≥7% v/v, ≥8% v/v, ≥9% v/v, ≥10% v/v, etc. With respect to tolerance of osmotic stress, exemplary concentrations (e.g., of LiCl) that induce osmotic stress are ≥100 mM, ≥150 mM, ≥200 mM, ≥250 mM, ≥300 mM, ≥350 mM, ≥400 mM, etc.

The invention includes obtaining increased production of metabolites by cells. As used herein, a "metabolite" is any molecule that is made or can be made in a cell. Metabolites include metabolic intermediates or end products, any of which may be toxic to the cell, in which case the increased production may involve tolerance of the toxic metabolite. Thus metabolites include small molecules, peptides, large proteins, lipids, sugars, etc. Exemplary metabolites include the metabolites demonstrated in the working examples (lycopene, polyhydroxybutyrate and ethanol); therapeutic proteins, such as antibodies or antibody fragments.

The invention also provides for selecting for a plurality of phenotypes, such as tolerance of a plurality of deleterious conditions, increased production of a plurality of metabolites, or a combination of these. An example of this is the multiple tolerance of high glucose and ethanol by yeast demonstrated in the working examples.

It may be advantageous to use cells that are previously optimized for the predetermined phenotype prior to introducing mutated global transcription machinery. Thus, in the production of lycopene, for example, rather than starting with a bacterial cell that produces only a small amount of lycopene, one preferentially uses a cell that produces a higher amount of lycopene, more preferably an optimized amount of lycopene. In such cases, the mutated global transcription machinery is used to further improve an already-improved phenotype.

Via the actions of the mutated global transcription machinery, the altered cells will have altered expression of genes. The methods of the invention can, in certain aspects, include identifying the changes in gene expression in the altered cell. Changes in gene expression can be identified using a variety of methods well known in the art. Preferably the changes in gene expression are determined using a nucleic acid microarray.

In some aspects of the invention, one or more of the changes in gene expression that are produced in a cell by mutated global transcription machinery can be reproduced in another cell in order to produce the same (or a similar) phenotype. The changes in gene expression produced by the mutated global transcription machinery can be identified as described above. Individual gene(s) can then be targeted for modulation, through recombinant gene expression or other means. For example, mutated global transcription machinery may produce increases in the expression of genes A, B, C, D, and E, and decreases in the expression of genes F, G, and H. The invention includes modulating the expression of one or more of these genes in order to reproduce the phenotype that is produced by the mutated global transcription machinery. To reproduce the predetermined phenotype, one or more of genes A, B, C, D, E, F, G, and H can be increased, e.g., by introducing into the cell expression vector(s) containing the gene sequence(s), increasing the transcription of one or more endogenous genes that encode the one or more gene products, or by mutating a transcriptional control (e.g., promoter/enhancer) sequence of the one or more genes, or decreased, e.g., by introducing into the first cell nucleic acid molecules that reduce the expression of the one or more gene products such as nucleic acid molecules are, or express, siRNA molecules, or by mutating one or more genes that encode the one or more gene products or a transcriptional control (e.g., promoter/enhancer) sequence of the one or more genes.

Optionally, the changes in gene expression in the cell containing the mutated global transcription machinery are used to construct a model of a gene or protein network, which then is used to select which of the one or more gene products in the network to alter. Models of gene or protein networks can be produced via the methods of Ideker and colleagues (see, e.g., Kelley et al., Proc Natl Acad Sci USA 100(20), 11394-11399 (2003); Yeang et al. Genome Biology 6(7), Article R62 (2005); Ideker et al., Bioinformatics. 18 Suppl 1:S233-40 (2002)) or Liao and colleagues (see, e.g., Liao et al., Proc Natl Acad Sci USA 100(26), 15522-15527 (2003); Yang et al., BMC Genomics 6, 90 (2005)),

The invention also includes cells produced by any of the methods described herein, and multicellular organisms that contain such cells. The cells are useful for a variety of purposes, including: industrial production of molecules (e.g., many of the tolerance phenotypes and increased metabolite production phenotypes); bioremediation (e.g., hazardous waste tolerance phenotypes); identification of genes active in cancer causation (e.g., apoptosis resistance phenotypes) ; identification of genes active in resistance of bacteria and other prokaryotes to antibiotics; identification of genes active in resistance of pests to pesticides; etc.

In another aspect, the invention provides methods for altering the production of a metabolite. The methods include mutating global transcription machinery to produce an altered cell, in accordance with the methods described elsewhere herein. The cell preferably is a cell that produces a selected metabolite, and as described above, preferably is previously optimized for production of the metabolite. Altered cells that produce increased or decreased amounts of the selected metabolite can then be isolated. The methods also can include culturing the isolated cells and recovering the metabolite from the cells or the cell culture. The steps of culturing cells and recovering metabolite can be carried out using methods well known in the art. Various preferred cell types, global transcription machinery and metabolites are provided elsewhere herein.

As further exemplified herein, the invention includes genetically modified yeast strains that can be used to produce ethanol. Any of a wide variety of yeasts can be modified in accordance with the present invention and used to produce ethanol. Exemplary yeasts are mentioned above and include, e.g., yeasts of the genera *Saccharomyces, Schizosaccharomyces, Kluyveromyces, Candida, Pichia, Hansenula, Trichosporon, Brettanomyces, Pachysolen* and *Yamadazyma* and industrial polyploid yeast strains. In certain embodiments the yeast is *S*. *cerevisiae, K. marxianus, K. lactis, K. thermotolerans, C. sonorensis, C. methanosorbosa, C. diddensiae, C. parapsilosis, C. naeodendra, C. balnkii, C. entomophila, C. shecatae, P. tannophilus* or *P. stipitis. K. marxianus, C. sonorensis, C. shehatae, Pachysolen tannophilus* and *Pichia stipitis* are examples of yeast cells that grow on xylose. They have a natural xylulose-5-phosphate to glyceraldehyde-3-phosphate pathway, natural functional aldose and/or xylose reductase genes, active xylitol dehydrogenase genes, and natural ability to transport xylose through the cell wall or membrane. The yeast can be haploid, diploid, or polyploid (having more than two copies of some or all of its genome) in various embodiments of the invention.

In certain embodiments of the invention the yeast is genetically engineered to express or overexpress (relative to wild type levels) one or more proteins that confer an increased ability to take up or metabolize a sugar. The sugar may be, e.g., a monosaccharide, disaccharide, or oligosaccharide. The sugar may be one that is not normally utilized in significant amounts by the yeast. The sugar may be xylose, arabinose, etc. A number of approaches are known in the art to engineer yeast for xylose metabolism. See, e.g., Jeffries, et al., Curr. Op. Biotechnol., 17: 320-326, 2006 and references therein, which are incorporated herein by reference. The yeast may be engineered to carry out the pentose phosphate pathway (PPP), the biochemical route for xylose metabolism found in many organisms. Suitable proteins include, but are not limited to, xylose reductase, xylitol dehydrogenase, phosphoketolase, and transporters or permeases that facilitate substrate entry into cells. In certain embodiments the yeast is able to metabolize at least two sugars to ethanol, e.g., glucose and xylose.

In certain embodiments of the invention one or more proteins from a first microorganism, e.g., a yeast, is expressed in a second microorganism. For example, one or more genes from a yeast that naturally metabolizes xylose (e.g., *P. stipitis*) can be expressed in a yeast that does not efficiently utilize xylose or utilizes it less efficiently. In certain embodiments a gene encoding a protein in the pentose phosphate pathway is overexpressed. In certain embodiments the aldose reductase gene is deleted, disrupted, or otherwise rendered nonfunctional.

In certain embodiments a xylose-fermenting recombinant yeast strain expressing xylose reductase, xylitol dehydrogenase, and xylulokinase and having reduced expression of PHO13 or a PHO13 ortholog is used. See, e.g., U.S. Pat. Publication No. 2006/0228789. In certain embodiments the yeast is a recombinant yeast containing genes encoding xylose reductase, xylitol dehydrogenase and xylulokinase. See, e.g., U.S. Pat. No. 5,789,210. In certain embodiments the yeast is genetically engineered or selected to reduce or eliminate production of one or more secondary metabolic products such as glycerol. For example, in one embodiment a gene encoding a channel responsible for glycerol export, such as the *FPS1* gene in *S. cerevisiae,* is deleted, disrupted, or otherwise rendered nonfunctional. In certain embodiments a glutamine synthase gene is overexpressed, e.g., *GLT1* in *S. cerevisiae* (Kong, et al., Biotechnol. Lett., 28: 2033-2038, 2006). In certain embodiments the yeast strain is engineered or selected to have reduced formation of surplus NADH and/or increased consumption of ATP. In certain embodiments the gene encoding glutamine synthetase (*GLN1* in *S. cerevisiae*) is overexpressed. In certain embodiments the gene encoding glutamate synthase (*GLT1* in *S. cerevisiae*) is overexpressed. In certain embodiments the gene encoding the NADPH-dependent glutamate dehydrogenase (*GDH1* in *S. cerevisiae*) is deleted or rendered nonfunctional.

Any one or more of the afore-mentioned modifications could be made. For example, in one embodiment the glutamine synthetase and glutamate synthase genes are overexpressed, and the NADPH-dependent glutamate dehydrogenase is deleted or rendered nonfunctional (Nissen, et al., Metabolic Engineering, 2: 69-77, 2000). The proteins can be expressed using any of a wide variety of expression control sequences, e.g., promoters, enhancers, known in the art to function in the yeast of interest. The promoters may be constitutive or inducible. In certain embodiments a strong promoter is used. One of skill in the art will be able to select appropriate promoters for a particular yeast of interest. For example, the *S*. *cerevisiae PGK1* promoter could be used in yeast in which this promoter is active. It will be appreciated that additional elements such as terminators, etc., may be employed as appropriate. Genetically modified cells could contain one or more than one copy (e.g., between 2-10) of the exogenously introduced gene. Multiple copies of the exogenous gene may be integrated at a single locus (so they are adjacent each other), or at several loci within the host cell's genome. The exogenous gene could replace an endogenous gene (e.g., a modified TBP gene could replace the endogenous gene). The introduced gene could be integrated randomly into the genome or, in certain embodiments, maintained as an episome. Different exogenous genes can be under the control of different types of promoters and/or terminators. Genetic modification of cells can be accomplished in one or more steps via the design and construction of appropriate vectors and transformation of the cell with those vectors. Electroporation and/or chemical (such as calcium chloride- or lithium acetate-based) transformation methods can be used. Methods for transforming yeast strains are described in WO 99/14335, WO 00/71738, WO 02/42471, WO 03/102201, WO 03/102152, WO 03/049525 and other references mentioned herein and/or known in the art. In certain embodiments a selectable marker, e.g., an antibiotic resistance marker or nutritional marker is used to select transformants.

It will be appreciated that proteins exhibiting sequence homology and similar functions to the proteins described herein (e.g., TBP, enzymes involved in xylose metabolism) exist in a variety of different yeast and other fungal genera. One of skill in the art will be able to identify such proteins and the genes encoding them by searching publicly available databases such as Genbank and the scientific literature. In certain embodiments of the invention the protein is at least 80%, at least 90%, at least 95%, at least 98% identical, etc. Methods for determining %identity are known in the art. Standard methods may be used to clone homologous proteins from yeast in which the protein has not yet been identified. Such methods include functional cloning based on complementation, cloning based on nucleic acid hybridization, and expression cloning.

Yeast strains of the present invention may be further manipulated to achieve other desirable characteristics, or even higher ethanol tolerance and/or ethanol or other metabolite yields. For example, selection of recombinant yeast strains by sequentially transferring yeast strains of the present invention on medium containing appropriate substrates or growing them in continuous culture under selective conditions may result in improved yeast with enhanced tolerance and/or fermentation rates.

The above aspects of the invention may be applied to a variety of fungi in addition to yeast. The invention encompasses modifying the TBP gene of fungi in a similar manner to that described for yeast. The invention also encompasses introducing a modified yeast TBP gene into a fungus of interest. Suitable fungi include any fungus naturally capable of producing ethanol or genetically engineered to enable it to produce ethanol. For example, in certain embodiments the fungus is a *Neurospora* species (Colvin, et al., J Bacteriol., 116(3):1322-8, 1973). In other embodiments the fungus is an *Aspergillus* species (Abouzied, et al., Appl Environ Microbiol. 52(5):1055-9, 1986). In other embodiments the fungus is a *Paecilomyces* sp. (Wu, et al., Nature, 321(26): 887-888). The invention further includes use of co-cultures containing two or more microorganisms for the production of ethanol. For example, a co-culture may contain *S*. *cerevisiae* and at least one other fungus, e.g., an *Aspergillus* species.

Standard fermentation methods can be used in the present invention. For example, cells of the invention are cultured in a fermentation medium that includes a suitable sugar or sugars. In certain embodiments the sugars are hydrolysates of a cellulose- or hemicelluose-containing biomass. The fermentation medium may contain other sugars as well, notably hexose sugars such as dextrose (glucose) fructose, oligomers of glucose such as maltose, maltotriose and isomaltotriose, and panose. In case of oligomeric sugars, enzymes may be added to the fermentation broth in order to digest these to the corresponding monomeric sugar. The medium will typically contain nutrients as required by the particular cell including a source of nitrogen (such as amino acids proteins, inorganic nitrogen sources such as ammonia or ammonium salts, and the like), and various vitamins, minerals and the like. Other fermentation conditions, such as temperature, cell density, selection of substrate(s), selection of nutrients, etc., may be selected as known in the art. Temperatures during each of the growth phase and the production phase may, in certain embodiments, range from above the freezing temperature of the medium to about 50 degrees C. The optimal temperature may be selected based on the particular microorganism. During the production phase, the concentration of cells in the fermentation medium may range, in non-limiting embodiments between about 1-150, e.g., 3-10 g dry cells/liter of fermentation medium. The ability to achieve increased cell density using the modified strains of the present invention in a variety of different substrate concentrations is described in the examples. Yeast cultures having such densities are an aspect of the present invention.

The fermentation may be conducted aerobically, microaerobically or anaerobically in various embodiments of the invention. The process can be performed continuously, in batch mode, or using a combination thereof.

If desired, e.g., if an acid is produced during the fermentation process, the medium may be buffered during the production phase of the fermentation so that the pH is maintained in a range of about 5.0 to about 9.0, e.g., about 5.5 to about 7.0. Suitable buffering agents include basic materials, for example, calcium hydroxide, calcium carbonate, sodium hydroxide, potassium hydroxide, potassium carbonate, sodium carbonate, ammonium carbonate, ammonia, ammonium hydroxide and the like. In general those buffering agents that have been used in conventional fermentation processes are also suitable here. The process of the invention can be conducted continuously, batch-wise, or some combination thereof.

Another method provided in accordance with the invention is a method for bioremediation of a selected waste product. "Bioremediation", as used herein, is the use of microbes, such as bacteria and other prokaryotes, to enhance the elimination of toxic compounds in the environment. One of the difficulties in bioremediation is obtaining a bacterial strain or other microbe that effectively remediates a site, based on the particular toxins present at that site. The methods for altering the phenotype of cells described herein represents and ideal way to provide such bacterial strains. As one example, bioremediation can be accomplished by mutating global transcription machinery of a cell to produce an altered cell in accordance with the invention and isolating altered cells that metabolize an increased amount of the selected waste product relative to unaltered cells. The isolated altered cells then can be cultured, and exposed to the selected waste product, thereby providing bioremediation of the selected waste product. As an alternative, a sample of the materials in the toxic waste site needing remediation could serve as the selection medium, thereby obtaining microbes specifically selected for the particular mixture of toxins present at the particular toxic waste site.

The invention also provides collections of nucleic acid molecules, which may be understood in the art as a "library" of nucleic acid molecules using the standard nomenclature of molecular biology. Such collections/libraries include a plurality of different nucleic acid molecule species, with each nucleic acid molecule species encoding global transcription machinery that has different mutation(s) as described elsewhere herein.

Other collections/libraries of the invention are collections/libraries of cells that include the collections/libraries of nucleic acid molecules described above. The collections/libraries include a plurality of cells, with each cell of the plurality of cells including one or more of the nucleic acid molecules. The cell types present in the collection are as described elsewhere herein, and include single cells as well as multicellular organisms that include one or more of such cells. In the libraries of cells, the nucleic acid molecules can exist as extrachromosomal nucleic acids (e.g., on a plasmid), can be integrated into the genome of the cells, and can replace nucleic acids that encode the endogenous global transcription machinery.

The collections/libraries of nucleic acids or cells can be provided to a user for a number of uses. For example, a collection of cells can be screened for a phenotype desired by the user. Likewise, a collection of nucleic acid molecules can be introduced into a cell by the user to make altered cells, and then the altered cells can be screened for a particular phenotype(s) of interest. For example, to use a phenotype described herein, a user seeking to increase lycopene production and possessing a bacterial strain that produces a certain amount of lycopene could introduce a collection of mutated global transcriptions factor(s) into the bacterial strain, and then screen for improved production of lycopene. Subsequent rounds of directed evolution by mutation and reintroduction of the global transcription machinery also can be carried out to obtain further improvements in lycopene production.

Collections/libraries can be stored in containers that are commonly used in the art, such as tubes, microwell plates, etc.

### Examples

### Materials and Methods

### Strains and Media

*E. coli* DH5α (Invitrogen, Carlsbad, CA) was used for routine transformations as described in the protocol as well as for all phenotype analysis in this experiment. Strains were grown at 37°C with 225 RPM orbital shaking in either LB-Miller medium or M9-minimal medium containing 5 g/L D-glucose and supplemented with 1mM thiamine (Maniatis, et al., Molecular cloning: a laboratory manual, Cold Spring Harbor Laboratory Press, Cold Spring Harbor, NY, 1982). Media was supplemented with 34 µg/ml of chloramphenicol for low copy plasmid propagation and 68 µg/ml of chloramphenicol, 20 µg/ml kanamycin, and 100 µg/ml ampicillin for higher copy plasmid maintenance as necessary. Cell density was monitored spectrophotometrically at 600 nm. M9 Minimal salts were purchased from US Biological (Swampscott, MA), X-gal was purchased from American Bioanalytical (Natick, MA) and all remaining chemicals were from Sigma-Aldrich (St. Louis, MO). Primers were purchased from Invitrogen.

### Library Construction

A low copy host plasmid (pHACM) was constructed using pUC19 (Yanisch-Perron, et al., Gene 33: 103-119, 1985) as a host background strain and replacing ampicillin resistance with chloramphenicol using the CAT gene in pACYC184 (Chang, et al., J Bacteriol 134: 1141-1156, 1978) and the pSC101 origin of replication from pSC101 (Bernardi, et al., Nucleic Acids Res 12: 9415-9426, 1984). The chloramphenicol gene from pACYC 184 was amplified with AatII and AhdI restriction site overhangs using primers CM_sense_AhdI: GTTGCCTGACTCCCCGTCGCCAGGCGTTTAAGGGCACCAATAAC (SEQ ID NO:1) and CM_anti_AatII: CAGAAGCCACTGGAGCACCTCAAAACTGCAGT (SEQ ID NO:2). This fragment was digested along with the pUC19 backbone and ligated together to form pUC19-Cm. The pSC101 fragment from pSC101 was amplified with AflIII and NotI restriction site overhangs using primers pSC_sense_AflIII: CCCACATGTCCTAGACCTAGCTGCAGGTCGAGGA (SEQ ID NO:3) and pSC_anti_NotI: AAGGAAAAAAGCGGCCGCACGGGTAAGCCTGTTGATGA TACCGCTGCCTTACT (SEQ ID NO:4). This fragment was digested along with the pUC19-Cm construct and ligated together to form pHACM.

The *rpoD* gene (EcoGene Accession Number: EG10896; B-number: b3067; SEQ ID NO:27) was amplified from *E. coli* genomic DNA using HindIII and SacI restriction overhangs to target the *lacZ* gene in pHACM to allow for blue/white screening using primers rpoD_sense_SacI: AACCTAGGAGCTCTGATTTAACGGCTTAAGTGCCGAAGAGC (SEQ ID NO:5) and rpoD_anti_HindIII: TGGAAGCTTTAACGCCTGATCCGGCCTACCGATTAAT (SEQ ID NO:6). Fragment mutagenesis was performed using the GenemorphII Random Mutagenesis kit (Stratagene, La Jolla, CA) using various concentrations of initial template to obtain low, medium, and high mutation rates as described in the product protocol. Following PCR, these fragments were purified using a Qiagen PCR cleanup kit (Qiagen, Valencia, CA), digested by HindIII and SacI overnight, ligated overnight into a digested pHACM backbone, and transformed into *E*. *coli* DH5α competent cells. Cells were plated on LB-agar plates and scraped off to create a liquid library. The total library size of white colonies was approximately 10⁵ to 10⁶.

### Phenotype Selection

Samples from the liquid library were placed into challenging environments to select for surviving mutants. For ethanol tolerance, strains were placed in filtered-LB containing 50 g/L of ethanol. These cultures were performed in 30 x 115 mm closed top centrifuge tubes shaking at 37°C. Strains were plated after 20 hours and selected for individual colony testing. For acetate tolerance, strains were serial subcultured twice in increasing concentrations of acetate starting at 20 g/L and increasing to 30 g/L in M9 minimal media. Cells were then plated onto LB plates and several colonies were selected for single-colony assays. For para-hydroxybenzoic acid (pHBA) tolerance, strains were cultured in 20 g/L of pHBA in M9 minimal media and plated after 20 hours to select for surviving cells. The plasmids from all strains identified with improved phenotypes were recovered and retransformed into a fresh batch of competent cells. Several colonies were selected from each plate to perform biological replicates to verify phenotypes.

### Sequence Analysis

Sequences of mutant sigma factors were sequenced using the following set of primers:
S1: CCATATGCGGTGTGAAATACCGC (SEQ ID NO:7),
S2: CACAGCTGAAACTTCTTGTCACCC (SEQ ID NO:8),
S3: TTGTTGACCCGAACGCAGAAGA (SEQ ID NO:9),
S4: AGAAACCGGCCTGACCATCG (SEQ ID NO:10),
A1: GCTTCGATCTGACGGATACGTTCG (SEQ ID NO:11),
A2: CAGGTTGCGTAGGTGGAGAACTTG (SEQ ID NO:12),
A3: GTGACTGCGACCTTTCGCTTTG (SEQ ID NO:13),
A4: CATCAGATCATCGGCATCCG (SEQ ID NO:14),
A5: GCTTCGGCAGCATCTTCGT (SEQ ID NO:15), and
A6: CGGAAGCGATCACCTATCTGC (SEQ ID NO:16).

Sequences were aligned and compared using Clustal W version 1.82.

### Example 1:

The main sigma factor, σ⁷⁰, was subjected to directed evolution in *E. coli* in search for increased tolerance phenotypes. This main sigma factor was chosen on the premise that mutations will alter promoter preferences and transcription rates and thus modulate the transcriptome at a global level. The *rpoD* gene and native promoter region were subjected to error-prone PCR and cloned into a low-copy expression vector (Fig. 1). A nearly 10⁵ to 10⁶ viable-mutant library was initially constructed and transformed into strains.

This library was subjected to selection by culturing in the extreme conditions of high ethanol, high acetate and high para-hydroxybenzoic acid (pHBA) concentrations. These conditions were selected because of their industrial relevance: Acetate is an *E. coli* byproduct that is inhibitory to cell growth while prospects for bioethanol production can be enhanced by engineering a strain with increased tolerance to ethanol, thus increasing possible yields (L. O. Ingram et al., Biotechnol Bioeng 58, 204-14 (Apr 5, 1998)). Furthermore, there is considerable industrial interest in the production of pHBA as a precursor for electronic coatings, which is, however, extremely toxic to cells (T. K. Van Dyk, L. J. Templeton, K. A. Cantera, P. L. Sharpe, F. S. Sariaslani, J Bacteriol 186, 7196-204 (Nov, 2004); J. L. Barker, J. W. Frost, Biotechnol Bioeng 76, 376-90 (Dec, 2001)). Each of these tolerance phenotypes has been investigated by traditional methods of randomized cellular mutagenesis, gene complementation and knockout searches, and microarray analysis (R. T. Gill, S. Wildt, Y. T. Yang, S. Ziesman, G. Stephanopoulos, Proc Natl Acad Sci U S A 99, 7033-8 (May 14, 2002)), with limited success to-date.

### Ethanol tolerance

Mutants of the sigma factor library were first selected on the basis of ability to grow in the presence of high concentrations of ethanol in LB complex medium (L. P. Yomano, S. W. York, L. O. Ingram, J Ind Microbiol Biotechnol 20, 132-8 (Feb, 1998)). For this selection, strains were serially subcultured twice at 50 g/L of ethanol overnight, then plated to select for tolerant mutants. A total of 20 colonies were selected and assayed for growth in varying ethanol concentrations. After isolation and validation of improved strains, the best mutant sigma factor was subjected to sequential rounds of evolution. With both subsequent iterations, the selection concentration was increased to 70 and 80 g/L of ethanol. In these enrichment experiments, cells were plated after 4 and 8 hours of incubation due to the strong selection pressure used. Isolated mutants from each round show improved overall growth in various ethanol concentrations (Fig. 2A).

Fig. 2B identifies the sequences of the best mutants isolated from each round of mutagenesis. Sequence alignments of ethanol tolerant sigma factors are provided in Fig. 2D. Interestingly, the second round mutation led to the formation of a truncated factor which is apparently instrumental in increasing overall ethanol fitness. This truncation arose from an artifact in the restriction enzyme digestion and includes part of region 3 and the complete region 4 of the protein. Region 4 is responsible for binding to the promoter region and a truncated form has been previously shown to increase binding affinity relatively to that of the full protein (U. K. Sharma, S. Ravishankar, R. K. Shandil, P. V. K. Praveen, T. S. Balganesh, J. Bacteriol. 181, 5855-5859 (1999)). It is therefore possible that this truncated mutant serves to act as a potent and specific inhibitor of transcription by binding to preferred promoter regions and preventing transcription since the remainder of the sigma factor machinery is removed. In the truncated form of the round 2 mutant, the I511 V mutation of the first round was reverted back to an isoleucine, leaving only one mutation.

This truncated form was subjected to a third round of mutagenesis and selection to yield a factor with 8 additional mutations. In this final round, the R603C mutation found in the prior two rounds was reverted back to the original residue and many new mutations appeared, leaving only the truncation as the only visible similarity between round 2 and round 3. These rounds of mutagenesis and resulting sequences suggest a difference compared with protein directed evolution. In the latter case, mutations which increase protein function are typically additive in nature. On the other hand, the mutations incurred in altering transcription machinery are not necessarily additive as these factors act as conduits to the transcriptome. In this regard, many local maxima may occur in the sequence space due to the various subsets of gene alterations which may lead to an improved phenotype.

All isolated strains harboring the mutant sigma factors exhibited increased growth rates relative to the control at elevated ethanol concentrations. Furthermore, the growth phenotype of the mutant strains in the absence of ethanol was not impacted (Table 1).

**Table 1: Directed evolution of ethanol tolerant sigma factors.**

| | Doubling Time (h) | Ratio of doubling times (t_{d,control} / t_{d,engineered mutant}) | | |
|---|---|---|---|---|
| Ethanol Concentration (g/L) | Control | Round 1 | Round 2 | Round 3 |
| 0 | 0.76 | 1.01 | 0.98 | 0.98 |
| 20 | 1.31 | 1.68 | 1.63 | 1.63 |
| 40 | 2.41 | 1.64 | 1.30 | 1.54 |
| 50 | 7.24 | 1.92 | 1.82 | 2.06 |
| 60 | 69.3 | 4.53 | 11.70 | 11.18 |
| 70 | 192.3 | 1.40 | 11.56 | 12.43 |
| 80 | ND | ND | 28.64 hours | 29.80 hours |
| | | | | |
| Maximum sustainable concentration (g/L) | 40 | 50 | 60 | 70 |

Improvements in the fold reduction of doubling time are presented for increasing concentrations of ethanol for the three rounds of directed evolution. The mutants in Rounds 2 and 3 show significant increases in the growth rate at higher concentrations of ethanol. A continual increase in the highest concentrations of sustainable cellular growth is seen throughout the rounds of directed evolution.

The truncated mutant isolated in the second round showed increased growth rates at higher ethanol concentrations; however, its growth rate was reduced at lower ethanol concentrations compared with the first round mutant. The mutant isolated from the third round showed recovered growth rates, similar to that of the first round, between 20 and 50 g/L of ethanol. Most importantly, each subsequent round increased the highest ethanol concentration at which cells were able to sustain growth for longer than 8 hours, without succumbing to the ethanol toxicity with an accompanying decrease in cell density. The drastic increase in ethanol tolerance obtained through this method is illustrated by the growth curves of the round 3 strain shown in (Fig. 2C) along with those of the wild type control. Sigma factor engineering (SFE) was able to increase the ethanol tolerance beyond the levels previously reported in the literature using more traditional methods. Furthermore, the application of iterative rounds of SFE was illustrated to be capable of further improving the cellular phenotype.

### Acetate and pHBA tolerance

As a second example, the original sigma factor mutant library was serial subcultured twice on 20 g/L followed by 30 g/L of acetate in M9-minimal medium. Single colonies were isolated from this mixture, retransformed to preclude any chromosome-based growth adaptation, and assayed for growth in varying acetate concentrations. Isolated strains showed a drastic increase in tolerance in the presence of high levels of acetate. Additionally, the growth rate was, once again, not substantially affected in the absence of acetate (Table 2). At 30 g/L of acetate, isolated strains had doubling times of 10.5 - 12.5 hours, approximately 1/5 of the doubling time of the severely inhibited control (56 hours doubling time).

**Table 2: Application of transcription machinery engineering for additional phenotypes.**

| g | | Ratio of doubling times (t_{d,control} / t_{d,engineered mutant}) | | | | |
|---|---|---|---|---|---|---|
| Acetate concentration (g/L) | Control doubling time (h) | Mutant Ac1 | Mutant Ac2 | Mutant Ac3 | Mutant Ac4 | Mutant Ac5 |
| 0 | 2.11 | 1.00 | 0.98 | 1.10 | 1.03 | 0.97 |
| 10 | 4.99 | 0.88 | 1.02 | 1.05 | 0.99 | 1.08 |
| 20 | 7.23 | 1.32 | 1.16 | 1.17 | 1.17 | 1.28 |
| 30 | 56.35 | 4.67 | 4.98 | 4.45 | 4.99 | 5.32 |
| | | | | | | |

| pHBA Concentration (g/L) | Control (OD at 13 h) | Mutant HBA1 (Ratio) | | | | |
|---|---|---|---|---|---|---|
| 0 | 1.14 | 0.97 | | | | |
| 5 | 0.56 | 1.17 | | | | |
| 10 | 0.35 | 1.21 | | | | |
| 15 | 0.097 | 1.55 | | | | |
| 20 | ND | ND | | | | |

Mutants were isolated which showed an increased tolerance in either elevated acetate levels or in the presence of high levels of pHBA. Increases in the tolerance are seen at elevated levels of the chemicals, however, no adverse effects are seen in the growth rates or yields in the absence of these chemicals.

Fig. 3A summarizes the various mutations classified by region in the isolated sigma factors eliciting an increased cellular tolerance for acetate. Sequence alignments of acetate tolerant sigma factors are provided in Fig. 3B. Only one of the five isolated mutants was truncated. The M567V mutation appeared in two of the acetate mutants and most of the mutations appear to be distributed among the functional domains of the sigma factor. It is interesting to note that even though strains have similar tolerance profiles, the underlying mutations are different suggesting different molecular mechanisms influencing the transcription profiles.

As a another example, the mutant library was cultured in the presence of 20 g/L of pHBA to select for strains with increased tolerance to this compound in terms of growth and viability at high pHBA concentrations. One strain was isolated with marked improvement in the growth yield at 13 hours compared with the control and essentially unchanged growth phenotype in the absence of pHBA (Table 2). Mutant HBA1 showed a truncated form of the sigma factor with a total of six mutations (Fig. 3A), with 4 of 6 residues being changed to a valine. Sequence alignments of pHBA tolerant sigma factors are provided in Fig. 3C.

These examples illustrate the potential of sigma factor engineering to introduce global transcriptome changes that allow the organism to access novel cellular phenotypes. Recently, we have successfully extended the concept of global transcription machinery engineering beyond tolerance phenotypes to select for mutants which increase metabolite overproduction rates (see below). Furthermore, this concept has been explored with other host systems including eukaryotic transcription machinery components. In each of these examples, the global changes brought about by random mutations in the components of transcriptional regulatory machinery is shown to improve to cellular phenotypes beyond levels attainable through rational engineering or traditional strain improvement by random mutagenesis.

For the first time, we demonstrated the application of directed evolution to alter the global transcription machinery. This strategy allowed for the directed modification of the genetic control of multiple genes simultaneously, as opposed to typical consecutive, gene-by-gene strategies. Furthermore, we found the paradigm of directed evolution to be applicable as it allowed sequential phenotypic improvements by probing deeper into the vast sequence space of transcription factor engineering. As a result, it is now possible to unlock complex phenotypes regulated by multiple genes which would be very unlikely to reach by the relatively inefficient iterative search strategies.

It is worth noting that the described method can also be applied in reverse to uncover the complicated interactions of the genotype-phenotype landscape. In such applications, one would employ a number of high-throughput cellular and molecular assays to assess the altered cellular state and ultimately deduce systematic mechanisms of action underlying the observed phenotype in these mutants. The application of directed evolution to global transcription machinery as described here is a paradigm shifting method for identifying genetic targets, eliciting desired phenotypes and realizing the goal of whole cell engineering.

### Example 2:

### Organic Solvent Tolerance

The application of global transcription machinery engineering has been extended to include additional tolerance phenotypes. Bacterial strain tolerance to organic solvents is useful in several situations: (1) bioremediation of hazardous waste, (2) bioproduction of organic solvents from bacteria, and (3) bioprocessing applications requiring a two-phase reactor (i.e. extractive fermentations to continuously remove hydrophobic products operation). To investigate the potential to increase solvent tolerance in *E*. *coli,* the original rpoD (σ⁷⁰) mutant library was cultured and harvested in exponential phase and transferred to a two-phase system containing LB medium and hexanes (10% v/v). Strains were isolated after 18 hours of growth in the presence of hexane. These individual colonies were again cultured to exponential phase and then cultured in the presence of hexane. Cell densities are measured after 17 hours. Cell densities from culture with hexane are shown in Fig. 4. The strains shown in Fig. 4 are re-transformed strains performed in biological replicates. All selected strains had an increase in cell density over the control strain containing an unmutated version of the rpoD gene. Furthermore, PCR analysis indicated that mutant strains Hex-3, Hex-8, Hex-11, Hex-12, Hex-13, Hex-17 and Hex-19 have a whole version of the sigma factor while strains Hex-2, Hex-6, Hex-9, Hex-10, and Hex-18 have a truncated version. Fig. 4 also shows the sequence (location of mutations) for the two best-performing mutants, Hex-12 and Hex-18.

Additionally, these strains were tested for growth in the presence of cyclohexane, which is known to be a more toxic organic solvent to microorganisms than hexane. Fig. 5 shows the cell densities from cultures with cyclohexane. Several of the strains isolated from the hexane selection also showed and increase in cell density over the control.

### Example 3:

### Antibiotic Resistance

The application of global transcription machinery engineering has been extended to include antibiotic resistance. Antibiotic resistance among microorganisms is becoming a significant problem placing a stress on health care and pharmaceutical companies to find alternatives ways to fight infections. Many resistant strains are known to contain specific genes encoding for a resistance. However, before microorganisms are able to evolve such a gene, they must first gain an initial resistance in an effort to persist in the presence of antibiotics. While incurring random mutations in the genome is one alternative, cells can also change their gene expression in response to these antibiotics. The use of global transcription machinery engineering was tested to identify the possibility of creating antibiotic resistant strains. This phenotype would ultimately be controlled by the altered expression of the transcriptome, mediated through the mutant transcription machinery. An analysis of the gene expression of these strains could lead to the identification of novel gene targets and enzymes which control the resistance of the strain. These targets could then lead to the development of small molecule drugs which inhibit or enhance the activity of the identified enzymes. The topic of antibiotic resistance was tested by culturing the mutant sigma factor library in the presence of 250 µg/ml of nalidixic acid, a quinolone (the same family of drugs as Ciprofloxacin), which is in excess of the minimum inhibitory concentration of the control of around 80 µg/ml. Fig. 6 presents the cell density (OD600) for various isolated strains at increasing concentrations of nalidixic acid. Several isolated strains showed significant growth in the presence of high concentrations of nalidixic acid. These strains are tested for verification after transformation of the plasmids into fresh host strains. Furthermore, these mutants are sequenced; PCR analysis indicated that mutant strains NdA-7 and NdA-15 are whole length sigma factors while NdA-10, NdA-11, NdA-12 and NdA-13 are truncated versions.

### Example 4:

### Metabolite overproduction phenotypes

The basic tenet of global transcription machinery engineering is the ability to create multiple and simultaneous gene expression modifications. Previously, this method was successfully employed for the identification of mutants with increased tolerance phenotypes. In these subsequent examples, a mutant library of the principal sigma factor, encoded by *rpoD,* was examined for its capacity to enhance metabolite overproduction phenotypes beyond those levels achievable by single genetic modifications.

### Lycopene Production

Previously, we have identified a number of single and multiple gene knockout targets which showed an increase of lycopene production in the background of a pre-engineereed strain (Alper et al., Nat Biotechnol 2005 and Alper et al., Metab Eng 2005). In this study, we sought to utilize the technique of global transcription machinery engineering to enhance lycopene production. Utilizing several available strain backgrounds which were previously engineered along with the parental strain, it was possible search for mutant factors, independently in each background, which resulted in an increased lycopene production. For this study, the parental strain, Δ*hnr,* and the two identified global maximum strains, Δ*gdhA*Δ*aceE*Δ*fdhF,* and Δ*gdhA*Δ*aceE*Δ*pyjiD,* were selected. The best mutant from each of the four tested genetic backgrounds was then swapped to investigate the landscape created by mixing 4 strains with the 4 identified mutant sigma factors.

### Identification of mutant sigma factors

The mutant sigma factor library was transformed into each of the four strains and selected based on lycopene production on minimal medium plates supplemented with 5 g/L of glucose. Selected strains were then cultured and assayed for lycopene production at 15 and 24 hours using M9 medium. Figs. 7A - 7D illustrate the results of these searches along with the sequence of sigma factor mutant from the best strain. Lycopene production is indicated for the strain with and without the control plasmid. For some backgrounds, this control plasmid resulted in a large decrease in lycopene production over the strain absent of this plasmid. It is interesting to note that all of these identified factors have been truncated. Furthermore, the mutant identified from the *hnr* knockout background was simply truncated and contained no mutations. Given the suspected mode of action for this truncation, it is possible that this mutant factor essentially suppresses all of the normal genes expressed under the control of *rpoD.* In an *hnr* mutant, a higher steady state level of the stationary phase sigma factor, σ^{s}, is available to take over the remainder of transcription. Furthermore, the second highest mutant in this background resulted in a full length sigma factor containing several mutations.

### Combinations of strains and identified mutant factors

The four strains with varying genetic backgrounds were then combined with the four independently identified mutant sigma factors to examine the resulting 16 strain landscape. It is interesting to initially note that none of the identified mutants in Figs. 7A - 7D which were sequenced for a given genetic background overlapped with those identified in another genetic background. As a result, it is initially suspected that the landscape would be diagonally dominant, indicating that the effect elicited by the mutant factor is specific to the genetic background. These 16 strains along with the controls were cultured in a 2x M9 medium with staged glucose feed. The lycopene level was assayed at 15, 24, 39, and 48 hour timepoints. Fig. 8 presents a dot plot which depicts the maximum fold increase in lycopene production achieved over the control during the fermentation. The size of the circle is proportional to the fold increase. As suspected, the landscape is clearly diagonally-dominant with mutant factors predominantly working in the strain background in which they were identified.

Fig. 9 illustrates the lycopene content after 15 hours for several strains of interest. The single round of mutagenesis in both the parental strain and *hnr* knockout was able to achieve similar results as strains previously engineered through the introduction of three distinct gene knockouts. However, in these backgrounds, lycopene levels were able to be further increased through the introduction of an additional mutant sigma factor.

These results indicate that (1) global transcription machinery engineering (gTME) is able to elicit metabolic phenotypes and, more importantly, (2) a single round of selection using gTME is more effective than a single knockout or overexpression modification. Furthermore, the identified mutant is not generally transferable across strain backgrounds, which suggests that there may be different modes of lycopene production in each of the strains. As an example of these modes, the maximum fold difference in the wild type strain was realized after only 15 hours and then converged with the control strain by the end of the fermentation. Conversely, the mutant factor in the Δ*gdhA*Δ*aceE*Δ*pyjiD* strain progressively increased in lycopene content compared with the control for increasing timepoints. Nevertheless, the highest lycopene production resulted in using gTME in the background of a previously engineered strain indicating that, given only one round of selection, it is better to start in an optimized strain. However, the results of ethanol tolerance suggest that it is possible to achieve continual improvements in fitness through the application of directed evolution, indicating that it may be possible to increase lycopene production further.

### Bioproduction of Polyhydroxybutyrate (PHB)

The application of global transcription machinery engineering has been extended to include a further example of metabolite overproduction. An additional metabolic phenotype (in addition to production of lycopene), bioproduction of polyhydroxybutyrate (PHB), was investigated using transcription machinery engineering. PHB is produced from the precursor molecule of acetyl-coA.

### Materials/Methods

*Escherichia coli* (XL-1 Blue, Stratagene, La Jolla, Calif.) transformed with a modified pJOE7 (Lawrence, A. G., J. Choi, C. Rha, J. Stubbe, and A. J. Sinskey. 2005. Biomacromolecules 6:2113-2119) plasmid was cultured at 37°C in Luria-Bertani (LB) medium containing 20 g/L glucose and 25 is µg/mL kanamycin. The modified pJOE7 was graciously given to us by Dr. Anthony Sinskey (MIT, Cambridge, MA) and contains *phaAB* from *C*. *necator* and the *phEC* from *Allochromatium vinosum* and encodes kanamycin resistance. As a no PHB control, the same plasmid without the *pha* genes was also cultured. Optical density was used to track cell growth using an Ultraspec 2100 pro (Amersham Biosciences, Uppsala, Sweden).

### Staining and Flow Cytometry

A nile red (Sigma-Aldrich, St. Louis, MO) stock solution was made by dissolving to 1 mg/mL in dimethyl sulfoxide unless otherwise noted. 3 µL of stock solution was added to 1 mL of staining buffer as indicated in the staining optimization. Flow cytometry was carried out on a FACScan (Becton Dickinson, Mountain View, CA) using the following settings; *Synechocystis* FSC=E00, SSC=411, FL-1=582, FL-2=551 and *E. coli* FSC=E00, SSC=411, FL-1=582, FL-2=535. Cells were excited with an air-cooled argon ion laser (488 nm), and FL-2 (585nm) was used to detect nile red fluorescence. Flow cytometry analysis was done on 50,000 cells using WinMDI 2.8.

Staining effectiveness was characterized by resolution, *R_{S}* (Eq. 1), where Mₙ is the geometric mean of the fluorescence distribution of n (n=1 is the PHB producing cell, n=2 is the no PHB control). δₙ is the standard deviation of the fluorescence distribution. *R_{S}* is a quantitative measure of the ability to differentiate two populations. ${R}_{S} = \frac{2 ⁢ \left({M}_{1}-{M}_{2}\right)}{{δ}_{1} + {δ}_{2}}$

Cell viability was accessed by ratio of the cfu in the final stained preparation to cells from the media.

### Chemical PHB Analysis

PHB was analyzed as shown previously (Taroncher-Oldenburg, G., and G. Stephanopoulos. 2000. Applied Microbiology and Biotechnology 54:677-680). >10 mg of cells was collected from culture by centrifugation (10 min, 3,200×g). The resulting pellet was washed once with cold deionized H₂O and dried overnight at 80°C. The dry pellets were boiled in 1 ml of concentrated H₂SO₄ for 60 min, diluted with 4 ml of 0.014 M H₂SO₄. Samples were centrifuged (15 min, 18,000×g) to remove cell debris, and liquid was analyzed by HPLC using an Aminex HPX-87H ion-exclusion column (300 x 7.8 mm; Bio-Rad, Hercules, Calif.) (Karr, D. B., J. K. Waters, and D. W. Emerich. 1983. Applied and Environmental Microbiology 46:1339-1344). Commercially available PHB (Sigma-Aldrich, St. Louis, Mo.), processed in parallel with the samples, was used as standards.

### E. coli Staining Optimization

*E. coli* XL1-blue harboring the modified pJOE and the no PHB control were cultured as described.

*Shock optimization*: Cultures were grown to stationary phase. A variety of different permeabilization methods were tested for resolution and viability after the shock. Sucrose shock was carried out as shown previously (Vazquez-Laslop, N., H. Lee, R. Hu, and A. A. Neyfakh. 2001. J. Bacteriol. 183:2399-2404). 1 mL of cells was cooled to 4°C for 10 min. The cells were then centrifuged (3 min, 3000×g, 4°C) and resuspended in 1 mL ice-cold TSE buffer (10 mM Tris-Cl [pH=7.5], 20% sucrose, 2.5 mM Na-EDTA). The cells were incubated on ice for 10 min then resuspended (3 min, 3000×g, 4°C) in 1 mL deionized water with 3µL nile red stock solution. Cells were stained in the dark for 30 min and analyzed on the FACScan. Isopropanol shocked cells were centrifuged (3 min, 3000×g) and resuspended in 70% isopropanol for 15 min. Cells were then centrifuged (3 min, 3000×g) and resuspended in deionized water with 3µL nile red stock solution. Cells were incubated for 30 min in the dark and analyzed on the FACScan. DMSO shock was performed by centrifuging (3 min, 3000×g) 1mL of cell culture. 50 µL of nile red stock solution was added directly to the pellet. The pellet was quickly vortexed and diluted to 1 mL in water after incubating for 30 s. Cells were incubated for 30 min in dark and analyzed on the FACScan. Heat shock was performed as in competent cell preparation (Sambrook, J., E. F. Fritsch, and T. Maniatis. 1989. Molecular Cloning: A Laboratory Manual, 2nd ed. Cold Spring Harbor Laboratory Press). 1 mL of cells was cooled for 10 min. Cells were then centrifuged (3 min, 3000×g, 4°C), and resuspended in 1 mL cold 80 mM MgCl₂ / 20 mM CaCl₂. Cells were centrifuged (3 min, 3000×g, 4°C) and resuspended in 1 mL 0.1 M CaCl₂ with 3µL nile red stock solution. Cells were heat shocked at 42°C for 90 s. Cells were incubated for 30 min in dark then analyzed on the FACScan.

*Concentration optimization*: Cells were prepared by sucrose shock using 3µL of different nile red solutions to a final concentration between 30 - 30,000 ng/mL.

*Sucrose concentration optimization*: Cells were prepared by sucrose shock using TSE buffer with varying sucrose concentrations (0, 5, 10, 15, 20%).

The mutant sigma factor library was introduced into *Escherichia coli* as described above. Strains were selected for increased exponential phase PHB in a glucose-minimal media. Additionally, a random knockout library created using transposon mutagenesis was also tested to compare the efficacy of transcription machinery engineering to that of traditional strain improvement methods. Fig. 10A presents the data for various strains (bars in red and yellow represent controls) obtained using sigma factor engineering. In comparison, Fig. 10B presents the results of selected strains from a random knockout library. Several mutants obtained using sigma factor engineering produced nearly 25% dcw (dry cell weight) of PHB. The best strain obtained in one round of sigma factor engineering was far superior to the best strain obtained using random knockouts. A second round of mutagenesis in the background of the best mutant is carried out as described above for further improvement of the PHB phenotype.

### Example 5:

### Library diversity and construction

The size and breadth of the sigma factor library is increased in one or more of the following ways.
(1) The library includes not only the main sigma factor of *E. coli* (σ⁷⁰, encoded by *rpoD*), but also one or more alternative forms, e.g., *rpoS, rpoF, rpoH, rpoN, rpoE* and/or *fecI.* It may be possible to further improve phenotypes and search for optimized strains through the simultaneous introduction of multiple mutant versions of transcription machinery units. The mutated sigma factor genes (or other global transcription machinery) are expressed, for example, using expression cassettes which coexpress two or more of these genes. The two or more genes may be two or more of the same type of transcription machinery (e.g., two versions of an *rpoD*) or may be two or more distinct transcription machinery (e.g., *rpoD* and *rpoS*).
   Likewise, more than one different mutant versions of global transcription machinery may be beneficial to properly optimize for a phenotype. For example, multiple mutated sigma 70 (rpoD) genes can be coexpressed.
(2) In addition to random mutations introduced by error prone PCR as described above, the library includes all possible truncations from both the C terminus and N terminus and combinations thereof.
(3) Furthermore, the library includes alternative chimeras of various regions of the sigma factors by artificially fusing the regions. For example, Region 1 of sigma factor 70 is used to replace Region 1 of sigma factor 38. A similar approach by using DNA shuffling to create diversity is well known in the art (e.g., gene shuffling patents of W. Stemmer et al., assigned to Maxygen; see listing at maxygen.com/science-patents).
(4) Sigma factors from other bacteria are included in the library in the same configurations (e.g., random mutations, truncations, chimeras, shuffling) as described for *E*. *coli* sigma factor 70 above. These factors may possess unique properties of DNA binding and may help to create a diversity of transcriptome changes.

### Example 6:

### Global transcription machinery engineering in eukaryotic cells

The directed evolution of global transcription machinery is applied to yeast and mammalian systems (e.g., CHO, HeLa, Hek cell lines) for enhanced recombinant protein production and resistance to apoptosis in inducing conditions.

A gene encoding global transcription machinery (e.g., TFIID) is subjected to error prone PCR, truncation and/or DNA shuffling in order to create a diverse library of global transcription machinery mutants. The library is introduced into the yeast or mammalian cells and, in a first experiment, the production of recombinant protein by the cells is examined. A readily assayable protein is preferred for these experiments, such as SEAP or a fluorescent protein (e.g., GFP). In the case of fluorescent proteins, cells can be selected using a fluorescence activated cell sorter or if grown in multiwell plates, a fluorescence plate reader can be used to determine the enhancement in protein production.

In a second experiment, anti-apoptosis phenotypes are examined in the yeast or mammalian cells.

### Example 7:

### SDS tolerance

The directed evolution of global transcription machinery was applied to the problem of cellular tolerance to sodium dodecyl sulfate (SDS).

The mutant *rpoD* library was transformed into *Escherichia coli* DH5α, which were then subcultured in LB medium containing increasing amounts of SDS (5%, then 15 % SDS, by mass). Strains were selected for increased tolerance in SDS. Strain SDS-2 was selected and retransformed to verify the phenotype. Strain SDS-2 was then tested at 5-20% SDS (by mass). This mutant was found to have increased growth at elevated SDS levels, without any detrimental effects to the growth in the absence of SDS. Fig. 11 shows the cell densities of cultures of isolated strains of SDS-tolerant sigma factor mutants at increasing concentrations of SDS, along with the sequence of the sigma factor mutant from the best strain.

### Example 8:

### Engineering multiple phenotypes

Global transcription machinery engineering was applied to the problem of imparting a multiple tolerance phenotype in *E. coli.* In order to obtain the tolerance to both ethanol and SDS, in a first set of experiments, strains were isolated following three alternative strategies: (i) mutants were isolated after treatment/selection in both ethanol and SDS, (ii) mutants were isolated which were tolerant to ethanol first, then subjected to an additional round of mutagenesis and selected using an ethanol/SDS mixture, and (iii) mutants were isolated which were tolerant to SDS first, then subjected to an additional round of mutagenesis and selected using an ethanol/SDS mixture. These strains were tested for growth in the presence of various concentrations of ethanol and SDS to obtain growth curves and to assess the effectiveness of these strategies. The experiments were conducted using the protocols described in other examples above.

In a second set of experiments, a mutant sigma factor is isolated from an ethanol tolerant strain and is co-expressed with a mutant sigma factor that is isolated from an SDS tolerant strain. These experiments are conducted using the protocols described in other examples above.

### Example 9:

### Extension of global transcription machinery engineering (gTME) to yeast systems

In any type of cellular system, a subset of proteins is responsible for coordinating global gene expression. As such, these proteins provide access points for diverse transciptome modifications broadly impacting phenotypes of higher organisms. This example demonstrates the application of gTME to the eukaryotic model system of yeast (*Saccharomyces cerevisiae*). In stark contrast to the transcriptional machinery of the prokaryotic system, eukaryotic transcription machinery is more complex in terms of the number of components and factors associated with regulating promoter specificity. First, there are three RNA polymerase enzymes with separate functions in eukaryotic systems while only one exists in prokaryotes. Furthermore, an example of this complexity is exemplified by nearly 75 components classified as a general transcription factor or coactivator of the RNA Pol II system (Hahn, Nat Struct Mol Biol, 11(5), 394-403, 2004). Components of the general factor TFIID include the TATA binding protein (*Spt15*) and 14 other associated factors (TAFs) and are thought to be the main DNA binding proteins regulating promoter specificity (Hahn, 2004). Moreover, TATA-binding protein mutants have been shown to change the preference of the three polymerases, suggesting a pivotal role for orchestrating the overall transcription in yeast (Schultz, Reeder, & Hahn, Cell, 69(4), 697-702, 1992). The focus of this study will be on two major proteins of transcription: the TATA-binding protein (*Spt15*) and a TAF (*TAF25*).

Crystal structures are available for the TATA-binding protein and clearly illustrate portions of the protein for direct DNA binding and other portions for protein binding with the TAFs and parts of the polymerase (Bewley, Gronenborn, & Clore, Annu Rev Biophys Biomol Struct, 27, 105-131, 1998; Chasman et al., Proc Natl Acad Sci U S A, 90(17), 8174-8178, 1993; J. L. Kim, Nikolov, & Burley, Nature, 365(6446), 520-527, 1993). This structure consists of two repeat regions which interact with the DNA and two helices which interact with proteins. Assays and mutational analysis suggest that the TATA-binding protein plays an important role in promoter specificity and global transcription. Furthermore, important residues have been suggested for DNA contact points and protein interaction points (Arndt et al., Mol Cell Biol, 12(5), 2372-2382, 1992; J. Kim & Iyer, Mol Cell Biol, 24(18), 8104-8112, 2004; Kou et al., Mol Cell Biol, 23(9), 3186-3201, 2003; Schultz, Reeder, & Hahn, 1992; Spencer & Arndt, Mol Cell Biol, 22(24), 8744-8755, 2002). The TAFs have received varying amounts of attention. The TAF25 protein, the subject of this study, has been analyzed using sequence alignment and through mutation analysis and has been shown to impact transcription of many genes (Kirchner et al., Mol Cell Biol, 21(19), 6668-6680, 2001). This protein is seen to have a series of helices and linkers which are critical to protein interactions. These proteins were investigated using the method of gTME to elicit three phenotypes of interest: (1) LiCl tolerance to model osmotic stress, (2) high glucose tolerance, and (3) the simultaneous tolerance to high ethanol and high glucose.

### Methods

*S. cerevisiae* strain BY4741 (*MAT*a; *his3*Δ*1*; *leu2*Δ*0*; *met15*Δ*0*; *ura3*Δ*0*) used in this study was obtained from EUROSCARF, Frankfurt, Germany. It was cultivated in YPD medium (10 g of yeast extract/liter, 20 g of Bacto Peptone/liter and 20 g glucose/liter). For yeast transformation, the Frozen-EZ Yeast Transformation II (ZYMO RESEARCH) was used. To select and grow yeast transformants bearing plasmids with *URA3* as selectable marker, a yeast synthetic complete (YSC) medium was used containing 6.7 g of Yeast Nitrogen Base (Difco)/liter, 20 g glucose/liter and a mixture of appropriate nucleotides and amino acids (CSM-URA, Qbiogene) referred here as to YSC Ura⁻. Medium was supplemented with 1.5% agar for solid media.

The library was created and cloned behind the TEF-mut2 promoter created previously as part of a yeast promoter library (Alper et al., Proc Natl Acad Sci U S A, 102(36), 12678-12683, 2005). The Taf25 gene was cloned from genomic DNA using the primers TAF25_Sense: TCGAGTGCTAGCAAAATGGATTTTGAGGAAGATTACGAT (SEQ ID NO:28) and TAF25_Anti: CTAGCGGTCGACCTAACGATAAAAGTCTGGGCGACCT (SEQ ID NO:29). The Spot15 gene was cloned from genomic DNA using the primers SPT15_Sense: TCGAGTGCTAGCAAAATGGCCGATGAGGAACGTTTAAAGG (SEQ ID NO:30) and SPT15_Anti: CTAGCGGTCGACTCACATTTTTCTAAATTCACTTAGCACA (SEQ ID NO:31). Genes were mutated using the GeneMorph II Mutagenesis Kit and products were digested using NheI and Sall and ligated to plasmid backbone digested with Xbal and SalI. The plasmids were transformed into *E. coli* DH5α, isolated using a plasmid MiniPrep Spin Kit and transformed into yeast. Plasmids were sequenced using the primers: Seq_Forward: TCACTCAGTAGAACGGGAGC (SEQ ID NO:32)and Seq_Reverse: AATAGGGACCTAGACTTCAG (SEQ ID NO:33).

Strains were isolated by serial subculturing in 200 to 400 mM LiCl, 200 to 300 g/L of glucose, and 5% Ethanol/100 g/L glucose to 6% Ethanol/120 g/L glucose as appropriate. Cells were isolated by plating onto selective medium plates and assayed for performance. Plasmids were isolated and retransformed to revalidate phenotypes in biological replicates.

### LiCl tolerance

Osmotic stress response and tolerance is a complex, pleiotropic response in cells. For yeast, it has been shown that elevated LiCl concentration can induce osmotic stress at concentrations around 100 mM (Haro, Garciadeblas, & Rodriguez-Navarro, FEBS Lett, 291(2), 189-191, 1991; Lee, Van Montagu, & Verbruggen, Proc Natl Acad Sci U S A, 96(10), 5873-5877, 1999; Park et al., Nat Biotechnol, 21(10), 1208-1214, 2003). Yeast cell libraries carrying the mutant versions of either the TBP or TAF25 were serially subcultured in the presence of 200 to 400 mM LiCl. Strains were isolated and retransformed to revalidate the phenotype was a result of the mutant factor. Interestingly, the best strains from each library showed varying improvements to LiCl. The TAF25 outperformed the respective TAF25 unmutated control at lower LiCl concentrations, but was not effective at concentrations above around 200 mM. Conversely, the SPT15 mutant was able to outperform the control at elevated levels of 150 to 400 mM. In each case, the growth phenotype in the absence of LiCl was not impacted by the presence of the mutant factor. A summary of the improvement in growth yield is provided in Figure 12. A sequence analysis (Figure 13) indicates that the improvement in LiCl tolerance was controlled by a single mutation in each of the proteins, with the SPT15 mutation occurring in the unconserved region.

### High glucose tolerance

High glucose fermentations have been explored for increased the ethanol produced from a batch culture of yeast. However, these "very high gravity fermentations" are often quite inhibitory to cell growth and typically are treated by altering the medium composition, rather than altering the cells (Bafrncová et al., Biotechnology Letters, 21(4), 337 - 341, 1999; Bai et al., J Biotechnol, 110(3), 287-293, 2004; Thatipamala, Rohani, & Hill, Biotechnology and Bioengineering, 40(2), 289-297, 1992). To explore this problem using gTME, yeast cell libraries carrying the mutant versions of either the TBP or TAF25 were serially subcultured in the presence of 200 to 400 g/L of glucose. Strains were isolated and retransformed to revalidate the phenotype was a result of the mutant factor. Strains showed a 2 to 2.5 fold increase in cell density after 16 hours of culturing. Unlike the case with LiCl, both the TAF25 and SPT15 proteins showed a similar response to elevated glucose with the maximum improvement over the control occurring between 150 and 250 g/L. However, the SPT15 mutant showed a larger improvement over the TAF25. Figure 14 presents the growth improvement of these mutants and the sequences are presented in Figure 15. In this case, both proteins had only a single mutation, however several suboptimal mutants were isolated for the SPT15 protein, some of which having as many as seven mutations. Both mutations shown here are located in known protein contact areas, especially the I143 residue in the TAF25 protein (Schultz, Reeder, & Hahn, 1992).

### Ethanol and glucose multiple tolerance

Successful fermentations of bioethanol for yeast require tolerance to both high glucose and ethanol concentrations. To this end, the multiple tolerance phenotype was tested through the simultaneous treatment of both mutant libraries to elevated levels of ethanol and glucose (5% and 100 g/L). Isolated strains were retransformed and assayed under a range of glucose concentrations in the presence of 5 and 6% ethanol. Interestingly, the SPT15 mutants outperformed the control at all concentrations tested, upwards of 13 fold improvement in some concentrations. This improvement far exceeded the overall improvement of the TAF25 mutant which was not able to grow in the presence of 6% ethanol. Figure 16 highlights the growth analysis of these best strains and sequences are provided in Figure 17. The improvements achieved through the introduction of the mutant transcription machinery far exceed the improvements obtained through other means of improving cellular phenotype. Furthermore, these results advance the potential to use yeast as a viable source of high ethanol production using a high gravity fermentation.

### Example 10:

Global transcription machinery engineering (gTME) is an approach for reprogramming gene transcription to elicit cellular phenotypes important for technological applications. Here we show the application of gTME to *Saccharomyces cerevisiae,* for improved glucose/ethanol tolerance, a key trait for many biofuels programs. Mutagenesis of the transcription factor Spt15p and selection led to dominant mutations that conferred increased tolerance and more efficient glucose conversion to ethanol. The desired phenotype results from the combined effect of three separate mutations in the *SPT15* gene (F177S, Y195H, K218R). Thus, gTME can provide a route to complex phenotypes that are not readily accessible by traditional methods.

The production of desirable compounds from microbes can often require a complete reprogramming of their innate metabolism. The evolution of such complex traits requires simultaneous modification in the expression levels of many genes, which may be inaccessible by sequential multi-gene modifications. Furthermore, the identification of genes requiring perturbation may be largely unanticipated by conventional pathway analysis. The cellular engineering approach termed "global transcription machinery engineering" (gTME) engineers (via error-prone PCR mutations) key proteins regulating the global transcriptome and generates, through them, a new type of diversity at the transcriptional level.

This approach has already been demonstrated by engineering sigma factors in prokaryotic cells (*1*), but the increased complexity of eukaryotic transcription machinery raises the question of whether gTME can be used to improve traits in more complex organisms. For example, eukaryotic systems have more specialization--- three RNA polymerase enzymes with separate functions, whereas only one exists in prokaryotes. Moreover, there are nearly 75 components have been classified as a general transcription factor or coactivator of the RNA Pol II system (2), and loss of function for many of these components is lethal. Components of general factor TFIID include the TATA binding protein (*SPT15*) and 14 other associated factors (TAFs) that are collectively thought to be the main DNA binding proteins regulating promoter specificity in yeast (*2-5*). Mutations in a TATA-binding protein have been shown to change the preference of the three polymerases and play an important role in promoter specificity (*6*).

Successful fermentations to produce ethanol using yeast require tolerance to both high glucose and ethanol concentrations. These cellular characteristics are important as very high gravity (VHG) fermentations, which are common in the ethanol industry, give rise to high sugar concentrations (and thus high osmotic pressure) at the beginning and high ethanol concentration at the end of a batch (*7, 8*). As with ethanol tolerance in *E. coli,* tolerance to ethanol and glucose mixtures does not seem to be a monogenic trait (*9*). Therefore, traditional methods of strain improvement have had limited success beyond the identification of medium supplementations and various chemical protectants (*10-14*).

To evaluate the approach of gTME in a eukaryotic system, two gTME mutant libraries were created from either (*SPT15*) or one of the TATA-binding protein associated factors (*TAF25*) (*15*). The yeast screening and selection was performed in the background of the standard haploid *S. cerevisiae* strain BY4741 containing the endogenous, unmutated chromosomal copy of *SPT15* and *TAF25.* As such, this genetic screen uses a strain that expresses both the wild type and mutated version of the protein and thus permits the identification of dominant mutations that are able to provide a novel function in the presence of the unaltered chromosomal gene. These libraries were transformed into yeast and were selected in the presence of elevated levels of ethanol and glucose. The *spt15* mutant library showed modest growth in the presence of 5% ethanol and 100 g/L of glucose, so the stress was increased in the subsequent serial subculturing to 6% ethanol and 120 g/L of glucose. Following the subculturing, strains were isolated from plates, plasmids containing mutant genes were isolated and retransformed into a fresh background, and tested for their capacity to grow in the presence of elevated glucose and ethanol levels. The best mutant obtained from each of these two libraries was assayed in further detail and sequenced.

The sequence characteristics of these altered genes conferring the best properties (one Spt15p and one Taf25p) are shown in Figure 18A. Each of these mutated genes contained 3 mutations, with those of *spt15* localized to the second repeat element forming a set of beta-sheets (*5*, *16*). These specific triple mutations in the *taf25* and *spt15* mutant genes are hereby referred to as the *taf25-300* and *spt15-300* mutations.

The *spt15-300* mutant outperformed the control at all concentrations tested, with the strain harboring the mutant protein providing upwards of 13 fold improvement in growth yield at some glucose concentrations (Fig. 18B and Fig. 22). The *taf25-300* mutant was unable to grow in the presence of 6% ethanol, consistent with the observations seen during the enrichment/selection phase. Despite these increases in tolerance, the basal growth rate of these mutants in the absence of ethanol and glucose stress was similar to that of the control. Furthermore, the differences in behavior between the *spt15-300* mutant and *taf25-300* mutant suggest that mutations in genes encoding different members of the eukaryotic transcription machinery are likely to elicit different (and unanticipated) phenotypic responses.

The remainder of this study focuses on the *spt15-300* mutant, as this triple mutation set (F177S, Y195H, K218R) provided the most desirable phenotype with respect to elevated ethanol and glucose. At ethanol concentrations above 10%, the *spt15-300* mutant exhibited statistically significantly improved cellular viability (over the course of 30 hours of culturing) above that of the control, even at concentrations as high as 20% ethanol by volume (Fig. 19A, 19B and Fig. 23).

Transcriptional profiling revealed that the mutant *spt15-300* exhibited differential expression of hundreds of genes (controlled for false-discovery (*17*)) in the unstressed condition (0% ethanol and 20 g/L glucose) relative to cells expressing the wild-type *SPT15* (*18*). This analysis mainly utilized the unstressed condition rather than the stressed (5% ethanol and 60 g/L glucose) since expression ratios were more reliable under this condition due to the similarity of growth rates, thus making gene expression profiles more comparable (Supplemental text, part c and Table 6). It is noted that the impact of the ethanol/glucose stress had a variable effect on many of the genes and often, the stress did not further affect many of the genes selected using unstressed conditions (Supplemental text, part c). Although this widespread alteration in transcription is similar to that observed in *E. coli* with an altered sigma factor, the majority of the genes with altered expression are upregulated unlike the balanced distribution seen with *E. coli* (Supplemental text, part b and Fig. 24). The transcriptional reprogramming in the *spt15-300* mutant was quite broad, yet exhibits some enrichment of certain functional groups such as oxidoreductase activity, cytoplasmic proteins, amino acid metabolism, and electron transport (Supplemental text, part b and Fig. 25). Unclassified genes or genes with no known function were also found with higher levels of expression. An analysis of promoter binding sites, as well as a search for active gene subnetworks using the Cytoscape (*19*) framework failed to unveil any substantial leads into a particular pathway or genetic network being predominately responsible for the observed genetic reprogramming (*15*).

To determine whether these upregulated genes acted individually or as an ensemble to provide increased ethanol/glucose tolerance, we examined the effect of individual gene knockouts on the phenotype. Twelve of the most highly expressed genes in the mutant under the unstressed conditions of 0% ethanol and 20 g/L of glucose were selected along with 2 additional genes (Supplemental text, part c and Tables 5, 6). Figure 20A summarizes the results of the loss-of-phenotype assay. The results show that deletion of the great majority of the overexpressed gene targets resulted in a loss of the capacity of the mutant *spt15-300* factor to impart an increased ethanol/glucose tolerance. All tested knockout strains not harboring the mutant *spt15-300* showed normal tolerance to ethanol and glucose stress, thus indicating that, individually, these genes are insufficient to constitute the normal tolerance to ethanol. Out of the 14 gene targets assayed, only loss of *PHM6* function did not reduce the novel phenotype. Thus, we hypothesize that each gene encodes a necessary component of an interconnected network although there may be some redundancy of function (Supplemental text, part c).

Three genes that exhibited the highest-fold increase in expression level in the *spt15-300* mutant were investigated as overexpression targets in the control strain in a gain-of-function assay. *PHO5, PHM6,* and *FMP16* were independently and constitutively overexpressed under the control of the *TEF* promoter, and transformants were assayed for their capacity to impart an ethanol and glucose tolerance phenotype. Figure 20B illustrates that overexpression of no single gene among the consensus, top candidate genes from the microarray analysis can produce a gain-of-phenotype similar to that of the mutant *spt15-300.*

We next constructed all possible single and double mutant combinations with the sites identified in the triple mutant (*15*). None of the single or double mutants came even close to achieving a similar phenotype to that of the isolated *spt15-300* triple mutant (Supplemental text, part d and Figs. 27-29). One could not predict the effect of these three mutations by a "greedy algorithm" search approach, or select these by traditional selection for mutations that cause incremental improvement as many of these isolated mutations are independently relatively neutral in phenotype fitness. Consequently, such a multiple mutant is accessible only through a technique that specifically focuses on the in vitro mutagenesis of the *SPT15* gene followed by a demanding selection.

Genes previously documented as *SPT3* dependent in expression (*20, 21*) were preferentially altered by our *spt15* mutant, as exhibited in the microarray data, with a Bonferroni-corrected p-value of 1x10⁻¹². Furthermore, 7 of the 10 most highly expressed genes in the *spt15-300* mutant are *SPT3* dependent genes. Genes that are downregulated in *spt3* mutants were relatively upregulated in the *spt15-300* mutant. The absence of negative cofactor 2 element (NC2) repression due to the Y195H mutation (22) may result in over-representation of upregulated genes because part of the negative regulation of the Spt15p can no longer take place. These data are consistent with previous work showing that the *spt15-21* mutation (a F177L and F177R change) suppresses an *spt3* mutation as the result of an altered interaction between the Spt15p and Spt3p (part of the SAGA complex) (*21*, *23, 24*). To further test the link between Spt3p, it was found that an *spt15-300* mutant gene was unable to impart its ethanol and glucose tolerance phenotype to an *spt3* knockout strain (Fig. 21A).

From the results of the site-directed mutagenesis and mechanism depicted in Figure 21B, it is conceivable that perturbations to the NC2 complex would also impact the ability of the *spt15-300* mutant to function; however, the essentiality of one of the genes in this heterodimer prevents such a follow-up experiment. Nevertheless, these results further underscore the importance of all three mutations acting in concert in order to create the complex phenotype mediated through an Spt3p / SAGA complex interaction. As a result, we posit that the mode-of-action is primarily a unique protein-protein-DNA interaction (*SPT15* mutant - *SPT3 -* DNA) leading to this transcriptional reprogramming of a large number of genes.

The capacity of the *spt15-300* mutant to utilize and ferment glucose to ethanol under a variety of conditions was assayed in simple batch shake flask experiments of low and high cell density under an initial concentration of 20 or 100 g/L of glucose (Supplemental text, part e and Figs. 30-32). In each of these cases, the mutant has growth characteristics superior to those of the control with a prolonged exponential growth phase which allows for a higher, more robust biomass production and a higher ethanol yield. Specifically, in high cell density fermentations, with an initial OD600 of 15, the mutant far exceeds the performance of the control with more rapid utilization of glucose, improved biomass yield, and with higher volumetric ethanol productivity (2 g/L of ethanol per hour) relative to the control strain (Table 3). In addition, sugars were rapidly and fully utilized at a yield that exceeds that of the control and approaches the theoretical value when taking account for the amount of glucose consumed for cell growth.

These results demonstrate the applicability of global transcription machinery engineering to alter cellular eukaryotic phenotypes. The isolation of dominant mutations permits the modification of vital functions for novel tasks, while the unmodified allele carries out the functions critical for viability. An examination of further modifications of other transcription factors through global transcription machinery engineering could additionally have the potential for drastically improving ethanol fermentations and improving the prospects of ethanol production. For the mutants analyzed, altered fermentation conditions and further pathway engineering are likely to further increase ethanol production (25, 26). Furthermore, the strain used in this study is a standard laboratory yeast strain and this method could be explored in industrial or isolated yeast exhibiting naturally higher starting ethanol tolerances. Finally, we note that the transcription factors modified in this study have similarity to those in more complex eukaryotic systems including those of mammalian cells, which raises the possibility of using this tool to elicit complex phenotypes of both biotechnological and medical interest in these systems as well.

### References and Notes

1. H. Alper, G. Stephanopoulos, *Awaiting Citation Information* (2006).
2. S. Hahn, *Nat Struct Mol Biol* **11***,* 394-403 (2004).
3. M. Hampsey, Microbiol Mol Biol Rev 62, 465-503 (1998).
4. J. L. Kim, D. B. Nikolov, S. K. Burley, Nature 365, 520-7 (1993).
5. D. I. Chasman, K. M. Flaherty, P. A. Sharp, R. D. Kornberg, Proc Natl Acad Sci U S A 90, 8174-8 (1993).
6. M. C. Schultz, R. H. Reeder, S. Hahn, Cell 69, 697-702 (1992).
7. R. Thatipamala, S. Rohani, G. Hill, Biotechnology and Bioengineering 40, 289-297 (1992).
8. F. W. Bai, L. J. Chen, Z. Zhang, W. A. Anderson, M. Moo-Young, J Biotechnol 110, 287-93 (2004).
9. F. van Voorst, J. Houghton-Larsen, L. Jonson, M. C. Kielland-Brandt, A. Brandt, Yeast 23, 351-9 (2006).
10. Z. P. Cakar, U. O. Seker, C. Tamerler, M. Sonderegger, U. Sauer, FEMS Yeast Res 5, 569-78 (2005).
11. K. Furukawa, H. Kitano, H. Mizoguchi, S. Hara, J Biosci Bioeng 98, 107-13 (2004).
12. M. Nozawa, T. Takahashi, S. Hara, H. Mizoguchi, J Biosci Bioeng 93, 288-95 (2002).
13. Y. Ogawa et al., J Biosci Bioeng 90, 313-20 (2000).
14. H. Takagi, M. Takaoka, A. Kawaguchi, Y. Kubo, Appl Environ Microbiol 71, 8656-62 (2005).
15. Materials and methods are available as supporting online material in Science Online.
16. J. H. Geiger, S. Hahn, S. Lee, P. B. Sigler, Science 272, 830-6 (1996).
17. J. D. Storey, R. Tibshirani, Proc Natl Acad Sci U S A 100, 9440-5 (2003).
18. For each gene, a p-value for differential expression between the two conditions was calculated using a t-test. To simultaneously test multiple hypotheses, p-values were corrected in a false discovery rate analysis (*17*). False discovery rates are a common method used for the analysis of large date sets (such as microarrays) which limits false positives, akin to a Bonferroni correction. In this case, 366 genes were found to be significantly differentially expressed, at a false discovery rate of 1%.
19. P. Shannon et al., Genome Res. 13, 2498-2504 (2003).
20. K. L. Huisinga, B. F. Pugh, Mol Cell 13, 573-85 (2004).
21. T. I. Lee et al., Nature 405, 701-4 (2000).
22. Y. Cang, D. T. Auble, G. Prelich, Embo J 18, 6662-71 (1999).
23. H. Kou, J. D. Irvin, K. L. Huisinga, M. Mitra, B. F. Pugh, Mol Cell Biol 23, 3186-201 (2003).
24. D. M. Eisenmann, K. M. Arndt, S. L. Ricupero, J. W. Rooney, F. Winston, Genes Dev 6, 1319-31 (1992).
25. T. L. Nissen, M. C. Kielland-Brandt, J. Nielsen, J. Villadsen, Metab Eng 2, 69-77 (2000).
26. P. Slininger, B. Dien, S. Gorsich, Z. Liu, Applied Microbiology and Biotechnology 10.1007/s00253-006-0435-1 (2005).
27. M. P. Klejman, X. Zhao, F. M. van Schaik, W. Herr, H. T. Timmers, Nucleic Acids Res 33, 5426-36 (2005).
28. D. K. Lee, J. DeJong, S. Hashimoto, M. Horikoshi, R. G. Roeder, Mol Cell Biol 12, 5189-96 (1992).
29. G. M. O'Connor, F. Sanchez-Riera, C. L. Cooney, Biotechnology and Bioengineering 39, 293-304 (1992).
30. Microarray data deposited to the GEO database under the accession number GSE5185.

### Materials and Methods

### Strains and Media

*S. cerevisiae* strain BY4741 (*MAT*a; *his3*Δ*1*; *leu2*Δ*0*; *met15*Δ*0*; *ura3Δ0*) used in this study was obtained from EUROSCARF (Frankfurt, Germany). It was cultivated in YPD medium (10 g of yeast extract/liter, 20 g of Bacto Peptone/liter and 20 g glucose/liter). For yeast transformation, the Frozen-EZ Yeast Transformation II (ZYMO RESEARCH) was used. To select and grow yeast transformants bearing plasmids with the *URA3* selectable marker, a yeast synthetic complete (YSC) medium was used containing 6.7 g of Yeast Nitrogen Base (Difco)/liter, 20 g glucose/liter and a mixture of appropriate nucleotides and amino acids (CSM-URA, Qbiogene) referred here as to YSC -Ura. Medium was supplemented with 1.5% agar for solid media. Stock solutions of 600 g/L of glucose, 5x solutions of CSM-URA and 10x YNB were used for the preparation of medium. Strains were grown at 30°C with 225 RPM orbital shaking. Gene knockout strains were obtained from the Invitrogen knockout strains collection and were all from the BY4741 genetic background. *E*. *coli* DH5α maximum efficiency competent cells (Invitrogen) were used for routine transformations as per manufacturer instructions and were routinely cultivated in LB medium containing 100 µg/ml of ampicillin. *E. coli* strains were routinely grown at 37°C. Cell density was monitored spectrophotometrically at 600 nm. All remaining chemicals were from Sigma-Aldrich. Primers were purchased from Invitrogen.

### Library Construction

The library was created and cloned behind the *TEF*-mut2 promoter created previously as part of a yeast promoter library (*1*) in the p416 plasmid (*2*). The *TAF25* and *SPT15* genes were cloned from genomic DNA, isolated from BY4741 yeast using the Promega Wizard Genomic DNA kit. Amplification was performed using Taq polymerase (NEB) using the primers TAF25_Sense: TCGAGTGCTAGCAAAATGGATTTTGAGGAAGATTACGAT (SEQ ID NO:28) and TAF25_Anti: CTAGCGGTCGACCTAACGATAAAAGTCTGGGCGACCT (SEQ ID NO:29). The Spt15 gene was cloned from genomic DNA using the primers SPT15_Sense: TCGAGTGCTAGCAAAATGGCCGATGAGGAACGTTTAAAGG (SEQ ID NO:30) and SPT15_Anti: CTAGCGGTCGACTCACATTTTTCTAAATTCACTTAGCACA (SEQ ID NO:31). Fragment mutagenesis was performed using the GenemorphII Random Mutagenesis kit (Stratagene) using various concentrations of initial template to obtain low (0 - 4.5 mutations/kb), medium (4.5 - 9 mutations/kb), and high mutation (9-16 mutations/kb) rates as described in the product protocol. Following PCR, these fragments were purified using a Qiagen PCR cleanup kit and were digested overnight at 37°C using NheI and SaII and ligated overnight at 16°C to plasmid backbone digested with Xbal and SaII. The plasmids libraries were transformed into *E. coli* DH5α and plated onto LB-agar plates containing 100 ug/ml of ampicillin. The total library size of was approximately 10⁵. Colonies of *E. coli* were scraped off the plate and plasmids were isolated using a plasmid MiniPrep Spin Kit and transformed into yeast. The yeast screening and selection was performed in the background of the standard haploid *S*. *cerevisiae* strain BY4741 containing the endogenous, unmutated chromosomal copy of *SPT15* and *TAF25.* Yeast transformation mixtures were plated on a total of 48- 150 x 10 mm Petri dishes for each of the two libraries (one for *taf25* and one for *spt15*). These transformants were scraped off the plates and placed into a liquid suspension for phenotype selection. Isolated strains were isolated using the Zymoprep yeast plasmid miniprep (ZYMO research) and back-transformed into *E. coli.* Plasmids were sequenced using the primers: Seq_Forward: TCACTCAGTAGAACGGGAGC (SEQ ID NO:32) and Seq_Reverse: AATAGGGACCTAGACTTCAG (SEQ ID NO:33). Sequences were aligned and compared using Clustal W version 1.82.

All mutant strains were compared to a control strain which harbored the unmutated version of either the *SPT15* or *TAF25* protein cloned into the same promoter and plasmid construct (the p416 plasmid containing the *TEF*-mut2 promoter as described above). As a result, the influence of the plasmid and interference between both plasmid and chromosomal copies of transcriptional machinery are neutralized through the use of the control. Due to similar promoter and plasmid constructs, the excess wild-type protein is expressed at the same level as the mutant protein. Furthermore, additional phenotype analysis comparing blank plasmids (those not expressing either the *SPT15* or the *TAF25*) versus those expressing a wild-type protein (either *SPT15* or *TAF25*) in the presence of various ethanol/glucose concentrations revealed similar growth rates. As a result, the overexpression of the wild-type protein does not impact the phenotype or selection of the control. Table 4 summarizes the comparison between strains harboring a blank plasmid, a plasmid containing the wild-type *SPT15* and a plasmid containing the spt15-300 mutant.

### Phenotype Selection

Samples from the pooled liquid library were placed into a challenging environment to select for surviving mutants. For the ethanol/glucose tolerance phenotype, the library was initially placed in YSC-URA containing 100 g/L of glucose and 5% ethanol by volume. These cultures were performed in 30 x 115 mm closed top centrifuge tubes containing 30 ml of culture volume and placed vertically in a shaking, orbital incubator at 30°C. Initially, the culture was started with an OD600 of 0.05. Both the *taf25* and *spt15* libraries were subcultured 2 times under these conditions. Since the *spt15* library grew under initial conditions, the stress was increased to 120 g/L of glucose and 6% ethanol for 2 more subculturings. A constant level of 100 g/L of glucose and 5% ethanol was used for 2 more subculturings of the *taf25* library. Following this selection phase, these mixtures were plated through streaking solutions onto a YSC-URA plate containing 20 g/L of glucose to ensure single-colony isolation without the need for diluting the sample. Approximately 20 colonies were randomly isolated from the large number of colonies which grew on the plates. These selected strains were then grown in overnight cultures and assayed for growth in 60 g/L glucose and 5% ethanol containing medium. For cells which showed an improvement in growth performance as measured by OD, plasmids were isolated and retransformed to revalidate phenotypes in biological replicates at several concentrations. These phenotype validations were performed as described below.

### Growth yield assays

Biological replicates were grown overnight in 5 ml of culture volume in a 14 ml Falcon culture tube. Medium containing 8 of the following condition: 5% ethanol with 20, 60, 100, or 120 g/L of glucose and 6% ethanol with 20, 60, 100, or 120 g/L of glucose were dispensed in 5 ml aliquots into 15 ml conical centrifuge tubes with caps. Cells were inoculated with an initial OD of 0.01. Strains are cultivated by placing the tubes vertically into a 30°C incubator with 225 RPM orbital shaking. After 20 hours, tubes are vortexed and cell densities are measured by taking optical density at 600 nm.

### Viability curve assays

Cultures were grown in 50 ml of YSC-URA medium in 250 ml flasks for 2 days at 30°C. Approximately 1 ml (precise amount to yield an OD600 of 0.5 when re-suspended in 10 ml) was placed into a 15 ml conical centrifuge tube and centrifuged at 500 x g for 15 minutes. Cells were then washed with 10 ml of 0.9% NaCl and recentrifuged. The cell pellet was then resuspended in YSC-URA containing 20 g/L of glucose and an appropriate amount of ethanol (between 10 and 20%). This tube was then incubated at 30°C with 225 RPM orbital shaking. A 100 µL sample was removed (following vortexing to ensure homogeneity) every three hours (including the zero timepoint) and appropriately diluted and plated onto YSC-URA plates. Plates were then cultured for 2 days to allow for colony formation and colony forming unit counts. Both the mutant and control strains were cultivated in biological replicate.

### Site-directed mutagenesis

Site directed mutagenesis was performed using the Stratagene Quickchange kit to introduce the single and double mutations into the *SPT15* gene. The mutagenesis followed the protocol of the kit as well used the sequencing primers described above for sequencing verification. The following primer sets were used:
Construct: F177S, Template: *SPT15* wild-type
   HA350-F177S_sen (SEQ ID NO:34), CGTCTAGAAGGGTTAGCATCCAGTCATGGTACTTTCTCCTCCTATGAGC
   HA351-F177S_ant (SEQ ID NO:35), GCTCATAGGAGGAGAAAGTACCATGACTGGATGCTAACCCTTCTAGACG
Construct: Y195H, Template: *SPT15* wild-type
   HA352-Y195H_sen (SEQ ID NO:36, CCAGAATTGTTTCCTGGTTTGATCCATAGAATGGTGAAGCC
   HA353-Y195H_ant (SEQ ID NO:37), GGCTTCACCATTCTATGGATCAAACCAGGAAACAATTCTGG
Construct: K218R, Template: *SPT15* wild-type
   HA354-K218R_sen (SEQ ID NO:38), GGAAAGATTGTTCTTACTGGTGCAAGGCAAAGGGAAGAAATTTACC
   HA355-K218R_ant (SEQ ID NO:39), GGTAAATTTCTTCCCTTTGCCTTGCACCAGTAAGAACAATCTTTCC
Construct:Y195H and K218R, Template: mutant *spt15*
   HA356-F177-Revert_sen (SEQ ID NO:40), CGTCTAGAAGGGTTAGCATTCAGTCATGGTACTTTCTCCTCCTATGAGC
   HA357-F177-Revert_ant (SEQ ID NO:41), GCTCATAGGAGGAGAAAGTACCATGACTGAATGCTAACCCTTCTAGACG
Construct: F177S and K218R, Template: mutant *spt15*
   HA358-Y195H-revert_sen (SEQ ID NO:42), CCAGAATTGTTTCCTGGTTTGATCTATAGAATGGTGAAGCC
   HA359-Y195H-revert_ant (SEQ ID NO:43), GGCTTCACCATTCTATAGATCAAACCAGGAAACAATTCTGG
Construct:F177S and Y195H, Template: mutant *spt15*
   HA360-K218R-revert_sen (SEQ ID NO:44), GGAAAGATTGTTCTTACTGGTGCAAAGCAAAGGGAAGAAATTTACC
   HA361-K218R-revert_ant (SEQ ID NO:45), GGTAAATTTCTTCCCTTTGCTTTGCACCAGTAAGAACAATCTTTCC

### Gene over-expression constructs

Overexpression constructs were created using the p416-TEF plasmid. PHO5 (YBR093C) was amplified from BY4741 genomic DNA using the primers PHO5_sen-XhoI: CCGCTCGAGCAAAACTATTGTCTCAATAGACTGGCGTTG (SEQ ID NO:46) and PHO5_anti-XbaI: GCTCTAGACCAATGTTTAAATCTGTTGTTTATTCAATT (SEQ ID NO:47). This fragment was then cloned into a vector which has been digested by Xhol and XbaI. PHM6 (YDR281C) was amplified from BY4741 genomic DNA using the primers PHM6_sen-SalI: ACGCGTCGACATTATTAAAACAAAAACTTCGTCATCGTCA (SEQ ID NO:48) and PHM6_anti-XbaI: GCTCTAGACCAAGATGGAAGATACCTCGAGGTGCATCG (SEQ ID NO:49). This fragment was then cloned into a vector which has been digested by SaII and XbaI. FMP16 (YDR070C) was amplified from BY4741 genomic DNA using the primers FMP16_sen-XhoI: CCGCTCGAGGTGCTTCTTAATAAACACCGTCATCTGGCC (SEQ ID NO:50) and FMP16_anti-XbaI: GCTCTAGAATAATGTTGAGAACCACTTTTTTGCGCACT (SEQ ID NO:51). This fragment was then cloned into a vector which has been digested by XhoI and XbaI.

### Fermentation

Low inoculum cultures were started using an overnight culture of yeast at an OD600 of 0.01 in 50 ml of medium containing either 20 or 100 g/L of glucose. Samples were taken every 3 hours for OD600 and supernatant analysis was conducted to measure ethanol and glucose concentrations. High inoculum cultures were created by growing 250 ml of yeast in a 1000 ml flask for 1.5 days, then collected by centrifugation at 500xg for 25 minutes. The cell pellet was then resuspended in 3 ml of YSC-URA without glucose. This solution was then appropriately inoculated into 40 ml of YSC-URA containing 100 g/L in a 250 ml flask to obtain a starting OD600 of around 15. Ethanol concentrations were determined by enzymatic assay kit (R-Biopharm, SouthMarshall, Michigan) and glucose concentrations were measured using a YSI 2300 glucose analyzer. Fermentations were run in biological replicates for 30 hours with samples taken every 3 hours.

### Microarray analysis.

Yeast strains (*spt15* mutant and control, grown in standard YSC-URA medium and medium containing 5% ethanol with 60 g/L of glucose) were grown to an OD of approximately 0.4 - 0.5 and RNA was extracted using the Ambion RiboPure Yeast RNA extraction kit. Microarray services were provided by Ambion, Inc. using the Affymetrix Yeast 2.0 arrays. Arrays were run in triplicate with biological replicates to allow for statistical confidence in differential gene expression. Microarray data as well as data regarding the MIAME compliance has been deposited to the GEO database with an accession number of GSE5185.

Functional enrichment, gene ontology, and network analysis were completed using the BiNGO application in Cytoscape 2.1 (*3*). Furthermore, Cytoscape 2.1 was used to search for active subnetworks using networks for protein-protein and protein-DNA networks assayed under YPD, starvation and oxidative stress conditions.

### Supplemental Text

***S. cerevisiae* SPT15 (TBP) has GeneID: 856891, protein accession no. NP_011075.1, (SEQ ID NO:52)**

madeerlkefkeankivfdpntrqvwenqnrdgtkpattfqseedikraapesekdtsat
sgivptlqnivatvtlgcrldlktvalharnaeynpkrfaavimrirepkttalifasgk
mvvtgakseddsklasrkyariiqkigfaakftdfkiqnivgscdvkfpirleglafshg
tfssyepelfpgliyrmvkpkivllifvsgkivltgakqreeiyqafeaiypvlsefrkm

### a) Sequence analysis of the spt15-300 and taf250-300 mutants

Sequence alignment of *spt15-300* mutant, comparing Wild-type_Spt15 (SEQ ID NO:53) and EtOH-Glc_SPT15_Mutant (SEQ ID NO:54):

Wild-type_Spt15 ATGGCCGATGAGGAACGTTTAAAGGAGTTTAAAGAGGCAAACAAGATAGT 50
EtOH-Glc_SPT15_Mutant ATGGCCGATGAGGAACGTTTAAAGGAGTTTAAAGAGGCAAACAAGATAGT 50
**************************************************

Wild-type_Spt15 GTTTGATCCAAATACCAGACAAGTATGGGAAAACCAGAATCGAGATGGTA 100
EtOH-Glc_SPT15_Mutant GTTTGATCCAAATACCAGACAAGTATGGGAAAACCAGAATCGAGATGGTA 100
**************************************************

Wild-type_Spt15 CAAAACCAGCAACTACTTTCCAGAGTGAAGAGGACATAAAAAGAGCTGCC 150
EtOH-Glc_SPT15_Mutant CAAAACCAGCAACTACTTTCCAGAGTGAAGAGGACATAAAAAGAGCTGCC 150
**************************************************

Wild-type_Spt15 CCAGAATCTGAAAAAGACACCTCCGCCACATCAGGTATTGTTCCAACACT 200
EtOH-Glc_SPT15_Mutant CCAGAATCTGAAAAAGACACCTCCGCCACATCAGGTATTGTTCCAACACT 200
**************************************************

Wild-type_Spt15 ACAAAACATTGTGGCAACTGTGACTTTGGGGTGCAGGTTAGATCTGAAAA 250
EtOH-Glc_SPT15_Mutant ACAAAACATTGTGGCAACTGTGACTTTGGGGTGCAGGTTAGATCTGAAAA 250
**************************************************

Wild-type_Spt15 CAGTTGCGCTACATGCCCGTAATGCAGAATATAACCCCAAGCGTTTTGCT 300
EtOH-Glc_SPT15_Mutant CAGTTGCGCTACATGCCCGTAATGCAGAATATAACCCCAAGCGTTTTGCT 300
**************************************************

Wild-type_Spt15 GCTGTCATCATGCGTATTAGAGAGCCAAAAACTACAGCTTTAATTTTTGC 350
EtOH-Glc_SPT15_Mutant GCTGTCATCATGCGTATTAGAGAGCCAAAAACTACAGCTTTAATTTTTGC 350
**************************************************

Wild-type_Spt15 CTCAGGGAAAATGGTTGTTACCGGTGCAAAAAGTGAGGATGACTCAAAGC 400
EtOH-Glc_SPT15_Mutant CTCAGGGAAAATGGTTGTTACCGGTGCAAAAAGTGAGGATGACTCAAAGC 400

**************************************************

Wild-type_Spt15          TGGCCAGTAGAAAATATGCAAGAATTATCCAAAAAATCGGGTTTGCTGCT 450
EtOH-Glc_SPT15_Mutant    TGGCCAGTAGAAAATATGCAAGAATTATCCAAAAAATCGGGTTTGCTGCT 450
                         **************************************************

Wild-type_Spt15          AAATTCACAGACTTCAAAATACAAAATATTGTCGGTTCGTGTGACGTTAA 500
EtOH-Glc_SPT15_Mutant    AAATTCACAGACTTCAAAATACAAAATATTGTCGGTTCGTGTGACGTTAA 500
                         **************************************************

Wild-type_Spt15          ATTCCCTATACGTCTAGAAGGGTTAGCATTCAGTCATGGTACTTTCTCCT 550
EtOH-Glc_SPT15_Mutant    ATTCCCTATACGTCTAGAAGGGTTAGCATCCAGTCATGGTACTTTCTCCT 550
                         ***************************** ********************

Wild-type_Spt15          CCTATGAGCCAGAATTGTTTCCTGGTTTGATCTATAGAATGGTGAAGCCG 600
EtOH-Glc_SPT15_Mutant    CCTATGAGCCAGAATTGTTTCCTGGTTTGATCCATAGAATGGTGAAGCCG 600
                         ******************************* ******************

Wild-type_Spt15          AAAATTGTGTTGTTAATTTTTGTTTCAGGAAAGATTGTTCTTACTGGTGC 650
EtOH-Glc_SPT15_Mutant    AAAATTGTGTTGTTAATTTTTGTTTCAGGAAAGATTGTTCTTACTGGTGC 650
                         **************************************************

Wild-type_Spt15          AAAGCAAAGGGAAGAAATTTACCAAGCTTTTGAAGCTATATACCCTGTGC 700
EtOH-Glc_SPT15_Mutant    AAGGCAAAGGGAAGAAATTTACCAAGCTTTTGAAGCTATATACCCTGTGC 700
                         ** ***********************************************

Wild-type_Spt15          TAAGTGAATTTAGAAAAATGTGA 723
EtOH-Glc_SPT15_Mutant    TAAGTGAATTTAGAAAAATGTGA 723
                         ***********************

Sequence alignment of *taf25-300* mutant, comparing Wild-type_Taf25 (SEQ ID NO:55) and EtOH-Glc_TAF25_Mutant (SEQ NO:56)

Wild-type_Taf25 ATGGATTTTGAGGAAGATTACGATGCGGAGTTTGATGATAATCAAGAAGG 50
EtOH-Glc_TAF25_Mutant ATGGATTTTGAGGAAGATTACGATGCGGAGTTTGATGATAATCAAGAAGG 50
**************************************************

Wild-type_Taf25 ACAATTAGAAACACCTTTTCCATCGGTTGCGGGAGCCGATGATGGGGACA 100
EtOH-Glc_TAF25_Mutant ACAATTAGAAACACCTTTTCCATCGGTTGCGGGAGCCGATGGTGGGGACA 100
***************************************** ********

Wild-type_Taf25 ATGATAATGATGACTCTGTCGCAGAAAACATGAAGAAGAAGCAAAAGAGA 150
EtOH-Glc_TAF25_Mutant ATGATAATGATGACTCTGTCGCAGAAAACATGAAGAAGAAGCAAAAGAGA 150
**************************************************

Wild-type_Taf25 GAGGCTGTAGTGGATGATGGGAGTGAAAATGCATTTGGTATACCCGAATT 200
EtOH-Glc_TAF25_Mutant GAGGCTGTAGAGGATGATGGGAGTGAAAATGCATTTGGTATACCCGAATT 200
********** ***************************************

Wild-type_Taf25 TACAAGAAAAGATAAGACTCTGGAGGAGATTCTAGAGATGATGGACAGTA 250
EtOH-Glc_TAF25_Mutant TACAAGAAAAGATAAGACTCTGGAGGAGATTCTAGAGATGATGGACAGTA 250
**************************************************

Wild-type_Taf25 CTCCTCCTATCATTCCCGATGCAGTAATAGACTACTATTTAACCAAAAAC 300
EtOH-Glc_TAF25_Mutant CTCCTCCTATCATTCCCGATGCAGTAATAGACTACTATTTAACCAAAAAC 300
**************************************************

Wild-type_Taf25 GGGTTTAACGTAGCAGATGTACGAGTGAAACGACTTTTAGCACTTGCTAC 350
EtOH-Glc_TAF25_Mutant GGGTTTAACGTAGCAGATGTACGAGTGAAACGACTTTTAGCACTTGCTAC 350
**************************************************

Wild-type_Taf25 TCAGAAATTTGTTAGTGATATAGCTAAGGATGCCTACGAATATTCCAGGA 400
EtOH-Glc_TAF25_Mutant TCAGAAATTTGTTAGTGATATAGCTAAGGATGCCTACGAATATTCCAGGA 400
**************************************************

Wild-type_Taf25 TCAGGTCTTCCGTAGCGGTATCTAATGCTAACAACAGTCAGGCGAGAGCT 450
EtOH-Glc_TAF25_Mutant TCAGGTCTTCCGTAGCGGTATCTAATGCTAACAACAGTCAGGCGAGAGCT 450
**************************************************

Wild-type_Taf25 AGGCAGCTATTGCAAGGACAGCAACAGCCTGGCGTGCAGCAGATTTCACA 500
EtOH-Glc_TAF25_Mutant AGGCAGCTATTGCAAGGACAGCAACAGCCTGGCGTGCAGCAGATTTCACA 500
**************************************************

Wild-type_Taf25 ACAACAACATCAACAGAATGAGAAGACTACAGCAAGCAAAGTTGTTCTGA 550
EtOH-Glc_TAF25_Mutant ACAACAACATCAACAGAATGAGAAGACTACAGCAAGCAGAGTTGTTCTGA 550
************************************** ***********

Wild-type_Taf25 CGGTGAACGATCTCAGTAGCGCTGTTGCTGAATACGGGCTCAATATAGGT 600
EtOH-Glc_TAF25_Mutant CGGTGAACGATCTCAGTAGCGCTGTTGCTGAATACGGGCTCAATATAGGT 600
**************************************************

Wild-type_Taf25 CGCCCAGACTTTTATCGTTAG 621
EtOH-Glc_TAF25_Mutant CGCCCAGACTTTTATCGTTAG 621
*********************

### b) Microarray analysis of perturbation

### i. Histogram of differentially expressed genes

Genes with differential expression at a p-value of less than or equal to 0.001 were plotted on a histogram to evaluate the breadth and impact of the *spt15-300* mutant under the unstressed conditions (0% ethanol and 20 g/L glucose). Figure 24 illustrates that the *spt15-300* mutant has a bias for upregulating genes. In particular, 111 genes were upregulated under statistical thresholds of p-value ≤ 0.001 and log2 fold ratio of ≥ 0.3. This contrasts with only 21 genes downregulated at the same thresholds of p-value ≤ 0.001 and log2 fold ratio of ≤ -0.3.

### ii. Gene ontology analysis

Gene ontology (GO) analysis allows for the identification of functional enrichment of various cellular functions in a selected subset of genes and can help identify classes of gene function which are strongly correlated with the enhanced phenotype. In particular, a GO analysis of the genes differentially expressed at a p-value threshold of 0.005 was conducted for the *spt15-300* mutant strain in the unstressed condition. This GO analysis revealed the following ontology gene clusters to be overrepresented in the differentially expressed genes of the *spt15-300* mutant strain: oxidreductase activity (p-value: 4.5x10⁻⁸), cytoplasmic proteins and enzymes (p-value: 5.3x10⁻⁴), amino acid and derivative metabolism (p-value: 5.7x10⁻⁴), vitamin metabolism (p-value: 4.9x10⁻³), and electron transport (p-value: 4.5x10⁻²)

Previously, we have identified and analyzed *E. coli* strains with enhanced ethanol tolerance (*4*). These results may be used in a comparative transcriptomics approach to complement these yeast microarrays to extract a conserved mechanism of ethanol tolerance. Figure 25 compares the gene ontology results between the *E. coli* and yeast mutant strains. Interestingly, despite the difference in these proteins and transcriptional machinery, both elicited a similar responses in oxidoreductase activity (GO:0016491) and electron transport (GO:0006118). This convergence of altered genes suggests that ethanol stress either causes an oxidative stress or requires cells with higher levels of reduction. This response is similar to proposed modes of action of ethanol in livers. In addition, recent studies in *drosophila* have identified the *hangover* gene, which when knocked out decreases ethanol tolerance (*5*). Interestingly, this gene knockout also makes the flies more susceptible to the oxidative stress of paraquat, which could suggest the importance and implication of oxidoreductase activity in higher level organisms ethanol tolerance as well. Outside of the oxidoreductase pathway, both *E. coli* and yeast mutants also possessed an increased transcript level of several pentose phosphate pathway genes and glycine metabolism. Nevertheless, the results of the follow-up experiments including loss-of-phenotype and over-expression indicate that no single gene is strictly responsible for the phenotype exhibited in these mutant strains, but rather relies on the concerted expression profile of a multitude of genes.

### c) Selected gene targets for loss-of-phenotype analysis

Transcriptional measurements were conducted using microarrays in an attempt to elucidate the phenotypic differences (ethanol/glucose tolerance) between the control strain and that harboring the mutant *spt15-300* gene. In order to test the hypothesis of distributed genetic control of the phenotype, we selected a small subset of highly overexpressed genes for loss-of-phenotype analysis. A total of 14 genes were selected for this purpose, as indicated and tabulated in Table 5 using the following criteria:
- First, genes were sorted based on overexpression ratios in the unstressed condition and selected the top 19 genes (this cutoff number was arbitrary). Of these 19 genes, *YBR117C (TKL2,* log2 = 1.243), *YDR034W* (log2 = 1.017), *YIL160C* (*POT1,* log2=0.680), *YIL169C* (log2 = 0.625), and *YOL052C* (log2 = 0.622) were excluded due to the inability to obtain these knockout strains in a BY4717 background from the knockout collection. Furthermore, genes with overlapping function, such as *PHM8* (log2 = 1.727) and *PHO11* (log2 = 1.410), were excluded. This left 12 genes, selected based on overexpression ratios in the unstressed condition.
- Secondly, genes were sorted to select those in the top 1% under both stressed and unstressed conditions. This yielded 6 genes of which only 2 (YKL086W and YIL099W) were overexpressed in the stressed condition *only.* In other words, the other 4 genes were overexpressed in the stressed condition as well as the unstressed.

The majority of the selected genes are from the group that show overexpression in the unstressed condition. The reason is twofold: (a) the number of overexpressed genes in the mutant relatively to the control is significantly reduced in the stressed condition (see further discussion below); (b) expression ratios under unstressed conditions are more comparable due to similar growth rate and absence of temporal effects. *Nevertheless, 6 of the 14 selected genes are overexpressed in the stressed condition.* The remaining 8 genes do not seem to be over-expressed in cultures grown under stressed conditions (60 g/L glucose and 5% ethanol). This selection is numerically described in Table 6. A significant overlap of gene targets was seen despite the choice of microarray sets. The exact same phenomenon was also observed in similar experiments we conducted aiming at eliciting ethanol tolerance in *E. coli* through the engineering of mutant sigma factors.

To explain this phenomenon, it is observed that 7 of the remaining 8 genes in question are already over-expressed in cells harboring the mutant transcription factor relatively to the wild type under normal conditions. In the presence of ethanol (stressed conditions), further over-expression of these genes in the mutant is significantly smaller relatively to the level of over-expression of the same genes achieved in the wild type. The result is that these genes are expressed at approximately similar levels in the mutant and the wild type under stressed conditions. As a result, these 7 genes do not appear over-expressed in the mutant but this is *relatively to the control, i.e., wild type cells grown under stressed conditions.* The above hypothesis would have been impossible to test had we carried out transcriptional analysis with spotted two channel microarrays. Fortunately, Affymetrix microarrays report absolute expression levels. These data (absolute fluorescence values) for the 14 genes are shown in the Table 7. One clearly sees that while the great majority of the genes are indeed overexpressed relatively to the wild type control in unstressed conditions, only 6 make it to the top 1% relatively to the wild type under stressed conditions. This is not surprising as there is a ceiling to the extent of gene overexpression that many of the 14 genes seem to reach in response to expression of the mutant transcription factor. The expression data suggest that, under normal conditions, the transcription factor mutant creates a "priming effect" exemplified through overexpression of several genes. As a result of this "priming," cells undergo a significantly reduced change in gene expression under stressed conditions. This apparently creates a beneficial overall effect as far as ethanol tolerance is concerned, the condition under which the mutant was selected.

The fact that for the vast majority of the gene targets tested, loss of function resulted in the loss of the ethanol/glucose tolerance phenotype, indicates that each gene encodes a necessary component of an interconnected network. The identification of this group of genes does not lead to a simple model as there is as yet no obvious connection between their functions. Nevertheless, it is important to note that all tested knockout strains not harboring the mutant *spt15-300* showed normal tolerance to ethanol and glucose stress, thus indicating that, individually, these genes are insufficient to constitute the normal tolerance to ethanol. These results corroborate the conclusion that the complex phenotype imparted by the mutant *spt15-300* is not only pleiotropic, but requires the concerted expression of multiple genes.

Obviously, this gene selection is neither exhaustive nor unique. The loss-of-phenotype exhibited by knocking out individually the majority of these genes in the mutant supports the hypothesis derived from this microarray study; namely, that the ethanol tolerance phenotype is dependent on a specific, reprogramming of the transcriptional levels of a multitude of genes, rather than a single, specific gene or localized pathway.

### d) Phenotype analysis of site-directed mutagenesis derived mutants

We constructed all possible single and double mutant combinations with the sites identified in the triple mutant. These combinations were created by site-directed mutagenesis and assayed under similar conditions to the original assay. The mutant phenotypes (assessed at 20, 60, 100 and 120 g/L of glucose in both 5% and 6% ethanol) were compared to the cumulative phenotype imparted by the *spt15-300* triple mutant in a scale where the value of the wild-type *SPT15* is zero and that of the isolated triple mutant is 1.0. The relative, cumulative fitness of the various mutants are calculated as follows: $Metric = \frac{Σ ⁢ \left(Fold improvement of mutant-1\right)}{Σ ⁢ \left(Fold improvement of triple mutant-1\right)}$

In this case, the fitness is normalized such that the fitness of the wild-type *SPT15* is 0.0 and that of the identified *spt15-300* mutant is 1.0. ). As shown in Figure 27 none of the single or double mutants came even close to achieving a similar phenotype to that of the isolated *spt15-300* triple mutant. The absolute cell density for the control strain under each of these conditions is depicted in Figures 28 and 29 for 5% and 6% ethanol respectively. Tables 8 and 9 list the fold improvement in cell yield (OD600) under each of these 8 conditions.

### e) Fermentation evaluation of the mutant

The capacity of the *spt15-300* mutant to utilize and ferment glucose to ethanol under a variety of conditions was assayed in simple batch shake flask experiments of low and high cell density with an initial concentration of 100 g/L of glucose. Furthermore, a low cell density inoculum experiment was performed with an initial glucose concentration of 20 g/L. These three conditions allowed for the assessment of performance of the mutant *spt15.* These results were compared to the control which was also cultured in 50 ml fermentations under the same conditions. Low cell density experiments were performed using an initial OD600 of 0.1 while high cell density fermentations were performed using an initial OD600 of 15. The mutant initially grew at a similar growth rate as the control, but was able to continue with an extended growth phase to reach a higher final biomass yield. A subsequent improvement in glucose utilization was achieved within the 30 hours for the mutant compared with the control. In addition, ethanol production was more significant in the mutant strain compared with the control in terms of both rate and yield. Figures 30-32 provide fermentation details including cell growth, glucose utilization and ethanol production. Table 3 summarizes the results from the high cell density fermentation in 100 g/L of glucose.

**Table 3: Fermentation results evaluating the ethanol production potential of the sp15 mutant. Cells were cultured in biological replicate in 100 g/L of glucose with a high inoculum of initial cell density of OD 15 (~4 g DCW/L). Fermentation profiles for the high cell density fermentation are provided and illustrate the capacity of this mutant to produce higher productivities of ethanol at the theoretical yield, surpassing the function of the control. Biomass yield from glucose is from reported values (29). Results represent the average between biological replicate experiments (Supplemental text, part e and Figs. 30-32).**

| | *spt15-300* Mutant | Control | % Improvement |
|---|---|---|---|
| Initial dry cell weight (g/L) | 4.06 | 4.10 | - |
| Final dry cell weight (g/L) | 6.46 | 5.39 | +20% |
| Volumetric productivity (g/L h⁻¹) | 2.03 | 1.20 | +69% |
| Specific productivity (g/dcw h) | 0.31 | 0.22 | +41% |
| Conversion yield calculated between 6 and 21 hours | 0.36 | 0.32 | +14% |
| True EtOH yield accounting for biomass production (Percent of 0.41 g/g representing the theoretical maximum) | 0.40 (98%) | 0.35 (86%) | +15% |
| $\left(\frac{g ⁢ { EtOH produced L}^{- 1}}{g Glucose utilized {L}^{- 1} - \left(\frac{1 g glucose}{0.5 g DCW}\right) ⁢ gDCW produced {L}^{- 1}}\right)$ | | | |

**Table 4: Comparison of behavior in ethanol/glucose stress between the blank plasmid, the plasmid expressing the wild-type SPT15, and the plasmid expressing the mutant spt15-300 gene. The overexpression of the wild-type SPT15 did not have an effect on ethanol tolerance relative to the tolerance of cells harboring the blank plasmid only.**

| OD600, 20 hours | | | | |
|---|---|---|---|---|
| Ethanol (%) | Glucose (g/L) | blank plasmid | SPT15 | spt15-300 |
| 5 | 20 | 0.144 | 0.1375 | 0.68 |
| 5 | 60 | 0.066 | 0.106 | 0.53 |
| 5 | 100 | 0.044 | 0.073 | 0.316 |
| 5 | 120 | 0.058 | 0.0695 | 0.3015 |
| 6 | 20 | 0.03 | 0.0285 | 0.2175 |
| 6 | 60 | 0.02 | 0.0265 | 0.241 |
| 6 | 100 | 0.012 | 0.0135 | 0.1785 |
| 6 | 120 | 0.016 | 0.018 | 0.067 |

**Table 5: List of selected genes from microarray analysis for loss-of-phenotype analysis with expression ratios and p-values using the unstressed (20 g/L glucose and 0% ethanol) conditions.**

| Gene | Gene Name | log2(mut/wt) | p-val | Function |
|---|---|---|---|---|
| *YBR093C* | *PHO5* | 2.492 | 1.09E-06 | Repressible acid phosphatase |
| *YML123C* | *PHO84* | 2.176 | 3.78E-06 | High-affinity inorganic Pi transp. and low-affinity manganese transp. |
| *YDR281C* | *PHM6* | 1.843 | 3.05E-04 | Protein of unknown function, expression is regulated by Pi levels |
| *YDR070C* | *FMP16* | 1.742 | 1.49E-03 | Uncharacterized, possibly mitochondrial |
| *YGR043C* | *YGR043C* | 1.584 | 2.33E-04 | Protein of unknown function |
| *YIL099W* | *SGA1* | 1.168 | 7.20E-04 | sporulation-specific glucoamylase involved in glycogen degrad |
| *YPL019C* | *VTC3* | 1.139 | 1.77E-06 | vacuolar H+-ATPase activity |
| *YDR019C* | *GCV1* | 1.026 | 2.44E-05 | mitochondrial glycine decarboxylase complex |
| *YGL263W* | *COS12* | 0.991 | 8.01E-04 | Protein of unknown function |
| *YPR192W* | *AQY1* | 0.971 | 2.25E-04 | Spore-specific water channel |
| *YHR140W* | *YHR140W* | 0.926 | 8.99E-04 | Hypothetical protein |
| *YAL061 W* | *YAL061 W* | 0.924 | 3.71E-03 | putative polyol dehydrogenase |
| *YBR072W* | *HSP26* | 0.902 | 1.79E-03 | Small heat shock protein with chaperone activity |
| *YKL086W* | *SRX1* | 0.873 | 4.70E-04 | Sulfiredoxin |

**Table 6: Overlap of gene sets when created using microarrays from either the unstressed (0% ethanol and 20 g/L glucose) conditions or stressed conditions (5% ethanol and 60 g/L glucose). The gene sets are created by selecting the top 1, 5, or 10% most differentially expressed genes from a given conditions. These genes are then compared with the set of genes obtain from the other microarray conditions at a given threshold (1, 5, or 10%). As an example, when comparing the top 1% of genes from both sets, 27 of the 57 genes are identical. As a result, selecting one condition over the other would provide a unique set of an additional 30 genes to round out the top 1%. A significant overlap of gene targets was seen despite the choice of microarray condition used. In general, the unstressed microarray was used for the analysis in this study. A comprehensive list of these genes may be easily extracted from the deposited micorarray data placed in the GEO database under the accession number of GSE5185.**

| | | Stressed Ratio | | |
|---|---|---|---|---|
| | Genes selected from the top... | 1% | 5% | 10% |
| Unstressed Ratio | 1% | 27/57 | 42/57 | 42/57 |
| | 5% | 44/57 | 118/288 | 160/288 |
| | 10% | 50/57 | 156/288 | 240/576 |

**Table 7: Selected genes with absolute expression levels in both stressed and unstressed conditions to demonstrate the impact of gene expression priming in the mutant strain.**

| | | **Unstressed** | | | **Stressed** | | | |
|---|---|---|---|---|---|---|---|---|
| | | (20 g/L glucose, 0% ethanol) | | | (60 g/L glucose, 5% ethanol) | | | |
| **ORF** | **NAME** | **WT** | **MUT** | **Ratio Top 1%** | **WT** | **MUT** | **Ratio Top 1%** | **"primed" by mut spt15** |
| YBR093C | *PHO5* | 579 | 3263 | Y | 3157 | 3157 | | Y |
| YML123C | *PHO84* | 2166 | 9790 | Y | 12132 | 11786 | | Y |
| YDR281C | *PHM6* | 193 | 694 | Y | 1170 | 1166 | | Y |
| YDR070C | *FMP16* | 832 | 2784 | Y | 2712 | 4218 | Y | Y |
| YGR043C | - | 617 | 1850 | Y | 1801 | 2936 | Y | Y |
| YIL099W | *SGA1* | 246 | 554 | Y | 425 | 824 | Y | Y |
| YPL019C | *VTC3* | 2116 | 4659 | Y | 6032 | 6145 | | Y |
| YDR019C | *GCV1* | 1887 | 3841 | Y | 5290 | 6380 | | Y |
| YGL263W | *COS12* | 170 | 337 | Y | 61 | 60 | | |
| YPR192W | *AQY1* | 224 | 440 | Y | 235 | 336 | Y | |
| YHR140W | - | 555 | 1056 | Y | 817 | 996 | | Y |
| YAL061W | - | 1117 | 2119 | Y | 1909 | 2700 | Y | Y |
| YBR072W | *HSP26* | 3514 | 6565 | Y | 13612 | 13991 | | Y |
| YKL086W | *SRX1* | 300 | 550 | Y | 900 | 1600 | Y | Y |

**Table 8: Fold improvement of mutants (single, double, and isolated triple mutant spt15) compared with the control strain in the presence of 5% ethanol and various glucose concentrations after 20 hours of incubation.**

| Glucose concentration | 20 g/L | 60 g/L | 100 g/L | 120 g/L |
|---|---|---|---|---|
| F177S | 0.87 | 1.13 | 0.90 | 1.19 |
| Y195H | 1.04 | 1.21 | 1.40 | 1.72 |
| K218R | 0.88 | 1.03 | 1.21 | 1.35 |
| F177S K218R | 1.57 | 1.67 | 1.41 | 1.17 |
| F177S Y195H | 1.70 | 2.05 | 1.91 | 1.62 |
| Y195H K218R | 1.52 | 1.55 | 1.33 | 1.00 |
| | | | | |
| F177S Y195H K218R | 4.95 | 5.00 | 4.33 | 4.34 |

**Table 9: Fold improvement of mutants (single, double, and isolated triple mutant spt15) compared with the control strain in the presence of 6% ethanol and various glucose concentrations after 20 hours of incubation.**

| Glucose concentration | 20 g/L | 60 g/L | 100 g/L | 120 g/L |
|---|---|---|---|---|
| F177S | 1.47 | 1.09 | 0.86 | 0.97 |
| Y195H | 1.94 | 1.19 | 1.61 | 1.41 |
| K218R | 1.29 | 1.06 | 1.25 | 0.97 |
| F177S K218R | 1.60 | 3.27 | 1.30 | 1.43 |
| F177S Y195H | 1.84 | 3.48 | 3.16 | 1.67 |
| Y195H K218R | 1.62 | 2.76 | 1.45 | 1.27 |
| | | | | |
| F177S Y195H K218R | 7.63 | 9.09 | 13.22 | 3.72 |

### References for Supplemental Information

1. H. Alper, C. Fischer, E. Nevoigt, G. Stephanopoulos, Proc Natl Acad Sci U S A 102, 12678-83 (Sep 6, 2005).
2. D. Mumberg, R. Muller, M. Funk, Gene 156, 119-22 (Apr 14, 1995).
3. P. Shannon et al., Genome Res 13, 2498-504 (Nov, 2003).
4. H. Alper, G. Stephanopoulos, Submitted, Awaiting Citation Information (2006).
5. H. Scholz, M. Franz, U. Heberlein, Nature 436, 845-7 (Aug 11, 2005).

### Industrial polyploid yeast strains

Using the same methods described above, the mutations described above have been assessed in the context of industrial polyploid yeast strains. The results obtained show that such strains also can be similarly improved by the methods of the invention.

Those skilled in the art will recognize, or be able to ascertain using no more than routine experimentation, many equivalents to the specific embodiments of the invention described herein. Such equivalents are intended to be encompassed by the following claims.

All references disclosed herein are incorporated by reference in their entirety.

The present invention will be further understood by reference to the following numbered clauses:
1. A genetically modified yeast strain comprising a mutated *SPT15* gene, optionally wherein prior to introduction of the mutated *SPT15* gene or mutation of an endogenous *SPT15* gene the yeast strain without the mutated *SPT15* gene had improved ethanol and/or glucose tolerance and/or ethanol production relative to a wild type yeast strain, and wherein the mutated *SPT15* gene further improves ethanol and/or glucose tolerance and/or ethanol production relative to the wild type yeast and the yeast strain without the mutated *SPT15* gene.
2. The genetically modified yeast strain of clause 1, wherein the mutated *SPT15* gene comprises mutations at two or more of positions F177, Y195 and K218.
3. The genetically modified yeast strain of clause 1, wherein the mutated *SPT15* gene comprises mutations at all three positions F177, Y195 and K218.
4. The genetically modified yeast strain of clause 1, wherein the mutated *SPT15* gene comprises two or more of the mutations F177S, Y195H and K218R, or conservative substitutions of the mutant amino acids.
5. The genetically modified yeast strain of clause 1, wherein the mutated *SPT15* gene comprises mutations the F177S, Y195H and K218R or conservative substitutions of of the mutant amino acids.
6. The genetically modified yeast strain of any of clauses 1-5, wherein the mutated *SPT15* gene is recombinantly expressed.
7. The genetically modified yeast strain of any of clauses 1-6, wherein the mutated *SPT15* gene is introduced into the yeast cell on a plasmid.
8. The genetically modified yeast strain of any of clauses 1-6, wherein the mutated *SPT15* gene is introduced into the genomic DNA of the yeast cell.
9. The genetically modified yeast strain of any of clauses 1-6, wherein the mutated *SPT15* gene is an endogenous gene in the genomic DNA of the yeast cell that is mutated *in situ.*
10. The genetically modified yeast strain of any of clauses 1-9, wherein the yeast strain is selected from *Saccharomyces* spp., *Schizosaccharomyces* spp., *Pichia* spp., *Paffia* spp., *Kluyveromyces* spp., *Candida* spp., *Talaromyces* spp., *Brettanomyces* spp., *Pachysolen* spp., *Debaryomyces* spp., and industrial polyploid yeast strains.
11. The genetically modified yeast strain of clause 10, wherein the yeast strain is a *S*. *cerevisiae* strain.
12. The genetically modified yeast strain of clause 1, wherein the yeast strain without the mutated *SPT15* gene is a yeast strain that is genetically engineered, selected, or known to have one or more desirable phenotypes for enhanced ethanol production.
13. The genetically modified yeast strain of clause 12, wherein the one or more desirable phenotypes are ethanol tolerance and/or increased fermentation of C5 and C6 sugars.
14. The genetically modified yeast strain of clause 13, wherein the phenotype of increased fermentation of C5 and C6 sugars is increased fermentation of xylose.
15. The genetically modified yeast strain of clause 14, wherein the genetically modified yeast strain is transformed with an exogenous xylose isomerase gene, an exogenous xylose reductase gene, and exogenous xylitol dehydrogenase gene and/or an exogenous xylulose kinase gene.
16. The genetically modified yeast strain of clause 14, wherein the genetically modified yeast strain comprises a further genetic modification that is deletion of non-specific or specific aldose reductase gene(s), deletion of xylitol dehydrogenase gene(s) and/or overexpression of xylulokinase.
17. The genetically modified yeast strain of clause 1, wherein the yeast strain without the mutated *SPT15* gene is a yeast strain that is respiration-deficient.
18. The genetically modified yeast strain of clause 1, wherein the yeast strain displays normal expression or increased expression of Spt3.
19. The genetically modified yeast strain of clause 1, wherein the yeast strain is not an Spt3 knockout or null mutant.
20. A method for making the genetically modified yeast strain of any of clauses 1-19, comprising introducing into a yeast strain one or more copies of the mutated *SPT15* gene and/or mutating *in situ* an endogenous gene in the genomic DNA of the yeast cell.
21. A method for producing ethanol comprising culturing the genetically modified yeast strain of any of clauses 1-19 in a culture medium that has one or more substrates that are metabolizable into ethanol, for a time sufficient to produce a fermentation product that contains ethanol.
22. The method of clause 21, wherein the one or more substrates that are metabolizable into ethanol comprise C5 and/or C6 sugars.
23. The method of clause 22, wherein the one or more C5 and/or C6 sugars comprise glucose and/or xylose.
24. A method for producing ethanol comprising culturing the genetically modified yeast strain comprising a mutated *SPT15* gene having mutations at F177S, Y195H and K218R, in a culture medium that has one or more substrates that are metabolizable into ethanol, for a time sufficient to produce a fermentation product that contains ethanol.
25. The method of clause 24, wherein the one or more substrates that are metabolizable into ethanol comprise C5 and/or C6 sugars.
26. The method of clause 25, wherein the one or more C5 and/or C6 sugars comprise glucose and/or xylose.
27. A fermentation product of the methods of any of clauses 21-26.
28. Ethanol isolated from the fermentation product of clause 27.
29. The ethanol of clause 28, wherein the ethanol is isolated by distillation of the fermentation product.
30. A method for producing a yeast strain having improved ethanol and/or glucose tolerance and/or ethanol production comprising
   providing a yeast strain comprising a mutated *SPT15* gene, and
   performing genetic engineering and/or selection for improved ethanol and/or glucose tolerance and/or improved ethanol production.
31. The method of clause 30, wherein the mutated *SPT15* gene comprises mutations at two or more of positions F177, Y195 and K218.
32. The method of clause 30, wherein the mutated *SPT15* gene comprises mutations at all three positions F177, Y195 and K218.
33. The method of clause 30, wherein the mutated *SPT15* gene comprises two or more of the mutations F177S, Y195H and K218R, or conservative substitutions of these mutations.
34. The method of clause 30, wherein the mutated *SPT15* gene comprises mutations the F177S, Y195H and K218R or conservative substitutions of these mutations.
35. The method of any of clauses 30-34, wherein the mutated *SPT15* gene is recombinantly expressed.
36. The method of any of clauses 30-35, wherein the mutated *SPT15* gene is introduced into the yeast cell on a plasmid.
37. The method of any of clauses 30-35, wherein the mutated *SPT15* gene is introduced into the genomic DNA of the yeast cell.
38. The method of any of clauses 30-35, wherein the mutated *SPT15* gene endogenous gene in the genomic DNA of the yeast cell that is mutated *in situ.*
39. The method of any of clauses 30-38, wherein the yeast strain is selected from *Saccharomyces* spp., *Schizosaccharomyces* spp., *Pichia* spp., *Paffia* spp., *Kluyveromyces* spp., *Candida* spp., *Talaromyces* spp., *Brettanomyces* spp., *Pachysolen* spp., *Debaryomyces* spp., and industrial polyploid yeast strains.
40. The method of clause 39, wherein the yeast strain is a *S*. *cerevisiae* strain.
41. The method of clause 39, wherein the yeast strain is Spt15-300.
42. A yeast strain produced by the method of any of clauses 30-41.
43. A method for producing ethanol comprising culturing the yeast strain of clause 42 in a culture medium that has one or more substrates that are metabolizable into ethanol, for a time sufficient to produce a fermentation product that contains ethanol.
44. The method of clause 43, wherein the one or more substrates that are metabolizable into ethanol comprise C5 and/or C6 sugars.
45. The method of clause 44, wherein the one or more C5 and/or C6 sugars comprise glucose and/or xylose.
46. A fermentation product of the methods of any of clauses 43-45.
47. Ethanol isolated from the fermentation product of clause 46.
48. The ethanol of clause 47, wherein the ethanol is isolated by distillation of the fermentation product.
49. A yeast strain that overexpresses any combination of at least 2, 3, 4, 5, 6, 7, 8, 9, 10, 11, 12, 13, or all 14 genes listed in Table 5, or genes with one or more substantially similar or redundant biological/biochemical activities or functions.
50. A genetically modified yeast strain that, when cultured in a culture medium containing an elevated level of ethanol, achieves a cell density at least 4 times as great as a wild type strain cultured in the culture medium containing an elevated level of ethanol.
51. The genetically modified yeast strain of clause 50, wherein the strain achieves a cell density between 4-5 times as great as a wild type strain.
52. The genetically modified yeast strain of clause 50, wherein the elevated level of ethanol is at least about 5%.
53. The genetically modified yeast strain of clause 50, wherein the elevated level of ethanol is at least about 6%.
54. The genetically modified yeast strain of clause 50-53, wherein the culture medium comprises one or more sugars at a concentration of at least about 20 g/L.
55. The genetically modified yeast strain of clause 54, wherein the one or more sugars are present at a concentration of at least about 60 g/L.
56. The genetically modified yeast strain of clause 55, wherein the one or more sugars are present at a concentration of at least about 100 g/L.
57. The genetically modified yeast strain of clause 56, wherein the one or more sugars are present at a concentration of at least about 120 g/L.

## Claims

1. A genetically modified yeast strain that, when cultured in a culture medium containing an elevated level of ethanol, achieves a cell density at least 4 times as great as a wild type strain cultured in the culture medium containing an elevated level of ethanol.

2. The genetically modified yeast strain of claim 1, wherein the strain achieves a cell density between 4-5 times as great as a wild type strain.

3. The genetically modified yeast strain of either claim 1 or claim 2, wherein the elevated level of ethanol is at least about 5%.

4. The genetically modified yeast strain of any one of claims 1 to 3, wherein the elevated level of ethanol is at least about 6%.

5. The genetically modified yeast strain of any one of claims 1 to 4, wherein the culture medium comprises one or more sugars at a concentration of at least about 20 g/L.

6. The genetically modified yeast strain of any one of claims 1 to 5, wherein the one or more sugars are present at a concentration of at least about 60 g/L.

7. The genetically modified yeast strain of any one of claims 1 to 6, wherein the one or more sugars are present at a concentration of at least about 100 g/L.

8. The genetically modified yeast strain of any one of claims 1 to 7, wherein the one or more sugars are present at a concentration of at least about 120 g/L.
